(19) Europäisches Patentamt
European Patent Office
Office européen des brevets

(11) Numéro de publication : **0 636 608 A1**

(12) # DEMANDE DE BREVET EUROPEEN

(21) Numéro de dépôt : **94401737.5**

(22) Date de dépôt : **28.07.94**

(51) Int. Cl.[6] : **C07D 209/34,** C07D 209/96, A61K 31/44, A61K 31/40, C07D 491/10, C07D 409/12, C07D 403/12, C07D 401/12, C07D 495/10, C07D 405/12, C07D 405/14

(30) Priorité : **30.07.93 FR 9309404**

(43) Date de publication de la demande : **01.02.95 Bulletin 95/05**

(84) Etats contractants désignés : **AT BE CH DE DK ES FR GB GR IE IT LI LU MC NL PT SE**

(71) Demandeur : **SANOFI**
**32-34, rue Marbeuf**
**F-75008 Paris (FR)**

(72) Inventeur : **Di Malta, Alain**
**170 rue de Vignebelle**
**F-34980 Saint Clement de Riviere (FR)**
Inventeur : **Foulon, Loic**
**14 rue de l'Ousse**
**F-31120 Pinsaguel (FR)**
Inventeur : **Garcia, Georges**
**27 rue des Aires**
**F-34980 St. Gely Du Fesc (FR)**
Inventeur : **Nisato, Dino**
**2 Rue de Terre Rouge**
**F-34680 Saint Georges d'Orques (FR)**
Inventeur : **Roux, Richard**
**420 Chemin des Rossignols**
**F-34570 Vailhauques (FR)**
Inventeur : **Serradeil-Legal, Claudine**
**45 Avenue des Troubadours**
**F-31750 Escalquens (FR)**
Inventeur : **Valette, Gérard**
**8 Rue de Montségur**
**F-31120 Lacroix-Sasgarde (FR)**
Inventeur : **Wagnon, Jean**
**90 rue des Galaxies,**
**Le Hameau de la Rauze**
**F-34000 Montpellier (FR)**

(74) Mandataire : **Gillard, Marie-Louise et al**
**Cabinet Beau de Loménie**
**158, rue de l'Université**
**F-75340 Paris Cédex 07 (FR)**

(54) **Dérivés du 1-benzènesulfonyl-1,3-dihydro-indol-2-one, leur préparation, les compositions pharmaceutiques en contenant.**

(57) L'invention concerne des dérivés du 1-benzènesulfonyl-1,3-dihydro-indol-2-one de formule :

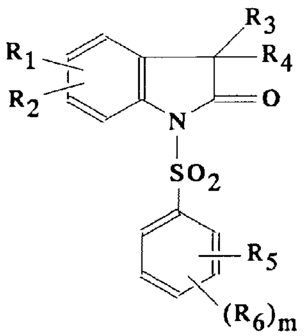

(I)

et leurs sels éventuels, ainsi que leur préparation et les compositions pharmaceutiques en contenant.
Ces composés ont une affinité pour les récepteurs de la vasopressine et/ou de l'ocytocine.

EP 0 636 608 A1

Jouve, 18, rue Saint-Denis, 75001 PARIS

La présente invention a pour objet des dérivés du 1-benzènesulfonyl-1,3-dihydro-indol-2-one, leur préparation et les compositions pharmaceutiques en contenant.

La demande de brevet international WO 91/01306 décrit des dérivés de 2-oxoindole, utiles pour le traitement de la démence sénile. Ces composés répondent à la formule :

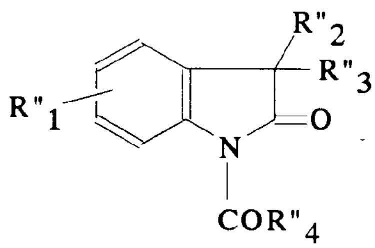

1

dans laquelle

- $R''_1$ présente un hydrogène, un halogène, un alkyle ou un alcoxy ;
- R"2 représente l'hydrogène ou un alkyle inférieur ;
- R"3 représente un alkyle, un cycloalkylméthyle, un benzodioxanylméthyle, ou un benzyle éventuellement substitué ;
- R"4 représente un 1-propylbutyle, un pyridyle ou un phényle éventuellement substitué.

Récemment, plusieurs demandes de brevet ont décrit des familles de composés à structure non-peptidique ayant une activité sur les récepteurs de la vasopressine et/ou de l'ocytocine. On peut citer les demandes de brevets européens EP 382 185, EP 444 945, EP 514 667, EP 469 984 et EP 526 348, les demandes internationales WO 91/05 549 et WO 93/15 051, la demande de brevet JP 04/321 669 et plus particulièrement la demande de brevet JP 03/127732. Cette dernière décrit des dérivés de l'acide indole-3-propanoique de formule :

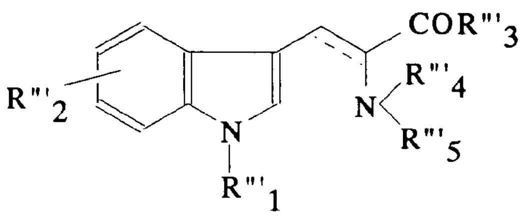

2

dans laquelle

- $R'''_1$ présente l'hydrogène, un alkyle, un alcényle, un phénylalkyle, un tétrahydrofuryle, un alcoxycarbonyle, un alcoxycarbonylalkyle, un carboxyalkyle ou un alcanoyle ;
- $R'''_2$ représente l'hydrogène, un hydroxyle, un alcoxy, un alkyle, un phénylalkyle, un phénylalcoxy ou un halogène ;
- $R'''_3$ représente un hydrogène, un alcoxy, un groupe amino libre ou substitué ou un résidu d'aminoacide ;
- $R'''_4$ représente l'hydrogène, un alkyle ou un phénylalkyle;
- $R'''_5$ représente un benzoyle, un phényle, un alkyle, un phénylalcénylcarbonyle, un thiénylcarbonyle, un phénylsulfonyle, un pyridylcarbonyle ou un imidazolylcarbonyle, les groupes phényle et alkyle du substituant $R'''_5$ pouvant être substitués.

Ces composés sont des antagonistes de la vasopressine.

Le brevet US 4 803 217 revendique des hapalindolinones obtenues par fermentation qui sont des antagonistes de la vasopressine. Ces composés sont représentés par la formule suivante :

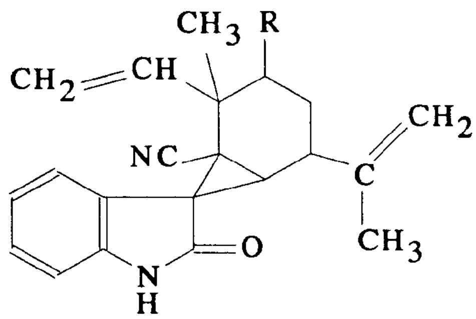

3

dans laquelle R représente H ou Cl.

On a maintenant trouvé des nouveaux dérivés du 1-benzènesulfonyl-1,3-dihydro-indol-2-one qui ont aussi une affinité pour les récepteurs de la vasopressine et de l'ocytocine.

La vasopressine est une hormone connue pour son effet antidiurétique et son effet dans la régulation de la pression artérielle. Elle stimule plusieurs types de récepteurs : $V_1$ ($V_{1a}$, $V_{1b}$), $V_2$. Ces récepteurs sont localisés dans le foie, les vaisseaux (coronaires, rénaux, cérébraux), les plaquettes, le rein, l'utérus, les glandes surrénales, le système nerveux central, l'hypophyse. L'ocytocine a une structure peptidique proche de celle de la vasopressine. Les récepteurs de l'ocytocine se trouvent aussi sur le muscle lisse de l'utérus, ils se trouvent également sur des cellules myoépithéliales de la glande mammaire, dans le système nerveux central et dans le rein. La localisation des différents récepteurs est décrite dans : S. JARS et al., Vasopressin and ocytocin receptors : an overview, *in* Progress in Endocrinology. H. IMURA and K. SHIZURNE ed., Experta Medica, Amsterdam, 1988, 1183-1188, ainsi que dans les articles suivants : Presse Médicale, 1987, 16 (10), 481-485, J. Lab. Clin. Med., 1989, 114 (6), 617-632 et Pharmacol. Rev., 1991, 43 (1), 73-108. La vasopressine exerce ainsi des effets cardiovasculaires, hépatiques, antidiurétiques, agrégants et des effets sur le système nerveux central et périphérique et sur la sphère utérine. L'ocytocine intervient dans la parturition, la lactation et le comportement sexuel.

Les composés selon la présente invention permettent soit de mimer les effets de l'hormone (pour les agonistes), soit de les inhiber (pour les antagonistes), de façon sélective. Les antagonistes des récepteurs de la vasopressine peuvent intervenir sur la régulation de la circulation centrale et périphérique, notamment les circulations coronaire, rénale et gastrique, ainsi que sur la régulation hydrique et la libération de l'hormone adrénocorticotrophique (ACTH). Les agonistes de la vasopressine peuvent remplacer avantageusement la vasopressine ou ses analogues dans le traitement du diabète insipide ; ils peuvent également être utilisés dans le traitement de l'énurésie, et dans la régulation de l'hémostase : traitement de l'hémophilie, du syndrome de Von Willebrand, antidote des agrégants plaquettaires, F.A. LASZLO, Pharmacol. Rev., 1991, 43, 73-108. Drug Investigation, 1990, 2 (Suppl. 5), 1-47. Les hormones elles-mêmes : la vasopressine et l'ocytocine ainsi que certains de leurs analogues peptidiques ou non peptidiques sont utilisés en thérapeutique et ont montré leur efficacité. On peut citer plusieurs revues et de nombreux articles de la littérature Vasopressin. P. GROSS et al. ed. John Libbey Eurotext, 1993, en particulier 243-257 et 549-562. F.A. LASZLO and F.A. LASZLO Jr., Clinical perspectives for vasopressin antagonists, Drug News Perspect., 1993, 6 (8); W.G. NORTH, J. Clin. Endocrinol., 1991, 73, 1316-1320. J.J. LEGROS et al., Prog. Neuro-Pharmcol. Biol. Psychiat., 1988, 12, 571-586 ; K.E. ANDERSSON et al., Drugs Today, 1988, 24 (7), 509-528 ; D.L. STUMP et al., Drugs, 1990, 39, 38-53 ; S. CALTABIANO et al., Drugs Future, 1988, 13, 25-30 ; Y. MURA et al., Clin. Nephrol. 1993, 40, 60-61 ; Faseb J. 1994, 8 (5), A 587 : 3398.

Ainsi les composés selon l'invention sont utiles notamment dans le traitement des affections du système nerveux central et périphérique, du système cardiovasculaire, de la sphère rénale, de la sphère gastique et dans les troubles du comportement sexuel, chez l'homme et chez l'animal.

La présente invention a pour objet des composés de formule :

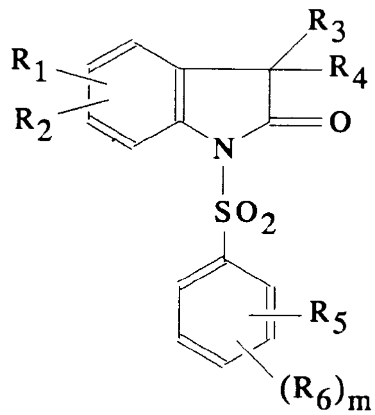

(I)

dans laquelle

- $R_1$ et $R_2$ représentent chacun indépendamment un hydrogène ; un hydroxy ; un ω -halogénoalcoxy en $C_1$-$C_7$ ; un halogène ;un alkyle en $C_1$-$C_7$ ; un trifluorométhyle ; un alcoxy en $C_1$-$C_7$ ; un polyhalogénoalcoxy en $C_1$-$C_7$ ; un ω -hydroxyalcoxy en $C_2$-$C_7$ ; un ω -méthoxyalcoxy dans lequel l'alkyle est en $C_2$-$C_7$ ; un ω -aminoalcoxy en $C_2$-$C_7$ libre ou substitué par un ou deux alkyles en $C_1$-$C_7$ ; un cycloalkyloxy en $C_3$-$C_7$ ; un cycloalkylméthoxy dans lequel le cycloalkyle est en $C_3$-$C_7$ ; un phénoxy; un benzyloxy ; un alkylthio en $C_1$-$C_7$ ; un phénylthio ; un nitro; un amino libre ou substitué par un ou deux alkyles en $C_1$-$C_7$ ; un cyano ; un ($C_1$-$C_6$)alkylcarbonyle ; un formyle ; un ($C_1$-$C_6$)alkylcarbonyloxy ; un formyloxy; un alkylsulfonamido en $C_1$-$C_7$ ; un phénylsulfonamido ; un benzylsulfonamido ; un alkylamido en $C_1$-$C_7$ ; un alcoxycarbonylamino en $C_1$-$C_7$ ; un uréido non substitué ou substitué par un phényle, par un benzyle ou par un ou deux alkyles en $C_1$-$C_7$ ; un thiouréido non substitué ou substitué par un phényle, par un benzyle ou par un ou deux alkyles en $C_1$-$C_7$ ;
- $R_3$ et $R_4$ représentent chacun indépendamment un alkyle en $C_1$-$C_7$ ; un cycloalkyle en $C_3$-$C_7$ ; un phényle ; un benzyle ; un cycloalkylméthyle dans lequel le cycloalkyle est en $C_3$-$C_7$ ; un ω -hydroxyalkyle en $C_2$-$C_7$ dans lequel l'hydroxyle est libre ou substitué par un groupe choisi parmi les groupes alkyle en $C_1$-$C_4$, ($C_1$-$C_5$) alcoxyalkyle dans lequel l'alkyle est en $C_1$-$C_4$, phénylalcoxyalkyle dans lequel l'alcoxy est en $C_1$-$C_2$ et l'alkyle en $C_1$-$C_4$, tétrahydrofuranyle et tétrahydropyranyle ;

ou

- $R_3$ et $R_4$ ensemble constituent un groupe $-(CH_2)_pX(CH_2)_q-$ ;

ou

- $R_3$ et $R_4$ ensemble avec le carbone auquel ils sont liés constituent un cycle hydrocarboné en $C_3$-$C_{12}$, saturé ou insaturé, éventuellement condensé, non substitué ou substitué par un ou plusieurs groupes alkyles en $C_1$-$C_7$ ; par un groupe oxo ; par un spirocycloalkyle en $C_3$-$C_5$ ; par un ou deux hydroxy libres ou substitués par un groupe choisi parmi les groupes alkyle en $C_1$-$C_4$, ($C_1$-$C_5$) alcoxyalkyle dans lequel l'alkyle est en $C_1$-$C_4$, ω -hydroxyalkyle dans lequel l'alkyle est en $C_1$-$C_4$, triphénylméthoxyalkyle dans lequel l'alkyle est en $C_1$-$C_4$, phénylalcoxyalkyle dans lequel l'alcoxy est en $C_1$-$C_2$ et l'alkyle en $C_1$-$C_4$, tétrahydrofuranyle, tétrahydropyranyle, formyle et ($C_1$-$C_7$)alkylcarbonyle ;
- $R_5$ et $R_6$ représentent chacun indépendamment un hydrogène ; un halogène ; un alkyle en $C_1$-$C_7$ ; un trifluorométhyle ; un cyano ; un nitro ; un amino libre ou substitué par un ou deux alkyles en $C_1$-$C_7$ ; un hydroxylamino ; un hydroxy ; un carboxy ; un guanidino non substitué ou substitué une ou deux fois par un alkyle en $C_1$-$C_7$, un phényle ou un benzyle ; un groupe $OR_7$ ; un groupe $SR_7$ ; un ($C_1$-$C_6$)alkylcarbonyle ; un formyle ; un alcoxycarbonyle en $C_1$-$C_7$ ; un phénoxycarbonyle ; un benzyloxycarbonyle ; un carbamoyle substitué par des groupes $R'_6$ et $R''_6$ ; un thiocarbamoyle libre ou substitué par un ou deux alkyles en $C_1$-$C_7$ ; un sulfamoyle ; un alkylsulfamoyle ou dialkylsulfamoyle dans lequel l'alkyle est en $C_1$-$C_7$ ; un groupe $SO_2R'_7$ ; un alkylsulfonamido dans lequel l'alkyle est en $C_1$-$C_7$ ; un phénylsulfonamido ; un benzylsulfonamido ; un groupe $COR'_7$ ; un groupe $NR_8R_9$ ; un groupe $CO\text{-}NH\text{-}CR_{10}R'_{10}\text{-}COR_{12}$ ; le cas échéant, le groupe phényle constitutif du substituant $R_5$ et/ou $R_5$, peut être non substitué ou substitué une ou plusieurs fois par un alkyle en $C_1$-$C_7$, un trifluorométhyle, un alcoxy en $C_1$-$C_7$, un halogène, un sulfamoyle, un alkylsulfamoyle dans lequel l'alkyle est en $C_1$-$C_7$, un carboxy, un alcoxycarbonyle dans lequel l'alkyle est en $C_1$-$C_7$, un ($C_1$-$C_6$)alkylcarbonyloxy, un imidazolyle ;
- $R'_6$ et $R''_6$ représentent chacun indépendamment l'hydrogène; un alkyle en $C_1$-$C_7$ non substitué ou substitué par un ou plusieurs halogènes ou par $R'''_6$ ; un cycloalkyle en $C_3$-$C_7$ non substitué ou substitué par un ($C_1$-$C_4$)alkyle ; un phényle ; un pyridyle; un méthylpyridyle ; un pipéridin-4-yle ; un méthylpipéridin-4-yle ; un pyrrolidin-1-yle ; ou $R'_6$ et $R''_6$ constituent avec l'atome d'azote auquel ils sont liés un groupe pyrrolidino substitué par un hydroxyméthyle, un carbamoyle libre ou substitué par un ou deux alkyles en $C_1$-$C_7$ ;

- $R'''_6$ représente un hydroxyle ; un alcoxy en $C_1$-$C_7$ ; un amino libre ou substitué par un ou deux alkyles en $C_1$-$C_7$ ; un carbamoyle libre ou substitué par un ou deux alkyles en $C_1$-$C_7$ ou dans lequel les deux substituants ensemble avec l'atome d'azote auquel ils sont liés constituent un pyrrolidino, un pipéridino ou un perhydroazépino ; un cyano ; un carboxy libre ou estérifié par un alkyle en $C_1$-$C_7$ ou par un benzyle ; un phényle ; un cycloalkyle en $C_3$-$C_7$ ; un adamanty ; un radical hétérocyclique choisi parmi les groupes pyridyle, méthylpyridyle, furanyle, tétrahydrofuranyle, thiényle, méthylthiényle, pyrrolidino, pipéridino, perhydroazépino ;
- $R_7$ représente un alkyle en $C_1$-$C_7$ ; un phényle ; un benzyle ; un cycloalkyle en $C_3$-$C_7$ ; un alcényle en $C_2$-$C_7$ ; un $\omega$ -halogénoalkyle en $C_2$-$C_7$ ; un polyhalogénoalkyle en $C_1$-$C_7$ ; un $\omega$-hydroxyalkyle en ($C_2$-$C_7$) ; un ($C_1$-$C_6$)alkylcarbonyle ; un formyle ; un $\omega$ -carboxyalkyle en $C_1$-$C_7$ libre ou estérifié par un alkyle en $C_1$-$C_7$ ou par un benzyle ; un $\omega$ -aminoalkyle en $C_2$-$C_7$ dans lequel le groupe amino est libre, substitué par un ou deux alkyles en $C_1$-$C_7$ ou sous forme d'ion ammonium ; un $\omega$ -carbamoylalkyle en $C_1$-$C_7$ libre ou substitué par un ou deux alkyles en $C_1$-$C_7$ ;
- $R'_7$ représente un groupe pipérazin-1-yl non substitué ou substitué en 4 par un groupe $R''_7$ ; un groupe pipéridino non substitué ou substitué en 4 par un groupe $R'''_7$ un groupe azétidin-1-yl non substitué ou substitué en 3 par un groupe $R'''_7$ ; un groupe pyrrolidino non substitué ou substitué par un groupe $R''''_7$;
- $R''_7$ représente un alkyle en $C_1$-$C_7$, un phényle, un benzyle, un ($C_1$- $C_6$)alkylcarbonyle ; un formyle ;
- $R'''_7$ représente $R''_7$ ou un amino libre ou portant un groupe protecteur ;
- $R''''_7$ représente $R'''_7$ ou un groupe carboxy libre ou estérifié par un alkyle en $C_1$-$C_7$;
- $R_8$ et $R_9$ représentent chacun indépendamment un hydrogène ; un alkyle en $C_1$-$C_7$; un benzyle ; un phényle ; $R_9$ peut de plus représenter un alcène en $C_3$-$C_8$; un ($C_1$-$C_6$)alkylcarbonyle ; un formyle ; un ($C_1$-$C_6$)alkylthiocarbonyle ; un cycloalkylcarbonyle dans lequel le cycloalkyle est en $C_3$-$C_7$ ; un cycloalkylthiocarbonyle dans lequel le cycloalkyle est en $C_3$-$C_7$ ; un $\omega$-amino($C_2$-$C_6$)alkylcarbonyle ; un $\omega$-hydroxy($C_1$-$C_6$)alkylcarbonyle ; un $\omega$-benzyloxy($C_1$-$C_6$)alkylcarbonyle ; un phénoxycarbonyle ; un phénoxythiocarbonyle ; un thiénocarbonyle ; un pyridylcarbonyle ; un méthylpyridylcarbonyle ; un alcoxycarbonyle en $C_1$-$C_7$ ; un benzoyle ; un phénacétyle ; un groupe $CO-CR_{10}R'_{10}-NR_{11}R'_{11}$ ; un groupe $CR_{10}R'_{10}COR_{12}$ ; un groupe $(CH_2)_t$ $COR_{12}$; un groupe $CO(CH_2)_{t'}COR_{12}$ ; un carbamoyle non substitué ou substitué par $R_{14}$ et $R'_{14}$ ; un thiocarbamoyle non substitué ou substitué par $R_{14}$ et $R'_{14}$ ; un radical hétérocyclique choisi parmi les groupes pyrazolyle, imidazolyle, triazolyle, tétrazolyle, pyridinyle, pyridazinyle, pyrimidinyle, pyridyle, thiazolyle ;
- ou $R_8$ et $R_9$ ensemble avec l'atome d'azote auquel ils sont liés constituent l'hydantoïne, la N-méthyl hydantoïne ou un hétérocycle choisi parmi le pyrrol-1-yle, le $\Delta$3-pyrrolin-1-yle, le pyrrolidin-1-yle ou l'isoindolin-2-yle dans lequel le noyau benzénique peut être non substitué ou substitué par un halogène, un alkyle en $C_1$-$C_7$, un trifluorométhyle ou un méthoxy ;
- $R_{10}$ et $R'_{10}$ représentent chacun indépendamment l'hydrogène, un alkyle en $C_1$-$C_7$ ou un benzyle ou $R_{10}$ et $R'_{10}$ ensemble avec l'atome de carbone auquel ils sont liés constituent un cycloalkyle en $C_3$-$C_7$ ;
- $R_{11}$ et $R'_{11}$ représentent chacun indépendamment l'hydrogène ou un alkyle en $C_1$-$C_7$ ;
- $R_{12}$ représente un hydroxy, un alcoxy en $C_1$-$C_7$, un amino non substitué ou substitué par un ou deux alkyles en $C_1$-$C_7$ ;
- $R_{13}$ représente l'hydrogène, un alkyle en $C_1$-$C_7$, un phényle, un benzyle, un ($C_1$-$C_6$)alkylcarbonyle, un formyle ; un alcoxycarbonyle en $C_1$-$C_7$, un carbamoyle non substitué ou substitué par un ou 2 alkyles en $C_1$-$C_7$ ;
- $R_{14}$ et $R'_{14}$ représentent chacun indépendamment un alkyle en $C_1$-$C_7$ non substitué ou substitué par $R_{15}$, un phényle non substitué ou substitué par $R'_{15}$, un cycloalkyle en $C_3$-$C_7$, un adamantyle ;
- ou $R_{14}$ et $R'_{14}$ ensemble avec l'atome d'azote auquel ils sont liés constituent un hétérocycle choisi parmi : la morpholine, la thiomorpholine, la pipérazine, l'azétidine, la pyrrolidine, la pipéridine ou la perhydroazépine, ledit hétérocycle étant non substitué ou substitué par un ou plusieurs groupes méthyles, par un phényle, par un groupe amino libre ou portant un groupe protecteur ;
- $R_{15}$ représente un phényle, un pyridyle, un hydroxy, un alcoxy en $C_1$-$C_7$, un amino libre ou substitué par un ou deux alkyles en $C_1$-$C_7$, un carboxy libre ou estérifié par un alkyle en $C_1$-$C_7$ ;
- $R'_{15}$ représente un hydroxy, un amino libre ou substitué par un ou deux alkyles en $C_1$-$C_7$ ;
- m est 1 ou, lorsque $R_6$ est un halogène, un alkyle en $C_1$-$C_7$ ou un alcoxy en $C_1$-$C_7$, m peut également être 2, 3 ou 4 ou bien $(R_6)_m$ peut représenter m substituants ayant différentes valeurs choisies parmi halogène, alkyle en $C_1$-$C_7$ ou alcoxy en $C_1$-$C_7$ ;

- p et q représentent chacun un nombre entier, leur somme pouvant varier de 3 à 6 ;
- t représente un nombre entier qui peut varier de 2 à 5 ;
- t' est un nombre entier qui peut varier de 0 à 3 ;
- X représente l'oxygène, un groupe $S(O)_n$, un groupe $NR_{13}$, un groupe $N(O)R_{13}$ ;
- n représente 0, 1 ou 2

avec la limitation que lorsque :

- $R_1$ et $R_2$ sont tels que définis ci-dessus à l'exception du groupe uréido substitué par un groupe benzyle ou du groupe thiouréido non substitué ou substitué par un phényle, un benzyle ou par un ou deux alkyles en $C_1$-$C_4$ ;
- $R_3$ et $R_4$ représentent chacun indépendamment un alkyle en $C_1$-$C_6$, un cycloalkyle en $C_3$-$C_7$, un phényle, un benzyle, un cycloalkylméthyle dans lequel le cycloalkyle est en $C_3$-$C_7$ ;

ou

- $R_3$ et $R_4$ ensemble constituent un groupe $-(CH_2)_pX(CH_2)_q-$ ; dans lequel X représente l'oxygène, le soufre ou un groupe $NR_{13}$,

ou

- $R_3$ et $R_4$ ensemble avec le carbone auquel ils sont liés constituent un cycle hydrocarboné en $C_3$-$C_{10}$, saturé ou insaturé, éventuellement condensé, non substitué ou substitué par un ou plusieurs groupes alkyles en $C_1$-$C_7$ ou par un spirocycloalkyle en $C_3$-$C_5$ ;
- $R_5$ et $R_6$ soient autres qu'un hydrogène ; un halogène ; un alkyle en $C_1$-$C_7$ ; un trifluorométhyle ; un cyano ; un nitro ; un amino libre ou substitué par un ou deux alkyles en $C_1$-$C_7$ ; un hydroxylamino ; un hydroxy ; un carboxy ; un groupe $OR_7$ ; un groupe $SR_7$ ; un ($C_1$-$C_6$)alkylcarbonyle ; un formyle ; un alcoxycarbonyle en $C_1$-$C_7$ ; un phénoxycarbonyle ; un benzyloxycarbonyle ; un carbamoyle substitué par des groupes $R'_6$ et $R''_6$ ; un thiocarbamoyle libre ou substitué par un ou deux alkyles en $C_1$-$C_7$ ; un sulfamoyle ; un alkylsulfamoyle ou dialkylsulfamoyle dans lequel l'alkyle est en $C_1$-$C_7$ ; un groupe $SO_2R'_7$ ; un alkylsulfonamido dans lequel l'alkyle est en $C_1$-$C_7$ ; un groupe $COR'_7$ ; un groupe $NR_5R_9$ ; un groupe CO-NH-CH($R_{10}$)-$COR_{12}$ ; le cas échéant, le groupe phényle constitutif du substituant $R_5$ et/ou $R_5$, pouvant être non substitué ou substitué une ou plusieurs fois par un alkyle en $C_1$-$C_7$, un trifluorométhyle, un méthoxy, un halogène, un sulfamoyle, un alkylsulfamoyle dans lequel l'alkyle est en $C_1$-$C_7$, un carboxy, un alcoxycarbonyle dans lequel l'alkyle est en $C_1$-$C_7$, un ($C_1$-$C_6$)alkylcarbonyloxy, un formyloxy ; un imidazolyle ;

dans lesquels groupes $R_5$ et/ou $R_5$ :

- $R'_6$ et $R''_6$ représentent chacun indépendamment l'hydrogène ; un alkyle en $C_1$-$C_7$ non substitué ou substitué par $R'''_6$ ; un phényle ; un pyridyle ; un méthylpyridyle; un pipéridin-4-yle ; un méthylpipéridin-4-yle ;
- $R'''_6$ représente un hydroxy ; un cyano ; un carboxy libre ou estérifié par un alkyle en $C_1$-$C_7$ ou par un benzyle ; un phényle ; un pyridyle ; un méthylpyridyle; un amino libre ou substitué par un ou deux alkyles en $C_1$-$C_7$;
- $R_7$ représente un alkyle en $C_1$-$C_7$ ; un phényle ; un benzyle; un cycloalkyle en $C_3$-$C_7$ ; un alcényle en $C_2$-$C_4$ ; un $\omega$ -halogénoalkyle en $C_2$-$C_7$ ; un polyhalogénoalkyle en $C_1$-$C_7$ ; un ($C_1$-$C_6$)alkylcarbonyle ; un formyle ; un $\omega$ -carboxyalkyle en $C_1$-$C_7$ libre ou estérifié par un alkyle en $C_1$-$C_4$ ou par un benzyle ; un $\omega$ -aminoalkyle en $C_2$-$C_7$ dans lequel le groupe amino est libre, substitué par un ou deux alkyles en $C_1$-$C_4$ ou sous forme d'ion ammonium ;
- $R'_7$ représente un groupe pipérazin-1-yl non substitué ou substitué en 4 par un groupe $R''_7$, un groupe pipéridino non substitué ou substitué en 4 par un groupe $R'''_7$, un groupe azétidin-1-yl non substitué ou substitué en 3 par un groupe $R'''_7$;
- $R''_7$ représente un alkyle en $C_1$-$C_4$, un phényle, un benzyle, un ($C_1$-$C_3$)alkylcarbonyle ;
- $R'''_7$ représente $R''_7$ ou un amino libre ou portant un groupe protecteur ;
- $R_5$ et $R_9$ représentent chacun indépendamment un hydrogène ; un alkyle en $C_1$-$C_7$ ; un phényle ; un benzyle ; $R_9$ peut de plus représenter un ($C_1$-$C_6$)alkylcarbonyle ; un formyle ; un cycloalkylcarbonyle dans lequel le cycloalkyle est en $C_3$-$C_7$ ; un cycloalkylthiocarbonyle dans lequel le cycloalkyle est en $C_3$-$C_7$ ; un $\omega$-amino($C_2$-$C_3$)alkylcarbonyle ; un $\omega$-hydroxy($C_1$-$C_3$)alkylcarbonyle ; un $\omega$-benzyloxy($C_1$-$C_3$)alkylcarbonyle ; un phénoxycarbonyle ; un thiénocarbonyle ; un pyridylcarbonyle ; un méthylpyridylcarbonyle ; un alcoxycarbonyle en $C_1$-$C_4$; un benzoyle ; un groupe CO-CH($R_{10}$)-$NR_{11}R'_{11}$ ; un groupe CH($R_{10}$)$CO_2R_{11}$ ; un groupe $(CH_2)_{t'}$ $COR_{12}$ ; un groupe $CO(CH_2)_{t''}COR_{12}$ ; un carbamoyle non substitué ou substitué par un phényle ou par un ou deux alkyles en $C_1$-$C_4$ ;
- t'' représente un nombre entier qui peut varier de 1 à 3 ;
- $R_{10}$ représente l'hydrogène, un alkyle en $C_1$-$C_4$ ou un benzyle ;
- $R_{11}$ et $R'_{11}$ représentent chacun indépendamment l'hydrogène ou un alkyle en $C_1$-$C_4$ ;
- $R_{12}$ représente un hydroxy, un alcoxy en $C_1$-$C_4$, un amino non substitué ou substitué par un ou deux

alkyles en $C_1$-$C_4$ ;

- $R_{13}$ représente l'hydrogène ; un alkyle en $C_1$-$C_4$ ; un phényle ; un benzyle ; un ($C_1$-$C_3$)alkylcarbonyle ; un formyle ; un alcoxycarbonyle en $C_1$-$C_4$ ; un carbamoyle non substitué ou substitué par un ou 2 alkyles en $C_1$-$C_4$ ;

ainsi que leurs sels éventuels.

Avantageusement, les composés de l'invention sont des composés de formule (I) :

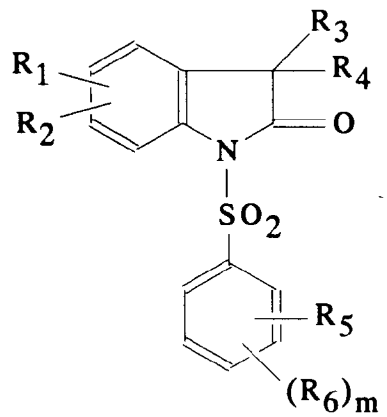

(I)

dans laquelle

- $R_1$ et $R_2$ représentent chacun indépendamment un hydrogène ; un hydroxy ; un ω -halogénoalcoxy en $C_1$-$C_7$ ; un halogène ; un alkyle en $C_1$-$C_7$ ; un trifluorométhyle ; un alcoxy en $C_1$-$C_7$ ; un polyhalogénoalcoxy en $C_1$-$C_7$ ; un ω -hydroxyalcoxy en $C_2$-$C_7$ ; un ω -méthoxyalcoxy dans lequel l'alkyle est en $C_2$-$C_7$ ; un ω -aminoalcoxy en $C_2$-$C_7$ libre ou substitué par un ou deux alkyles en $C_1$-$C_7$ ; un cycloalkyloxy en $C_3$-$C_7$ ; un cycloalkylméthoxy dans lequel le cycloalkyle est en $C_3$-$C_7$ ; un phénoxy ; un benzyloxy ; un alkylthio en $C_1$-$C_7$ ; un phénylthio ; un nitro ; un amino libre ou substitué par un ou deux alkyles en $C_1$-$C_7$ ; un cyano ; un ($C_1$-$C_6$)alkylcarbonyle ; un formyle ; un ($C_1$-$C_6$)alkylcarbonyloxy ; un formyloxy; un alkylsulfonamido en $C_1$-$C_7$ ; un phénylsulfonamido ; un benzylsulfonamido ; un alkylamido en $C_1$-$C_7$ ; un alcoxycarbonylamino en $C_1$-$C_7$ ; un uréido non substitué ou substitué par un phényle, par un benzyle ou par un ou deux alkyles en $C_1$-$C_7$ ; un thiouréido non substitué ou substitué par un phényle, par un benzyle ou par un ou deux alkyles en $C_1$-$C_7$ ;
- $R_3$ et $R_4$ représentent chacun indépendamment un alkyle en $C_1$-$C_7$ ; un cycloalkyle en $C_3$-$C_7$ ; un phényle ; un benzyle ; un cycloalkylméthyle dans lequel le cycloalkyle est en $C_3$-$C_7$ ; un ω -hydroxyalkyle en $C_2$-$C_7$ dans lequel l'hydroxyle est libre ou substitué par un groupe choisi parmi les groupes alkyle en $C_1$-$C_4$, ($C_1$-$C_2$) alcoxyalkyle dans lequel l'alkyle est en $C_1$-$C_4$, phénylalcoxyalkyle dans lequel l'alcoxy est en $C_1$-$C_2$ et l'alkyle en $C_1$-$C_4$, tétrahydrofuranyle et tétrahydropyranyle ;

ou

- $R_3$ et $R_4$ ensemble constituent un groupe $-(CH_2)_pX(CH_2)_q-$ ;

ou

- $R_3$ et $R_4$ ensemble avec le carbone auquel ils sont liés constituent un cycle hydrocarboné en $C_3$-$C_{12}$, saturé ou insaturé, éventuellement condensé, non substitué ou substitué par un ou plusieurs groupes alkyles en $C_1$-$C_7$ ; par un groupe oxo ; par un spirocycloalkyle en $C_3$-$C_5$ ; par un hydroxy libre ou substitué par un groupe choisi parmi les groupes alkyle en $C_1$-$C_4$, ($C_1$-$C_2$) alcoxyalkyle dans lequel l'alkyle est en $C_1$-$C_4$, phénylalcoxyalkyle dans lequel l'alcoxy est en $C_1$-$C_2$ et l'alkyle en $C_1$-$C_4$, tétrahydrofuranyle ou tétrahydropyranyle ;
- $R_5$ et $R_6$ représentent chacun indépendamment un hydrogène ; un halogène ; un alkyle en $C_1$-$C_7$ ; un trifluorométhyle ; un cyano ; un nitro ; un amino libre ou substitué par un ou deux alkyles en $C_1$-$C_7$ ; un hydroxylamino ; un hydroxy ; un carboxy ; un guanidino non substitué ou substitué une ou deux fois par un alkyle en $C_1$-$C_7$, un phényle ou un benzyle ; un groupe $OR_7$ ; un groupe $SR_7$ ; un ($C_1$-$C_6$)alkylcarbonyle ; un formyle ; un alcoxycarbonyle en $C_1$-$C_7$ ; un phénoxycarbonyle ; un benzyloxycarbonyle ; un carbamoyle substitué par des groupes $R'_6$ et $R''_6$ ; un thiocarbamoyle libre ou substitué par un ou deux alkyles en $C_1$-$C_7$ ; un sulfamoyle ; un alkylsulfamoyle ou dialkylsulfamoyle dans lequel l'alkyle est en $C_1$-$C_7$ ; un groupe $SO_2R'_7$ ; un alkylsulfonamido dans lequel l'alkyle est en $C_1$-$C_7$ ; un phénylsulfonamido ; un benzylsulfonamido ; un groupe $COR'_7$ ; un groupe $NR_8R_9$ ; un groupe $CO-NH-CR_{10}R'_{10}-COR_{12}$ ; le cas

échéant, le groupe phényle constitutif du substituant $R_5$ et/ou $R_6$, peut être non substitué ou substitué une ou plusieurs fois par un alkyle en $C_1$-$C_7$, un trifluorométhyle, un alcoxy en $C_1$-$C_7$, un halogène, un sulfamoyle, un alkylsulfamoyle dans lequel l'alkyle est en $C_1$-$C_7$, un carboxy, un alcoxycarbonyle dans lequel l'alkyle est en $C_1$-$C_7$, un ($C_1$-$C_6$)alkylcarbonyloxy, un imidazolyle ;

- $R'_6$ et $R''_6$ représentent chacun indépendamment l'hydrogène ; un alkyle en $C_1$-$C_7$ non substitué ou substitué par un ou plusieurs halogènes ou par $R'''_6$ ; un phényle ; un pyridyle; un méthylpyridyle ; un pipéridin-4-yle ; un méthylpipéridin-4-yle ; un pyrrolidin-1-yle ; ou $R'_6$ et $R''_6$ constituent avec l'atome d'azote auquel ils sont liés un groupe pyrrolidino substitué par un hydroxyméthyle, un carbamoyle libre ou substitué par un ou deux alkyles en $C_1$-$C_7$ ;
- $R'''_6$ représente un hydroxyle ; un alcoxy en $C_1$-$C_7$ ; un amino libre ou substitué par un ou deux alkyles en $C_1$-$C_7$ ; un carbamoyle libre ou substitué par un ou deux alkyles en $C_1$-$C_7$ ou dans lequel les deux substituants ensemble avec l'atome d'azote auquel ils sont liés constituent un pyrrolidino ou un pipéridino ; un cyano; un carboxy libre ou estérifié par un alkyle en $C_1$-$C_7$ ou par un benzyle ; un phényle ; un cycloalkyle en $C_3$-$C_7$ ; un adamantyle ; un radical hétérocyclique choisi parmi les groupes pyridyle, méthylpyridyle, furanyle, tétrahydrofuranyle, thiényle, méthylthiényle, pyrrolidino ou pipéridino ;
- $R_7$ représente un alkyle en $C_1$-$C_7$ ; un phényle ; un benzyle ; un cycloalkyle en $C_3$-$C_7$ ; un alcényle en $C_2$-$C_7$ ; un ω -halogénoalkyle en $C_2$-$C_7$ ; un polyhalogénoalkyle en $C_1$-$C_7$ ; un ($C_1$-$C_6$)alkylcarbonyle ; un formyle ; un ω -carboxyalkyle en $C_1$-$C_7$ libre ou estérifié par un alkyle en $C_1$-$C_7$ ou par un benzyle ; un ω -aminoalkyle en $C_2$-$C_7$ dans lequel le groupe amino est libre, substitué par un ou deux alkyles en $C_1$-$C_7$ ou sous forme d'ion ammonium ; un ω -carbamoylalkyle en $C_1$-$C_7$ libre ou substitué par un ou deux alkyles en $C_1$-$C_7$ ;
- $R'_7$ représente un groupe pipérazin-1-yl non substitué ou substitué en 4 par un groupe $R''_7$ ; un groupe pipéridino non substitué ou substitué en 4 par un groupe $R'''_7$ ; un groupe azétidin-1-yl non substitué ou substitué en 3 par un groupe $R'''_7$ ; un groupe pyrrolidino non substitué ou substitué par un groupe $R''''_7$;
- $R''_7$ représente un alkyle en $C_1$-$C_7$, un phényle, un benzyle, un ($C_1$-$C_6$)alkylcarbonyle ; un formyle ;
- $R'''_7$ représente $R''_7$ ou un amino libre ou portant un groupe protecteur ;
- $R''''_7$ représente $R'''_7$ ou un groupe carboxy libre ou estérifié par un alkyle en $C_1$-$C_7$;
- $R_6$ et $R_9$ représentent chacun indépendamment un hydrogène ; un alkyle en $C_1$-$C_7$; un benzyle ; un phényle ; $R_9$ peut de plus représenter un alcène en $C_3$-$C_8$ dans lequel la double liaison est en $C_3$-$C_4$ ; un ($C_1$-$C_6$)alkylcarbonyle ; un formyle ; un ($C_1$-$C_6$)alkylthiocarbonyle ; un cycloalkylcarbonyle dans lequel le cycloalkyle est en $C_3$-$C_7$ ; un cycloalkylthiocarbonyle dans lequel le cycloalkyle est en $C_3$-$C_7$ ; un ω-amino($C_2$-$C_6$)alkylcarbonyle ; un ω-hydroxy($C_1$-$C_6$)alkylcarbonyle ; un ω -benzyloxy($C_1$-$C_6$)alkylcarbonyle ; un phénoxycarbonyle ; un thiénocarbonyle ; un pyridylcarbonyle ; un méthylpyridylcarbonyle ; un alcoxycarbonyle en $C_1$-$C_7$ ; un benzoyle ; un phénacétyle ; un groupe $CO-CR_{10}R'_{10}-NR_{11}R'_{11}$ ; un groupe $CR_{10}R'_{10}COR_{12}$ ; un groupe $(CH_2)_t\ COR_{12}$; un groupe $CO(CH_2)_{t'}COR_{12}$ ; un carbamoyle non substitué ou substitué par $R_{14}$ et $R'_{14}$ ; un thiocarbamoyle non substitué ou substitué par $R_{14}$ et $R'_{14}$ ; un radical hétérocyclique choisi parmi les groupes pyrazolyle, imidazolyle, triazolyle, tétrazolyle, pyridinyle, pyridazinyle, pyrimidinyle, pyridyle, thiazolyle ;
- ou $R_6$ et $R_9$ ensemble avec l'atome d'azote auquel ils sont liés constituent l'hydantoïne, la N-méthyl hydantoïne ou un hétérocycle choisi parmi le pyrrol-1-yle, le pyrrolidin-1-yle ou l'isoindolin-2-yle dans lequel le noyau benzénique peut être non substitué ou substitué par un halogène, un alkyle en $C_1$-$C_7$, un trifluorométhyle ou un méthoxy ;
- $R_{10}$ et $R'_{10}$ représentent chacun indépendamment l'hydrogène, un alkyle en $C_1$-$C_7$ ou un benzyle ou $R_{10}$ et $R'_{10}$ ensemble avec l'atome de carbone auquel ils sont liés constituent un cycloalkyle en $C_3$-$C_7$ ;
- $R_{11}$ et $R'_{11}$ représentent chacun indépendamment l'hydrogène ou un alkyle en $C_1$-$C_7$ ;
- $R_{12}$ représente un hydroxy, un alcoxy en $C_1$-$C_7$, un amino non substitué ou substitué par un ou deux alkyles en $C_1$-$C_7$ ;
- $R_{13}$ représente l'hydrogène, un alkyle en $C_1$-$C_7$, un phényle, un benzyle, un ($C_1$-$C_6$)alkylcarbonyle, un formyle ; un alcoxycarbonyle en $C_1$-$C_7$, un carbamoyle non substitué ou substitué par un ou 2 alkyles en $C_1$-$C_7$ ;
- $R_{14}$ et $R'_{14}$ représentent chacun indépendamment un alkyle en $C_1$-$C_7$ non substitué ou substitué par $R_{15}$, un phényle non substitué ou substitué par $R'_{15}$, un cycloalkyle en $C_3$-$C_7$, un adamantyle ;
- ou $R_{14}$ et $R'_{14}$ ensemble avec l'atome d'azote auquel ils sont liés constituent un hétérocycle choisi parmi : la morpholine, la thiomorpholine, la pipérazine, l'azétidine, la pyrrolidine ou la pipéridine , ledit hétérocycle étant non substitué ou substitué par un ou plusieurs groupes méthyles, par un phényle, par un groupe amino libre ou portant un groupe protecteur ;
- $R_{15}$ représente un phényle, un pyridyle, un hydroxy, un alcoxy en $C_1$-$C_7$, un amino libre ou substitué par

un ou deux alkyles en $C_1$-$C_7$, un carboxy libre ou estérifié par un alkyle en $C_1$-$C_7$ ;
- $R'_{15}$ représente un hydroxy, un amino libre ou substitué par un ou deux alkyles en $C_1$-$C_7$ ;
- m est 1 ou, lorsque $R_6$ est un halogène, un alkyle en $C_1$-$C_7$ ou un alcoxy en $C_1$-$C_7$, m peut également être 2, 3 ou 4 ou bien $(R_6)_m$ peut représenter m substituants ayant différentes valeurs choisies parmi halogène, alkyle en $C_1$-$C_7$ ou alcoxy en $C_1$-$C_7$ ;
- p et q représentent chacun un nombre entier, leur somme pouvant varier de 3 à 6 ;
- t représente un nombre entier qui peut varier de 2 à 5 ;
- t' est un nombre entier qui peut varier de 1 à 3 ;
- X représente l'oxygène, un groupe $S(O)_n$, un groupe $NR_{13}$, un groupe $N(O)R_{13}$ ;
- n représente 0, 1 ou 2

avec la limitation que lorsque :
- $R_1$ et $R_2$ sont tels que définis ci-dessus à l'exception du groupe uréido substitué par un groupe benzyle ou du groupe thiouréido non substitué ou substitué par un phényle, un benzyle ou par un ou deux alkyles en $C_1$-$C_4$ ;
- $R_3$ et $R_4$ représentent chacun indépendamment un alkyle en $C_1$-$C_6$, un cycloalkyle en $C_3$-$C_7$, un phényle, un benzyle, un cycloalkylméthyle dans lequel le cycloalkyle est en $C_3$-$C_7$ ;

ou

- $R_3$ et $R_4$ ensemble constituent un groupe $-(CH_2)_pX(CH_2)_q-$ ; dans lequel X représente l'oxygène, le soufre ou un groupe $NR_{13}$,

ou

- $R_3$ et $R_4$ ensemble avec le carbone auquel ils sont liés constituent un cycle hydrocarboné en $C_3$-$C_{10}$, saturé ou insaturé, éventuellement condensé, non substitué ou substitué par un ou plusieurs groupes alkyles en $C_1$-$C_7$ ou par un spirocycloalkyle en $C_3$-$C_5$ ;
- $R_5$ et $R_6$ soient autres qu'un hydrogène ; un halogène ; un alkyle en $C_1$-$C_7$ ; un trifluorométhyle ; un cyano ; un nitro ; un amino libre ou substitué par un ou deux alkyles en $C_1$-$C_7$ ; un hydroxylamino ; un hydroxy ; un carboxy ; un groupe $OR_7$; un groupe $SR_7$ ; un $(C_1$-$C_6)$alkylcarbonyle ; un formyle ; un alcoxycarbonyle en $C_1$-$C_7$ ; un phénoxycarbonyle ; un benzyloxycarbonyle ; un carbamoyle substitué par des groupes $R'_6$ et $R''_6$ ; un thiocarbamoyle libre ou substitué par un ou deux alkyles en $C_1$-$C_7$ ; un sulfamoyle ; un alkylsulfamoyle ou dialkylsulfamoyle dans lequel l'alkyle est en $C_1$-$C_7$ ; un groupe $SO_2R'_7$ ; un alkylsulfonamido dans lequel l'alkyle est en $C_1$-$C_7$ ; un groupe $COR'_7$ ; un groupe $NR_5R_9$ ; un groupe $CO$-$NH$-$CH(R_{10})$-$COR_{12}$ ; le cas échéant, le groupe phényle constitutif du substituant $R_5$ et/ou $R_5$, pouvant être non substitué ou substitué une ou plusieurs fois par un alkyle en $C_1$-$C_7$, un trifluorométhyle, un méthoxy, un halogène, un sulfamoyle, un alkylsulfamoyle dans lequel l'alkyle est en $C_1$-$C_7$, un carboxy, un alcoxycarbonyle dans lequel l'alkyle est en $C_1$-$C_7$, un $(C_1$-$C_6)$alkylcarbonyloxy, un formyloxy ; un imidazolyle ;

dans lesquels groupes $R_5$ et/ou $R_5$ :
- $R'_6$ et $R''_6$ représentent chacun indépendamment l'hydrogène ; un alkyle en $C_1$-$C_7$ non substitué ou substitué par $R'''_6$ ; un phényle ; un pyridyle ; un méthylpyridyle; un pipéridin-4-yle ; un méthylpipéridin-4-yle ;
- $R'''_6$ représente un hydroxy ; un cyano ; un carboxy libre ou estérifié par un alkyle en $C_1$-$C_7$ ou par un benzyle ; un phényle ; un pyridyle ; un méthylpyridyle; un amino libre ou substitué par un ou deux alkyles en $C_1$-$C_7$;
- $R_7$ représente un alkyle en $C_1$-$C_7$ ; un phényle ; un benzyle ; un cycloalkyle en $C_3$-$C_7$ ; un alcényle en $C_2$-$C_4$ ; un ω -halogénoalkyle en $C_2$-$C_7$ ; un polyhalogénoalkyle en $C_1$-$C_7$ ; un $(C_1$-$C_6)$alkylcarbonyle ; un formyle ; un ω -carboxyalkyle en $C_1$-$C_7$ libre ou estérifié par un alkyle en $C_1$-$C_4$ ou par un benzyle ; un ω -aminoalkyle en $C_2$-$C_7$ dans lequel le groupe amino est libre, substitué par un ou deux alkyles en $C_1$-$C_4$ ou sous forme d'ion ammonium ;
- $R'_7$ représente un groupe pipérazin-1-yl non substitué ou substitué en 4 par un groupe $R''_7$, un groupe pipéridino non substitué ou substitué en 4 par un groupe $R'''_7$, un groupe azétidin-1-yl non substitué ou substitué en 3 par un groupe $R'''_7$;
- $R''_7$ représente un alkyle en $C_1$-$C_4$, un phényle, un benzyle, un $(C_1$-$C_3)$alkylcarbonyle ;
- $R'''_7$ représente $R''_7$ ou un amino libre ou portant un groupe protecteur ;
- $R_5$ et $R_9$ représentent chacun indépendamment un hydrogène ; un alkyle en $C_1$-$C_7$ ; un phényle ; un benzyle ; $R_9$ peut de plus représenter un $(C_1$-$C_6)$alkylcarbonyle ; un formyle ; un cycloalkylcarbonyle dans lequel le cycloalkyle est en $C_3$-$C_7$ ; un cycloalkylthiocarbonyle dans lequel le cycloalkyle est en $C_3$-$C_7$ ; un ω-amino$(C_2$-$C_3)$alkylcarbonyle ; un ω-hydroxy$(C_1$-$C_3)$alkylcarbonyle ; un ω-benzyloxy$(C_1$-$C_3)$alkylcarbonyle ; un phénoxycarbonyle ; un thiénocarbonyle ; un pyridylcarbonyle ; un méthylpyridylcarbonyle ; un alcoxycarbonyle en $C_1$-$C_4$; un benzoyle ; un groupe $CO$-$CH(R_{10})$-$NR_{11}R'_{11}$ ; un groupe $CH(R_{10})CO_2R_{11}$ ; un groupe $(CH_2)_{t''}$ $COR_{12}$ ; un groupe $CO(CH_2)_{t''}COR_{12}$ ; un carbamoyle non substitué

ou substitué par un phényle ou par un ou deux alkyles en $C_1$-$C_4$ ;

- t'' représente un nombre entier qui peut varier de 1 à 3 ;
- $R_{10}$ représente l'hydrogène, un alkyle en $C_1$-$C_4$ ou un benzyle ;
- $R_{11}$ et $R'_{11}$ représentent chacun indépendamment l'hydrogène ou un alkyle en $C_1$-$C_4$ ;
- $R_{12}$ représente un hydroxy, un alcoxy en $C_1$-$C_4$, un amino non substitué ou substitué par un ou deux alkyles en $C_1$-$C_4$ ;
- $R_{13}$ représente l'hydrogène ; un alkyle en $C_1$-$C_4$ ; un phényle ; un benzyle ; un ($C_1$-$C_3$)alkylcarbonyle ; un formyle ; un alcoxycarbonyle en $C_1$-$C_4$ ; un carbamoyle non substitué ou substitué par un ou 2 alkyles en $C_1$-$C_4$ ;

ainsi que leurs sels éventuels.

Lorsqu'un composé selon l'invention présente un ou des carbones asymétriques, l'invention comprend tous les isomères optiques de ce composé.

Lorsqu'un composé selon l'invention présente une isomérie conformationnelle de type axial-équatorial, l'invention comprend tous les isomères conformationnels de ce composé.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide mandélique ou un acide camphosulfonique, et ceux qui forment des sels physiologiquement acceptables tels que le chlorhydrate, le bromhydrate, le sulfate, l'hydrogénosulfate, le dihydrogénophosphate, le maléate, le fumarate, le 2-naphtalènesulfonate.

Les sels des composés de formule (I) comprennent également les sels avec des bases organiques ou minérales, par exemple les sels des métaux alcalins ou alcalino-terreux comme les sels de sodium, de potassium, de calcium, les sels de sodium et de potassium étant préférés, ou avec une amine, telle que le trométamol, ou bien les sels d'arginine, de lysine, ou de toute amine physiologiquement acceptable.

Selon la présente invention, par halogène on entend un atome choisi parmi le fluor, le chlore, le brome ou l'iode, de préférence le fluor ou le chlore. Par groupe protecteur de l'amine, on entend un groupe tel que par exemple, un alkyle en $C_1$-$C_4$ tel que méthyle ou *tert*-butyle ; un benzhydryle ; un trityle ; un benzoyle ; un ($C_1$-$C_4$)alkylcarbonyle, tel qu'un acétyle ; un ($C_1$-$C_4$)alcoxycarbonyle tel que méthoxycarbonyle, éthoxycarbonyle ou le *tert*-butoxycarbonyle, le benzyloxycarbonyle; le benzyle, ou le benzyle substitué tel que p-nitrobenzyle, p-chlorobenzyle ou p-méthoxybenzyle.

Selon la présente invention, par alkyle en $C_1$-$C_4$ ou respectivement en $C_1$-$C_3$ , $C_1$-$C_5$, $C_1$-$C_6$, $C_1$-$C_7$, $C_2$-$C_6$ ou $C_2$-$C_7$ on entend un alkyle droit ou ramifié.

Selon la présente invention par cycle hydrocarboné en $C_3$-$C_{12}$ éventuellement condensé, saturé ou insaturé, on entend différents cycles hydrocarbonés de structure monocyclique, bicyclique ou tricyclique, par exemple un cyclobutane, un cyclopentane, un cyclohexane, un cycloheptane, un cyclooctane, un indane, un hexahydroindane, un adamantane, un norbornane, un norbornène, un dihydrophénalène, un tricyclo [5.2.1.$0^{2,6}$] décane, un tricyclo [5.2.1.$0^{2,6}$]dec-8-ène, un bicyclo [2.2.1]heptane, un bicyclo [3.3.1]nonane.

Selon la présente invention, lorsque $R_3$ et $R_4$ ensemble avec le carbone auquel ils sont liés constituent un cycle hydrocarboné en $C_3$-$C_{12}$, saturé ou insaturé, éventuellement condensé, substitué par un hydroxy, les groupes préférés pour substituer ledit hydroxy, sont : les groupes méthyle, éthyle, méthoxyméthyle, méthoxyéthyle, phénylméthoxyméthyle, tétrahydrofuranyle et tétrahydropyranyle.

Les composés de formule (I) dans lesquels $R_1$ est en position 5 de l'indol-2-one et $R_2$ est l'hydrogène sont des composés préférés.

Les composés de formule (I) dans lesquels $R_1$ est un atome de chlore ou de fluor ou un groupe éthoxy en position 5 de l'indol-2-one et $R_2$ est l'hydrogène sont des composés préférés.

Les composés de formule (I) dans lesquels $R_3$ et $R_4$ ensemble avec le carbone auquel ils sont liés constituent un cycle hydrocarboné en $C_3$-$C_{12}$ sont des composés préférés; particulièrement préférés sont les composés dans lesquels $R_3$ et $R_4$ ensemble avec le carbone auquel ils sont liés constituent un cycloheptane, un adamantane, un tricyclo [5.2.1.$0^{2,6}$]dec-8-ène, un tricyclo [5.2.1.$0^{2,6}$]décane, un bicyclo [2.2.1]heptane, un bicyclo [3.3.1]nonane, ou un cyclohexane non substitué ou substitué par un spirocycloalkyle en $C_3$-$C_5$ ou par un ou deux groupes alkyles en $C_1$-$C_7$.

Plus particulièrement, on préfère les composés dans lesquels $R_3$ et $R_4$ ensemble avec le carbone auquel ils sont liés constituent un cyclohexane substitué par un groupe choisi parmi : méthoxy, éthoxy, 2-méthoxyéthoxy.

Les composés de formule (I) dans lesquels les substituants $R_5$ et $R_5$ sont en position 2,4- du noyau phényle sont des composés préférés.

Les composés de formule (I) dans lesquels $R_5$ représente un groupe orthométhoxy et $R_5$ en position para représente un groupe choisi parmi :

- (pipéridin-1-yl)carboxamido,
- (2-cyanoprop-2-yl)carbonyle,
- pyrrolidin-1-yl,
- 3,3-diéthylguanidino
- N',N'-diéthylthioureido.

sont des composés préférés.

Dans la description et dans les exemples, les abréviations suivantes sont utilisées :

DCM : dichlorométhane
Ether : éther éthylique
Ether iso : éther isopropylique
Boc : *tert*-butoxycarbonyle
Me, MeO : méthyle, méthoxy
Et, EtO : éthyle, éthoxy
Pr, iPr, nPr : propyle, isopropyle, n-propyle
Bu, iBu, tBu : butyle, isobutyle, *tert*-butyle
Ph : phényle
Bz : benzyle
Ts : tosyle
Ac : acétyle
AcOEt : acétate d'éthyle
AcOH : acide acétique
HCl : acide chlorhydrique
MeOH : méthanol
EtOH : éthanol
DMF : diméthylformamide
THF : tétrahydrofuranne
DMSO : diméthylsulfoxyde
DIPEA : diisopropyléthylamine
NaOH : soude
$NaHCO_3$ : hydrogénocarbonate de sodium
TEA : triéthylamine
TFA : acide trifluoroacétique
TMEDA : tétraméthyléthylènediamine
Réactif de lawesson : 2,4-bis-(4-méthoxyphényl)-1,3-dithia-2,4-diphosphétane-2,4-disulfure
F : point de fusion
Solution saline : eau saturée en chlorure de sodium
CCM : chromatographie en couche mince
HPLC : chromatographie liquide à haute pression
Eau chlorhydrique : acide chlorhydrique dilué, environ 1N
TA : température ambiante
Eb : température d'ébullition
RMN : résonnance magnétique nucléaire
s : singulet
se : singulet élargi
d : doublet
t : triplet
q : quadruplet
m : massif
mt : multiplet.

La présente invention a également pour objet un procédé de préparation des composés selon l'invention et de leurs sels caractérisé en ce que :

1/ on fait agir sur un 1,3-dihydro-indol-2-one disubstitué en position 3, de formule :

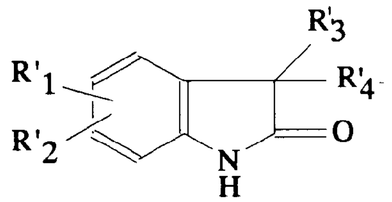

(II)

dans laquelle R'$_1$, R'$_2$, R'$_3$ et R'$_4$ représentent, respectivement, soit $R_1$, $R_2$, $R_3$ et $R_4$ tels que définis pour (I), soit des groupes précurseurs de $R_1$, $R_2$, $R_3$ et $R_4$ un halogénure de benzènesulfonyle de formule :

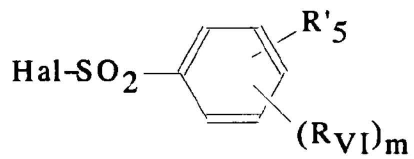

(III)

dans laquelle Hal représente un atome d'halogène, de préférence le chlore et R'$_5$ et $R_{VI}$ représentent, respectivement, soit $R_5$ et $R_6$ tels que définis ci-dessus pour (I), soit des groupes précurseurs de $R_5$ et $R_6$ ; et,

2/ soit, lorsque R'$_1$ = $R_1$, R'$_2$ = $R_2$, R'$_3$ = $R_3$, R'$_4$ = $R_4$, R'$_5$ = $R_5$ et $R_{VI}$ = $R_5$, on isole le composé de formule (I) ainsi obtenu ;

3/ soit, lorsque l'un quelconque des groupes R'$_1$, R'$_2$, R'$_3$, R'$_4$, R'$_5$ et/ou $R_{VI}$ représente respectivement un groupe précurseur de $R_1$, $R_2$, $R_3$, $R_4$, $R_5$ et/ou $R_6$, on soumet le composé obtenu, dénommé ci-après composé de formule (I)', à un traitement ultérieur pour préparer le composé de formule (I) par transformation de l'un quelconque des groupes R'$_1$, R'$_2$, R'$_3$, R'$_4$, R'$_5$ et/ou $R_{VI}$ en, respectivement, $R_1$, $R_2$, $R_3$, $R_4$, $R_5$ et/ou $R_6$ ; et,

4/ le cas échéant, on transforme le composé de formule (I) ainsi obtenu en un de ses sels.

La réaction de l'étape 1 est effectuée dans un solvant anhydre tel que le DMF ou le THF, en présence d'un hydrure métallique tel que l'hydrure de sodium par exemple, ou en présence d'un alcoolate comme le *tert*-butylate de potassium.

Les 1,3-dihydro-indol-2-one (II) sont connus ou peuvent être préparés selon différents modes opératoires par des méthodes connues.

Des composés (II) dans lesquels R'$_1$ et/ou R'$_2$ représentent un halogène et R'$_3$, R'$_4$ ensemble avec le carbone auquel ils sont liés constituent un spirocyclobutane, un spirocyclohexane ou un spirocycloheptane sont connus, par exemple dans D.W.Robertson et al., J.Med.Chem., 1987, 30 (5),824-829. De plus, le 5-chloro-3-spirocyclopentaneindol-2-one est décrit dans le brevet US 3 947 451.

Pour la préparation des composés (II), lorsque R'$_3$ et R'$_4$ représentent ensemble un groupement hydrocarboné, on peut utiliser la réaction de Brunner décrite par R.F. Moore et S.G.P. Plant dans J. Chem. Soc., 1951, 3475-3478 et qui conduit à la préparation de composés (II) dans lesquels $CR_3R_4$ représente un cyclopentane ou un cyclohexane.

Cette réaction est effectuée par cyclisation d'un dérivé de phénylhydrazide de formule :

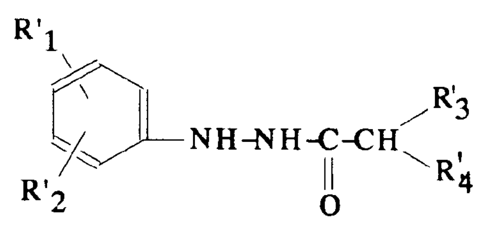

(IV)

dans laquelle R'$_1$, R'$_2$, R'$_3$ et R'$_4$ sont tels que définis ci-dessus pour (II), par exemple par chauffage dans la quinoléine en présence d'oxyde de calcium.

Le dérivé de phénylhydrazide (IV) est obtenu selon les mêmes auteurs par action d'un dérivé de l'hydrazine de formule :

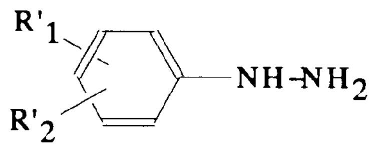

(V)

dans laquelle R'$_1$ et R'$_2$ ont les définitions données ci-dessus pour (II), sur un halogénure d'acide de formule :

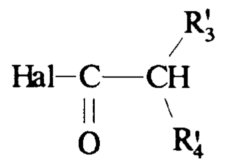

(VI)

dans laquelle R'$_3$ et R'$_4$ ont les définitions données ci-dessus pour (II).

Selon un mode de réalisation particulier, lorsque R'$_3$ et R'$_4$ ensemble avec le carbone auquel ils sont liés constituent un cycle hydrocarboné polycondensé, par exemple un norbomane ou un norbornène, on opère d'après la méthode décrite par J. Wolff et al., Tetrahedron, 1986, 42 (15), 4267-4272 : on prépare d'abord un sel de lithium du composé (IV) par action d'un réactif lithié tel que le n-butyllithium, dans un solvant inerte tel que le THF, à basse température, puis on effectue la cyclisation par chauffage dans un solvant tel que le naphtalène ou le prehnitène (1,2,3,4-tétraméthylbenzène).

Les composés (II) dans lesquels R'$_1$ = R'$_2$ = H et CR'$_3$R'$_4$ représentent l'adamantane sont décrits dans I. Fleming et al., J.Chem.Soc., Perkin Trans I, 1991, 3, 617-626. Les composés (II) dans lesquels R'$_3$ et R'$_4$ ensemble avec l'atome de carbone avec lequel ils sont liés constituent un adamantane et R'$_1$ et R'$_2$ sont différents de l'hydrogène peuvent être préparés par la méthode décrite ci-dessus.

Les dérivés de l'hydrazine (V) sont connus ou préparés par des méthodes connues. Il en est de même pour les halogènures d'acide (VI).

On peut également préparer un 1,3-dihydro-indol-2-one disubstitué en position 3 (II), à partir d'un 1,3-dihydro-indol-2-one de formule :

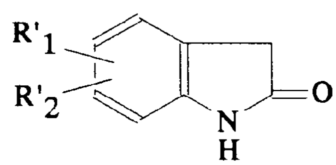

(VII)

dans laquelle R'$_1$ et R'$_2$ ont les valeurs définies ci-dessus pour (II) en utilisant différents procédés.

Par exemple, selon la méthode décrite par A.S. Kende et J.C. Hodges dans Synth. Commun., 1982, 12 (1), 1-10, on réalise l'addition d'un agent alkylant dans un solvant approprié. Ainsi pour préparer un composé (II) dans lequel R'$_3$ = R'$_4$, la réaction est effectuée dans le THF à -75°C en présence de TMEDA, par addition d'un alkyl lithium comme le butyllithium puis réaction avec un halogénure de formule R'$_3$Hal ; lorsque R'$_3$ et R'$_4$ sont différents, la réaction d'alkylation peut être effectuée en 2 étapes, par 2 halogénures d'alkyle différents de formule R'$_3$Hal et R'$_4$Hal. Pour préparer un composé (II) dans lequel R'$_3$ et R'$_4$ ensemble forment un groupe de formule $-(CH_2)_n-$ avec n variant de 3 à 12, on fait agir un composé de formule $Z(CH_2)_nZ$ dans laquelle Z représente un groupe nucléofuge tel qu'un halogène, de préférence le brome ou l'iode, un groupe tosyloxy ou un groupe mésyloxy. Selon une variante de ce procédé, on peut également effectuer la réaction par addition, sur un composé de formule (VII), d'un alcoolate alcalin tel que le *tert*-butylate de potassium dans le THF à -40°C puis addition d'un composé de formule $Z-(CH_2)_nZ$ tel que défini ci-dessus.

On prépare de la même façon les composés (II) dans lesquels R'$_3$ et R'$_4$ ensemble avec le carbone auquel ils sont liés constituent un cycle hydrocarboné en $C_4$-$C_8$ substitué par un ou plusieurs groupes alkyles en $C_1$-$C_7$ ou par un spirocycloalkyle en $C_3$-$C_5$.

Les composés de formule (II) dans lesquels R'$_3$ et R'$_4$ représentent chacun indépendamment un alkyle ou un phényle sont connus. Par exemple, le brevet DE 3 300 522 décrit des 5-alcoxy-3,3-diméthylindol-2-ones.

Lorsque R'$_3$ et R'$_4$ ensemble constituent un groupe $-(CH_2)_p X(CH_2)_q-$, dans lequel p, q et X ont les valeurs

données ci-dessus pour (I), on peut préparer un 1,3-dihydro-indol-2-one disubstitué en position 3 de formule (II), à partir d'un 1,3-dihydro-indol-2-one non substitué en 3 (VII) par action d'un composé de formule :

$$Z\text{-}(CH_2)_p\text{-}X\text{-}(CH_2)_q\text{-}Z \qquad (VIII)$$

dans laquelle Z est tel que défini ci-dessus et X, p et q ont les valeurs définies ci-dessus pour (I). La réaction est effectuée en présence d'un alcoolate, par exemple le *tert*-butylate de potassium, dans un solvant anhydre, tel que le THF par exemple.

Lorsque X représente un atome d'azote substitué par un ($C_1$-$C_6$)alkylcarbonyle, un formyle, un alcoxycarbonyle en $C_1$-$C_7$ ou par un alkylcarbamoyle en $C_1$-$C_7$, la substitution sur X peut être réalisée soit sur le dérivé 1,3-dihydro-indol-2-one (II), soit sur le composé final (I) à partir d'un composé dans lequel l'atome d'azote (X = NH) n'est pas substitué.

Ainsi lorsque X représente un atome d'azote substitué par un alcoxycarbonyle en $C_1$-$C_7$, on prépare d'abord un composé (II) ou (I) dans lequel X est NH, puis on fait agir le chloroformiate approprié pour obtenir le composé (II) ou (I) souhaité. De la même façon, on fait agir sur un composé (II) ou (I) dans lequel X = NH un isocyanate d'alkyle en $C_1$-$C_7$, pour obtenir un dérivé (II) ou un composé (I) dans lequel X représente un atome d'azote substitué par un alkylcarbamoyle. On fait agir sur un composé (II) ou un composé (I) dans lequel X = NH soit un chlorure d'acide ou un anhydride ,soit l'acide formique en présence d'anhydride acétique, pour préparer un composé de formule (II) ou (I) dans lequel X représente dans chaque cas un atome d'azote substitué soit par un ($C_1$-$C_6$)alkylcarbonyle, soit par un formyle.

Lorsque X représente un atome de soufre ou un atome d'azote substitué par $R_{13}$, on peut également préparer d'abord un composé de formule :

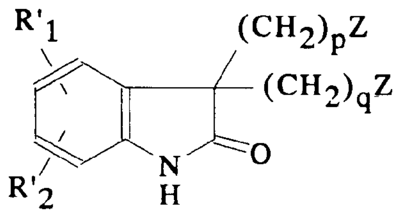

(II)'

dans laquelle R'$_1$, R'$_2$, Z, p et q ont les définitions données ci-dessus, et effectuer une substitution nucléophile avec un sel de l'acide sulfhydrique ou une amine de formule $H_2NR_{13}$ dans un solvant tel qu'un alcool, un éther, le DMF, ou leur mélange, à une température comprise entre 5°C et la température de reflux.

Les 1,3-dihydro-indol-2-ones de formule (II)' sont obtenus à partir des diols correspondants, tels quels ou protégés, par exemple par un groupe tétrahydropyran-2-yle. La réaction peut être effectuée selon J. Chem. Soc., Chem. Commun., 1989, 1619, par action du dibromotriphénylphosphorane.

Les composés (II) dans lesquels R'$_3$ et R'$_4$ ensemble avec le carbone auquel ils sont liés constituent un cycle pyrrolidine ou N-alkyl pyrrolidine, pipéridine ou N-alkyl pipéridine sont décrits par M.J. Kornet dans J. Med. Chem., 1976, 19 (7), 892-899.

En particulier, la horsfiline de formule :

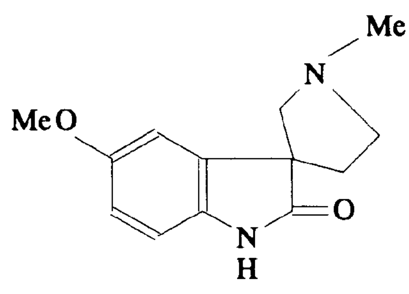

est un alcaloïde décrit dans A. Jossang et al., J. Org. Chem., 1991, 56 (23), 6527-6530.

Pour préparer un composé de formule (II) dans lequel R'$_3$ et R'$_4$ ensemble avec le carbone auquel ils sont liés constituent respectivement un tricyclo [5.2.1.0$^{2,6}$]décane ou un tricyclo [5.2.1.0$^{2,6}$]dec-8-ène, on fait agir sur un composé de formule (VII), respectivement un composé (VII)' ou un composé (VII)" de formules :

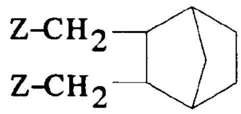

(VII)'

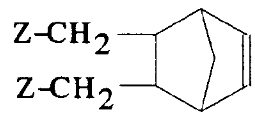

(VII)"

dans lesquelles Z est tel que défini ci-dessus. On utilise des composés (VII)' et (VII)" substitués par un ou plusieurs groupes alkyles en $C_1$-$C_7$ pour préparer des composés (II) dans lesquels lesdits carbocycles sont substitués.

On peut également préparer un composé de formule (II) dans lequel R'$_3$ et R'$_4$ ensemble avec le carbone auquel ils sont liés constituent un tricyclo [5.2.1.0$^{2,6}$]décane par hydrogénation catalytique par exemple en présence de palladium sur charbon ou de nickel de Raney ® d'un composé de formule (II) dans lequel R'$_3$ et R'$_4$ ensemble avec le carbone auquel ils sont liés constituent un tricyclo [5.2.1.0$^{2,6}$]déc-8-ène.

On peut également préparer un composé de formule (I) dans lequel $R_3$ et $R_4$ ensemble avec le carbone auquel ils sont liés constituent un tricyclo[5.2.1.0$^{2,6}$]décane par hydrogénation catalytique par exemple en présence de palladium sur charbon ou de nickel de Raney ® d'un composé de formule (I) dans lequel $R_2$ et $R_4$ ensemble avec le carbone auquel ils sont liés constituent un tricyclo[5.2.1.0$^{2,6}$]déc-8-ène.

Pour préparer un composé (II) dans lequel R'$_3$ et R'$_4$ ensemble avec le carbone auquel ils sont liés constituent respectivement un indane ou un hexahydroindane, on fait agir sur un composé (VII), respectivement un composé (VIII)' ou un composé (VIII)" de formules :

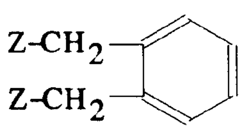

(VIII)'

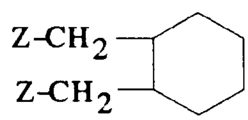

(VIII)"

dans lesquelles Z a la valeur indiquée ci-dessus pour (VIII). On utilise des composés (VIII)' et (VIII)" substitués par un ou plusieurs groupes alkyles en $C_1$-$C_7$ pour préparer des composés (II) dans lesquels l'indane ou l'hexahydroindane sont substitués.

On peut utiliser la méthode de A.S. Kende et J.C. Hodges décrite ci-dessus ou sa variante décrite ci-dessus pour préparer des composés de formule (II) dans lesquels les substituants R'$_3$ et R'$_4$ ensemble avec le carbone auquel ils sont liés constituent un cycle hydrocarboné en $C_3$-$C_{12}$, saturé ou insaturé, éventuellement condensé, substitué par un ou plusieurs groupes alkyles en $C_1$-$C_7$ ou par un groupe choisi parmi : un groupe oxo protégé sous forme d'acétal, un spirocycloalkyle en $C_3$-$C_5$, un ou deux hydroxyles substitués par un alkyle en $C_1$-$C_4$, un ($C_1$-$C_5$) alcoxyalkyle dans lequel l'alkyle est en $C_1$-$C_4$, un triphénylméthoxyalkyle dans lequel l'alkyle est en $C_1$-$C_4$, un phénylalcoxyalkyle dans lequel l'alcoxy est en $C_1$-$C_2$ et l'alkyle en $C_1$-$C_4$, un tétrahydrofuranyle, un tétrahydropyranyle. Pour obtenir les composés de formule (I) dans lesquels $R_3$ et $R_4$ ensemble avec le carbone auquel ils sont liés constituent un cycle hydrocarboné en $C_3$-$C_{12}$, saturé ou insaturé, éventuellement condensé, substitué par un ou deux hydroxyles, on effectue une déprotection des composés de formule (I) correspondants dans lesquels le ou les groupes hydroxyles sont substitués par un( $C_1$-$C_5$) alcoxyalkyle dans lequel l'alkyle est en $C_1$-$C_4$, un tétrahydrofuranyle ou un tétrahydropyranyle. Cette déprotection est effectuée en milieu acide, par exemple en présence d'un acide minéral ou organique, dans un solvant alcoolique ou éthérifié tel que le THF, à une température comprise entre 15°C et la température de reflux ; par exemple la déprotection peut se faire en présence d'acide chlorhydrique ou de pyridinium toluènesulfonate dans un alcool.

Pour obtenir les composés de formule (I) dans lesquels $R_3$ et $R_4$ ensemble avec le carbone auquel ils sont liés constituent un cycle hydrocarboné en $C_3$-$C_{12}$, saturé ou insaturé, éventuellement condensé, substitué par un ou deux ω-hydroxy($C_1$-$C_4$)alcoxy, on effectue une déprotection des composés de formule (I) correspondants dans lesquels $R_3$ et $R_4$ ensemble avec le carbone auquel ils sont liés constituent un cycle hydrocarboné en $C_3$-$C_{12}$, saturé ou insaturé, éventuellement condensé, substitué par un ou deux ($C_1$-$C_5$)alcoxy ($C_1$-$C_4$)alcoxy. Cette déprotection est effectué en milieu acide, par exemple l'acide trifluoroacétique, dans un solvant tel que le DCM.

On peut également préparer des composés de formule (I) dans lesquels $R_3$ et $R_4$ ensemble avec le carbone auquel ils sont liés constituent un cycle hydrocarboné en $C_3$-$C_{12}$, saturé ou insaturé, éventuellement condensé,

substitué par un ou deux ($C_1$-$C_4$)alcoxy ou par un ou deux ($C_1$-$C_5$)alcoxy ($C_1$-$C_4$)alcoxy, par alkylation de composés de formule (I) dans lesquels $R_3$ et $R_4$ ensemble avec le carbone auquel ils sont liés constituent un cycle hydrocarboné en $C_3$-$C_{12}$, saturé ou insaturé, éventuellement condensé, substitué par un ou deux hydroxyles. Pour ce faire, on opère plus particulièrement avec des réactifs alkylants puissants tels que des trifluorométhanesulfonates d'alkyle, dans des solvants tels que le DCM ou le tétrachlorure de carbone, en présence d'une base telle que la 2,6-di-*tert*-butylpyridine, selon la méthode décrite dans Carbohydrate Research, 1975, 44, C5-C7. Les trifluorométhanesulfonates d'alkyle peuvent être obtenus à partir des iodures d'alkyles en les faisant réagir avec un sel de l'acide trifluorométhanesulfonique tel que le sel d'argent (Chemical Reviews, 1977, 77).

On peut préparer des composés de formule (I) dans lesquels $R_3$ et $R_4$ ensemble avec le carbone auquel ils sont liés constituent un cycle hydrocarboné en $C_3$-$C_{12}$, saturé ou insaturé, éventuellement condensé, substitué par un ou deux formyloxy ou respectivement par un ou deux ($C_1$-$C_7$)alkylcarbonyloxy par action du diméthylsulfate en présence de carbonate de césium, ou respectivement par action d'un halogénure d'acide ou d'un anhydride sur un composé de formule (I) dans lequel $R_3$ et $R_4$ ensemble avec le carbone auquel ils sont liés constituent un cycle hydrocarboné en $C_3$-$C_{12}$, saturé ou insaturé, éventuellement condensé, substitué par un ou deux hydroxyles.

On peut également obtenir un composé de formule (I) dans lequel $R_3$ et $R_4$ ensemble avec le carbone auquel ils sont liés constituent un tricyclo[5.2.1.$0^{2,6}$]décan-8,9-diol à partir d'un composé de formule (I) dans lequel $R_3$ et $R_4$ ensemble avec le carbone auquel ils sont liés constituent un tricyclo[5.2.1.$0^{2,6}$]déc-8-ène que l'on fait réagir avec l'acide métachloroperbenzoïque, à température ambiante, dans un solvant tel que le DCM pour obtenir intermédiairement un composé de formule (I) dans lequel $R_3$ et $R_4$ ensemble avec le carbone auquel ils sont liés constituent un tricyclo[5.2.1.$0^{2,6}$]décan-8,9-époxy ; le dérivé époxyde intermédiaire est ensuite hydrolysé par chauffage à reflux dans l'eau en présence d'acide sulfurique ou bien en milieu basique.

Les composés de formule (II) dans lesquels $R'_3$ et $R'_4$ ensemble avec le carbone auquel ils sont liés constituent soit un cycle hydrocarboné en $C_3$-$C_{12}$, saturé ou insaturé, éventuellement condensé substitué par un groupe oxo, soit un groupe $-(CH_2)_p-X-(CH_2)_q-$ avec X représentant un groupe SO, $SO_2$, ou $N(O)R_{13}$ sont préparés par des réactions d'oxydation connue à partir des composés correspondants de formule (II) dans lesquels $R'_3$ et $R'_4$ ensemble avec l'atome de carbone auquel ils sont liés constituent respectivement soit un cycle hydrocarboné en $C_3$-$C_{12}$, saturé ou insaturé, éventuellement condensé, substitué par un hydroxyle, soit un groupe $-(CH_2)_p-X-(CH_2)_q-$ avec X représentant un atome de soufre ou un groupe $NR_{13}$.

Par exemple, l'oxydation d'alcools secondaires en cétone peut s'effectuer en présence de complexes de l'oxyde de chrome tel que le pyridinium chlorochromate dans un solvant inerte tel que le chlorure de méthylène ou par des oxydants tels que le DMSO selon les méthodes décrites dans Tetrahedron, 1978, 34, 1651.

L'oxydation des composés (II) comportant un atome de soufre ou d'azote (X = S, $NR_{13}$) peut s'effectuer en présence d'eau oxygénée ou de peracides tels que l'acide peracétique ou métachloroperbenzoïque dans des solvants inertes tels que les cétones ou l'acide acétique à des températures comprise entre 0°C et 50°C.

Lorsque $R'_3$ et $R'_4$ représentent chacun un phényle, on peut utiliser le procédé décrit dans Helv. Chim. Acta, 1946, 29, 415-432 pour préparer un composé (II).

Les dérivés de 1,3-dihydro-indol-2-one (VII) sont connus ou préparés par des méthodes connues. On peut citer par exemple J.V.RajanBabu dans J. Org. Chem., 1986, 51, 1704-1712.

Les composés de formule (II) portant certains substituants $R'_1$, $R'_2$ sur leur partie benzénique sont utilisés comme précurseurs pour la préparation de composés de formule (II) portant d'autres substituants $R'_1$, $R'_2$. Par exemple, les composés (II) dans lesquels $R'_1$ et/ou $R'_2$ = H peuvent être nitrés par les réactifs classiques ; ils peuvent également être acylés par action d'un chlorure d'acide de formule RCOCl dans lequel R représente un alkyle en $C_1$-$C_7$, en présence d'un acide de Lewis tel que le chlorure d'aluminium pour préparer un composé (II) dans lequel $R'_1$ et/ou $R'_2$ = COR. A partir d'un composé (II) dans lequel $R'_1$ est un groupe nitro et $R'_2$ est l'hydrogène, on prépare par hydrogénation catalytique le composé (II) dans lequel $R'_1$ est un groupe amino.

Les halogénures de benzènesulfonyle (III) sont préparés par des méthodes connues. Ainsi par exemple, le chlorure de 4-diméthylaminobenzènesulfonyle est préparé selon C.N. Sukenik et al., J. Amer. Chem. Soc., 1977, 99, 851-858. Plus généralement, les halogénures de benzènesulfonyle (III) dans lesquels le substituant $R'_5$ est un groupe diméthylamino sont connus ou préparés par des méthodes connues; le chlorure de p-benzyloxybenzènesulfonyle est préparé selon la demande de brevet européen EP 229 566.

Le chlorure d'alcoxybenzènesulfonyle est préparé à partir de l'alcoxybenzènesulfonate de sodium, lui-même préparé par action d'un halogénure d'alkyle sur l'hydroxybenzènesulfonate de sodium.

Le chlorure de 2,4-diméthoxybenzènesulfonyle est préparé selon J. Am. Chem. Soc., 1952, 74, 2008.

On peut préparer les chlorures d'halogénoalcoxybenzènesulfonyle selon le brevet US 2 540 057.

Les halogénures de benzènesulfonyle de formule :

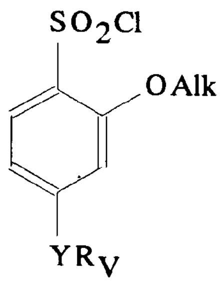

(III)'

dans laquelle

- Alk représente un alkyle en $C_1$-$C_7$ ;
- Y représente O ou S ;
- $R_V$ représente un alkyle en $C_1$-$C_7$, un cycloalkyle en $C_3$-$C_7$, un alcényle en $C_2$-$C_7$, un ω -halogénoalkyle en $C_1$-$C_7$, un polyhalogénoalkyle en $C_1$-$C_7$, un benzyle, un ($C_1$-$C_6$)alkylcarbonyle, un formyle ; un ω -carboxyalkyle en $C_1$-$C_7$ estérifié par un alkyle en $C_1$-$C_4$ ou par un benzyle,

sont préparés d'après D. Hofmann et al. dans Liebigs Ann. Chem., 1982,287-297.

On opère sur des composés benzéniques portant les substituants YRV et OAlk en position -1,3 par action du chlorosulfonate de triméthylsilyle dans un solvant tel que le DCM à TA. On applique ensuite la méthode de R. Passerini et al. dans Gazz. Chim. Ital., 1960, 90, 1277-89 et on neutralise ensuite, par exemple par du carbonate alcalin, puis on fait agir un halogénure tel que $POCl_3$ pour obtenir l'halogénure de benzènesulfonyle souhaité.

Les halogénures de benzènesulfonyle (III) dans lesquels le substituant $R'_5$ représente un alcoxycarbonyle, un phénoxycarbonyle, un benzyloxycarbonyle, un alkylthio, un phénylthio, un benzylthio ou un groupe $SR_7$, $R_7$ étant tel que défini pour (I), sont préparés selon Col. Czechoslov. Chem. Commun., 1984, 49, 1184, à partir d'un dérivé de l'aniline substitué par le même groupement $R'_5$, ledit dérivé de l'aniline étant lui-même obtenu à partir du dérivé nitré correspondant.

Les dérivés de l'acide nitrobenzoïque sont connus ; par une réaction appropriée d'estérification de cet acide, on obtient les esters d'alkyle et de phényle correspondants.

Les dihalogénures de benzène di-sulfonyle (III, $R'_5$ = $SO_2Hal$) sont connus ou préparés par des méthodes connues. Par exemple, le dichlorure de 2,4-diméthoxybenzène-1,5-disulfonyle est décrit dans R.J.W.Cremlyn, J. Chem. Soc. C, 1969, 1344.

Les chlorures d'halogénoalcoxybenzènesulfonyle (III, $R'_5$ = ω -halogénoalcoxy) sont utilisés pour la préparation de composés selon l'invention dans lesquels le substituant $R_5$ est un ω -aminoalcoxy non substitué ou substitué par un ou deux alkyles, selon le schéma suivant:

$$-O-Alk'-Hal + NHAA' \rightarrow -O-Alk'-NAA'$$

dans lequel -Alk' représente un alkyle en $C_2$-$C_7$

- A et A' représentent indépendamment l'hydrogène ou un alkyle en $C_1$-$C_7$.

Pour certaines valeurs des substituants $R_1$, $R_2$, $R_5$ et/ou $R_6$ les composés selon l'invention (I) peuvent être préparés à partir d'un précurseur de formule (I)' substitué par un groupe $R'_1$, $R'_2$, $R'_5$ et/ou $R_{VI}$ appelé groupe précurseur de $R_1$, $R_2$, $R_5$ et/ou $R_6$ en utilisant des méthodes connues de l'homme de l'art.

Dans la description qui va suivre, on expose la préparation des composés de formule (I) portant des substituants $R_1$ et/ou $R_5$ ; les mêmes méthodes s'appliquent à la préparation des composés dans lesquels les substituants $R_2$ et/ou $R_5$ ont les valeurs indiquées pour $R_1$ et $R_5$.

Les composés (I) dans lesquels $R_1$ et/ou $R_5$ est un hydroxyle peuvent être obtenus par hydrogénation catalytique, par exemple en présence de palladium sur charbon, d'un composé de formule (I)' dans lequel $R'_1$ et/ou $R'_5$ est un benzyloxy. Ces composés peuvent également être préparés à partir de composé analogues de formule (I)' dans lesquels $R'_1$ et/ou $R'_5$ représente un groupe amino en utilisant la méthode décrite dans J. Org. Chem., 1977, 42, 2053.

Les composés de formule (I) dans lesquels $R_1$ et/ou $R_5$ représente un alcoxy en $C_1$-$C_7$ peuvent être préparés directement par le procédé selon l'invention à partir des composés de formule (II) et (III) correctement substitués.

Les composés (I)' dans lesquels $R'_1$ et/ou $R'_5$ représente un hydroxyle permettent également de préparer des composés (I) dans lesquels $R_1$ et/ou $R_5$ est un alcoxy en $C_1$-$C_7$ par action d'un halogénure d'alkyle en $C_1$-$C_7$ en présence d'une base telle qu'un hydrure métallique ou un carbonate alcalin ou alcalino-terreux comme $K_2CO_3$ ou $Cs_2CO_3$ dans un solvant tel que le THF ou le DMF. De même, on prépare les composés de formule (I) dans lesquels $R_1$ et/ou $R_5$ représente un ω-aminoalkyloxy par action d'une ω-chloroalkylamine sur les

composés dans lesquels $R'_1$ et/ou $R'_5$ = OH ; de même on prépare les composés dans lesquels $R_1$ et/ou $R_5$ représente un ω-hydroxyalcoxy par action d'un chloroalkylalcool ; dans le cas particulier de la préparation d'un composé (I) dans lequel $R_1$ et/ou $R_5$ = $O(CH_2)_2OH$, on peut également faire agir le carbonate d'éthylène sur un composé (I) 'dans lequel $R'_1$ et/ou $R'_5$ = OH.

Les composés de formule (I) dans lesquels $R_1$ et/ou $R_5$ représente un ($C_1$-$C_6$)alkylcarbonyloxy ou un formyloxy sont obtenus respectivement par action d'un halogènure d'acide ou d'un anhydride sur un composé (I)' dans lequel $R'_1$ et/ou $R'_5$ est un hydroxyle, ou par exemple, par action d'acide formique en présence de didicyclohexylcarbodiimide (J. HUANG et al., J. Chem. Res. (S), 1991, 292-293).

Les composés de formule (I) dans lequel $R_5$ est un groupe $OR_7$, $R_7$ représentant un ω-carbamoylalkyle en $C_1$-$C_7$ libre ou substitué par un ou deux alkyles en $C_1$-$C_7$ peuvent être préparés à partir d'un composé (I )' dans lequel $R'_5$ représente un groupe $OR_V$, $R_V$ représentant un ω-carboxyalkyle en $C_1$-$C_7$ estérifié par un alkyle en $C_1$-$C_7$. Cette préparation est effectuée de façon classique pour l'homme de l'art par action d'une amine correctement choisie.

Pour préparer des composés de formule (I) dans lesquels $R_1$ et/ou $R_5$ représente un ($C_1$-$C_7$)monoalkylamino, on fait réagir un composé de formule (I)' dans lequel $R'_1$ et/ou $R'_5$ représente un groupe amino avec un aldéhyde ou une cétone, en milieu acide,en présence d'un agent réducteur tel que le cyanoborohydrure de sodium ; par une réaction identique, on prépare les composés (I) dans lesquels $R_1$ et/ou $R_5$ représente un dialkylamino.

On peut préparer les composés de formule (I) dans lesquels $R_5$ représente un groupe amino substitué par un benzyle, lui-même éventuellement substitué, ou par un alcène en $C_3$-$C_8$ par action d'un chlorure de benzyle ou d'un chloroalcène en $C_3$-$C_8$ sur un composé de formule (I)' dans lequel $R'_5$ est un groupe amino ou alkylamino.

Les composés de formule (I) dans lesquels $R_5$ représente un groupe Δ3-pyrrolin-1-yle, se préparent par action, sous atmosphère inerte, du cis-1,4-dichlorobut-2-ène sur les composés de formule (I)' dans lesquels $R'_5$ est un groupe amino en présence d'une base comme la triéthylamine. Par hydrogénation on prépare ensuite les composés de formule (I) dans lesquels $R_5$ est un groupe pyrrolidin-1-yle. La réaction du cis-1,4-dichlorobut-2-ène avec les composés (I)' dans lesquels $R'_5$ est un groupe amino, peut également s'effectuer à l'air en présence d'une base comme le carbonate de sodium, et conduit, dans ces conditions, à la formation d'un mélange d'un composé de formule (I) dans lequel $R_5$ représente un Δ3-pyrrolin-1-yle et d'un composé de formule (I) dans lequel $R_5$ représente un groupe pyrrol-1-yle que l'on peut séparer par chromatographie.

Les composés de formule (I) dans lesquels $R_5$ représente un groupe isoindolin-2-yle se préparent par action de l'α,α'-dibromo-o-xylène sur les composés de formule (I)' dans lesquels $R'_5$ est un groupe amino, en présence d'une base telle que la triéthylamine, et dans un solvant tel que le diméthylformamide à reflux.

Les composés de formule (I) dans lesquels $R_5$ représente un groupe 1-méthyl-2,4-dioxoimidazolin-3-yle ($NR_5R_9$ = N-méthylhydantoïne) se préparent en deux étapes : on fait réagir la sarcosine sur un composé de formule (I)' dans lequel $R'_5$ représente un phénoxycarboxamido, en présence d'une base telle que la triéthylamine, pour obtenir un composé de formule (I)' dans lequel $R'_5$ représente un N'-carboxyméthyl-N'-méthyluréido, puis par chauffage à 100°C, sous vide, le produit obtenu précédemment se cyclise.

De la même façon, on prépare un composé de formule (I) dans laquelle $R_5$ représente un groupe 2,4-dioxoimidazolin-3-yle ($NR_5R_9$) = hydantoine) par action de la glycine sur un composé de formule (I)' tel que défini ci-dessus.

Lorsque $R'_1$ et/ou $R'_5$ représente un amino, on peut également effectuer une nitrosation, par exemple en présence d'acide nitreux ou de nitrite de sodium pour préparer un composé (I)' dans lequel $R'_1$ et/ou $R'_5$ représente un sel de diazonium ; par des réactions connues de l'homme de l'art, on accède alors aux composés (I) selon l'invention dans lesquels $R_1$ et/ou $R_5$ est un cyano, un halogéno ou un thioalkyle en $C_1$-$C_7$. Enfin par des réactions classiques à partir de composés (I)' dans lesquels $R'_1$ et/ou $R'_5$ = $NH_2$, on peut préparer des composés (I) dans lesquels $R_1$ et/ou $R_5$ représente l'un des groupes de formule, RCONH-, ROCONH-, RNHCONH- ou $RSO_2NH$-, dans lesquels R représente un alkyle en $C_1$-$C_7$, un phényle ou un benzyle.

Les composés de formule (I) dans lesquels $R_5$ représente un alcoxycarbonyle en $C_1$-$C_7$ peuvent se préparer directement par le procédé selon l'invention. Par des méthodes connues de l'homme de l'art, ils permettent d'obtenir les composés de formule (I) dans lesquels $R_5$ est un groupe carboxy.

Les composés de formule (I)' dans lesquels $R'_5$ est un benzyloxycarbonyle permettent également d'obtenir par hydrogénation catalytique les composés (I) dans lesquels $R_5$ est un carboxyle. Par action d'un halogénure de thionyle, on obtient les composés de formule (I)' dans lesquels $R'_5$ est un halogénocarbonyle. A partir de tels composés, on prépare des composés de formule (I) dans lesquels $R_5$ est un carbamoyle substitué par $R'_6$ et R" par action d'un composé $HNR'_6R''_6$. On peut également utiliser les composés de formule (I)' dans lesquels le substituant $R'_5$ est un phénoxycarbonyle pour obtenir les composés (I) dans lesquels $R_5$ est un phénylcarbamoyle ou un alkylcarbamoyle en $C_1$-$C_7$ par action d'une aniline ou d'une alkylamine en $C_1$-$C_7$. Une

aniline substituée sur le phényle ou, respectivement, une alkylamine substituée sur l'alkyle permettent d'obtenir des composés de formule (I) dans lesquels $R_5$ est un phénylcarbamoyle substitué sur le phényle ou, respectivement, un alkylcarbamoyle substitué sur l'alkyle par $R'''_6$.

De la même façon, les composés de formule (I) dans lesquels $R_5$ est un groupe $CONR_8CR_{10}R'_{10}COR_{12}$ sont préparés à partir de composés de formule (I)' dans lesquels $R'_5$ représente soit un groupe COCl, soit un groupe phénoxycarbonyle, par action de $H_2NCR_{10}R'_{10}COR_{12}$.

Les composés de formule (I) dans lesquels $R_5$ est un groupe $COR'_7$ sont préparés à partir de composés (I)' correspondants dans lesquels $R'_5$ est un phénoxycarbonyle, par action de $R'_7H$.

Un composé (I)' dans lequel $R'_5$ est un groupement nitro permet d'obtenir par hydrogénation catalytique, par exemple en présence d'oxyde de platine, de nickel de Raney®, ou de palladium sur charbon, ou par réduction chimique, par exemple en présence d'étain ou de fer en milieu acide, un composé (I) dans lequel $R_5$ est un groupement amino; on peut ensuite préparer d'autres composés dans lesquels le groupe amino est substitué en utilisant des réactions bien connues de l'homme de l'art.

Par exemple lorsque l'on souhaite obtenir un composé (I) selon l'invention dans lequel $R_5$ est un groupe $NR_5R_9$, $R_9$ étant un benzoyle éventuellement substitué, on fait agir sur un composé (I)' dans lequel $R'_5$ est un groupe amino, le chlorure de benzoyle dans lequel le phényle porte le substituant approprié, en présence d'une amine comme la triéthylamine. On peut, par exemple, faire agir le chlorure de 4-chlorosulfonylbenzoyle pour préparer un composé (I)' dans lequel $R'_5$ est un groupe 4-chlorosulfonylbenzamido, puis par action de l'ammoniac ou, respectivement, d'une alkylamine en $C_1$-$C_4$, on obtient un composé (I) dans lequel le substituant $R_5$ est un groupe 4-sulfamoylbenzamido ou, respectivement, un groupe 4-alkylsulfamoylbenzamido. De la même façon, on fait agir le chlorure d'acide $R_{11}R'_{11}NCR_{10}R'_{10}COCl$ sur un composé de formule (I)' dans lequel $R'_5$ est un groupe $NHR_5$ pour préparer un composé de formule (I) dans lequel $R_5$ est un $NR_8$ substitué par $COCR_{10}R'_{10}NR_{11}R'_{11}$.

Lorsque l'on souhaite préparer un composé (I) dans lequel $R_5$ est un groupe $NR_8R_9$, $R_9$ étant respectivement un ($C_1$-$C_6$)alkylcarbonyle ou un formyle, on fait agir sur un composé (I)' dans lequel $R'_5$ est un groupe amino, l'anhydride approprié ou le chlorure d'acide approprié, ou, respectivement, l'acide formique en présence d'anhydride acétique, en présence d'une amine comme la triéthylamine.

Selon un autre exemple de préparation, un composé (I) dans lequel $R_5$ est un groupe alkylsulfonamido est obtenu par action d'un halogénure d'alkylsulfonyle sur un composé (I)' dans lequel $R'_5$ est un groupe amino.

Les composés de formule (I)' dans lesquels $R'_5$ est un groupe amino sont également utiles pour la préparation de composés dans lequel ce groupe amino est substitué par un groupe $(CH_2)_t$-$COR_{12}$. Dans ce cas, on fait agir sur (I)' en présence de chlorure cuivreux, un composé de formule Hal-$(CH_2)_t$-COOAlk dans lequel Hal représente un halogène, par exemple le brome et Alk représente un alkyle en $C_1$-$C_7$ ; le cas échéant, on transforme l'ester ainsi obtenu en acide ou en amide. On peut utiliser l'action d'un anhydride, tel que l'anhydride succinique ou l'anhydride glutarique, sur un composé (I)' dans lequel $R'_5$ est un amino pour préparer un composé (I) dans lequel $R_5$ = $NHCO(CH_2)_{t'}CO_2H$ avec t' = 2 ou 3. Le cas échéant on transforme l'acide ainsi obtenu en ester ou en amide.

On peut également utiliser l'action du chlorure d'éthyloxalyle ou, respectivement, du chlorure d'éthyloxalyle sur un composé (I)' dans lequel $R'_5$ est un amino pour préparer un composé (I) dans lequel $R_5$ est un groupe $-NHCOCO_2Et$ ou $-NHCOCH_2CO_2Et$.

De la même façon, les composés de formule (I) dans lesquels $R_5$ est un groupe amino substitué par un groupe $CR_{10}R'_{10}COR_{12}$ sont préparés par action d'un composé de formule Hal-$CR_{10}R'_{10}COR_{12}$ sur les composés (I)' correspondants dans lequel le substituant $R'_5$ est un amino.

Par action d'un chloroformiate d'alkyle en $C_1$-$C_7$ ou de phényle sur un composé (I)' dont le substituant $R'_5$ est un amino, on prépare un composé (I) dans lequel $R_5$ est un groupe amino substitué par un alcoxycarbonyle en $C_1$-$C_7$ ou un phénoxycarbonyle.

De même, par action d'un chlorure de phénoxythiocarbonyle sur un composé de formule (I)' dans lequel $R'_5$ est un groupe amino, on obtient un composé de formule (I) dans lequel $R_5$ est un phénoxythiocarbonylamino.

Par action de l'ammoniac sur un composé de formule (I)' dans lequel $R'_5$ est un groupe amino substitué par un phénoxycarbonyle ou un phénoxythiocarbonyle, on prépare un composé de formule (I) dans lequel $R_5$ est un uréido ou un thiouréido; par action sur un tel composé de formule (I)' d'une aniline correctement substituée ou d'une mono ou dialkylamine en $C_1$-$C_7$ correctement substituées, on prépare un composé de formule (I) dans lequel $R_5$ est un N'-phényluréido correctement substitué, un N'-alkyluréido ou N',N'-dialkyluréido dans lesquels l'alkyle est en $C_1$-$C_7$ correctement substitués.

On peut également préparer un composé (I) dans lequel $R_5$ est un uréido ($-NHCONR_{14}R'_{14}$) ou respectivement, un thioureido ($-NHCSNR_{14}R'_{14}$) par action d'un composé $NHR_{14}R'_{14}$, sur un composé (I)' dans lequel $R'_5$ est un groupe phénoxycarbonylamino ou, respectivement, phénoxythiocarbonylamino.

On peut aussi préparer un composé (I) dans lequel $R_5$ est un uréido ($-NHCONR_{14}R'_{14}$) ou respectivement un thiouréido par action d'un chlorure de carbamoyle ($ClCONR_{14}R'_{14}$), ou respectivement d'un chlorure de thiocarbamoyle, sur un composé de formule (I)' dans lequel $R'_5$ est un groupe amino.

On peut encore préparer un composé (I) dans lequel $R_5$ est un thiouréido par action du réactif de lawesson sur un composé (I)' dans lequel $R'_5$ est l'uréido correspondant.

Les composés (I) dans lesquels $R_5$ est un groupe guanidino non substitué ou substitué une ou deux fois par un alkyle en $C_1$-$C_7$, un phényle ou un benzyle peuvent être préparés à partir des composés (I)' dans lesquels $R'_5$ est un groupe phénoxyamido par action du cyanamide ou d'un de ses dérivés correctement substitué sur l'azote.

Par action d'une amine appropriée en présence de phosgène sur un composé (I)' dont le substituant $R_5$' est un amino, on prépare un composé (I) dans lequel $R_5$ est un carbamoyle non substitué ou substitué par un ou 2 groupes alkyles en $C_1$-$C_7$.

On peut également préparer un composé (I) dans lequel $R_5$ est un groupe amino substitué par un alkylcarbamoyle ou par un phénylcarbamoyle par action d'un isocyanate d'alkyle ou de phényle sur un composé (I)' dont le substituant $R'_5$ est un amino.

Par ailleurs, un composé (I) dans lequel $R_5$ est un groupe sulfamoyle non substitué ou substitué par un alkyle en $C_1$-$C_7$ est préparé par action de l'ammoniac ou d'une alkylamine en $C_1$-$C_7$ sur un composé (I)' dans lequel $R'_5$ est un groupe halogénosulfonyle.

L'affinité des composés selon l'invention pour les récepteurs de la vasopressine a été déterminée <u>in vitro</u> en utilisant la méthode décrite dans C. J. Lynch et al., J. Biol. Chem., 1985, <u>260</u> (5), 2844-2851. Cette méthode consiste à étudier le déplacement de la vasopressine tritiée fixée aux sites $V_1$ de membranes de foie de rats. Les concentrations inhibitrices de 50 % ($CI_{50}$) de la fixation de la vasopressine tritiée des composés selon l'invention sont faibles, allant jusqu'à $10^{-7}$M.

L'affinité des composés (I) selon l'invention pour les récepteurs $V_2$ a été mesurée sur une préparation membranaire de rein de boeuf selon une méthode adaptée de P. Crause et al., Molecular and Cellular Endocrinology, 1982, <u>28</u>, 529-541 et de F.L. Stassen et al., J. Pharmacol. Exp. Ther., 1982, <u>223</u>, 50-54. Les composés selon l'invention inhibent la fixation de l'arginine-vasopressine tritiée aux récepteurs de la préparation membranaire. Les $CI_{50}$ des composés selon l'invention sont faibles, allant jusqu'à $10^{-9}$M.

L'activité antagoniste des récepteurs $V_2$ des composés selon l'invention a été montrée par le test de dosage de l'activité adénylate cyclase effectué selon une méthode adaptée de M. Laburthe et al., Molecular Pharmacol., 1986, <u>29</u>, 23-27. On utilise une préparation membranaire de rein de boeuf et chaque produit est incubé 10 minutes à 37°C, seul ou en présence d'AVP (arginine vasopressine) à la concentration de $3.10^{-8}$M. L'AMP cyclique (adénosine monophosphate cyclique) produite est mesurée par dosage radioimmunologique. On détermine la concentration inhibant de 50 %($CI_{50}$) la stimulation de l'adénylate cyclase induite par $3.10^{-8}$M d'AVP. Les $CI_{50}$ déterminées sont de l'ordre de $10^{-7}$M, allant jusqu'à $10^{-8}$M.

L'activité agoniste ou antagoniste des récepteurs de la vasopressine des composés selon l'invention, administrés par voie orale, est évaluée chez le rat (Souche OFA, Sprague-Dawley) en surcharge hydrique, traité à la vasopressine. L'activité antagoniste des composés, selon l'invention a été également évaluée chez le rat normalement hydraté (souche OFA ou souche Sprague-Dawley) selon la technique décrite dans Br. J. Pharmacol., 1992, <u>105</u>, 787-791. L'effet diurétique a été observé pour certains composés à la dose de 10 mg/kg.

De même, l'affinité des composés (I) selon l'invention pour les récepteurs de l'ocytocine a été déterminée <u>in vitro</u> par déplacement d'un analogue radioiodé de l'ocytocine fixé aux récepteurs d'une préparation membranaire de glandes mammaires de rattes gestantes, selon une technique proche de celle décrite par J. Eland et al. dans Eur. J. Pharmacol., 1987, <u>147</u>, 197-207. Les $CI_{50}$ des composés selon l'invention atteignent $10^{-8}$M.

Les composés selon l'invention sont actifs après administration par différentes voies, notamment par voie orale.

Aucun signe de toxicité n'est observé avec ces composés aux doses pharmacologiquement actives.

Ainsi les composés selon l'invention peuvent être utilisés dans le traitement ou la prévention de différentes affections vasopressine-dépendantes ou ocytine-dépendantes, les affections cardiovasculaires, comme l'hypertension, l'hypertension pulmonaire, l'insuffisance cardiaque, l'infarctus de myocarde, ou la vasospasme coronaire, en particulier chez le fumeur, les angines instables et PTCA (percutaneous transluminal coronary angioplasty), l'ischémie cardiaque, les dérèglements de l'hémostase notamment l'hémophilie, le syndrome de Von Willebrand ; les affections du système nerveux central, la migraine, le vasospasme cérébral, l'hémorragie cérébrale, les oedèmes cérébraux, la dépression, l'anxiété, les états psychotiques, les troubles de la mémoire par exemple ; les affections du système rénal comme les oedèmes, le vasospasme rénal, la nécrose du cortex rénal, l'hyponatriémie, l'hypokaliémie, le syndrome de Schwartz Bartter ; les affections du système gastrique comme le vasospasme gastrique, l'hépatocirrhose, les ulcères, la pathologie des vomissements, par exemple la nausée y compris la nausée due à une chimiothérapie, le mal des transports, ou encore le syndrome de la

sécrétion inappropriée de l'hormone antidiurétique (SIADH), le diabète insipide et l'énurésie. Les composés selon l'invention peuvent également être utilisés dans le traitement des troubles du comportement sexuel ; chez la femme, les composés selon l'invention peuvent être utilisés pour traiter la disménorrhée ou le travail prématuré. On peut également utiliser les composés selon l'invention dans le traitement des cancers pulmonaires à petites cellules, des encéphalopathies hyponatriémique, de la maladie de Raynaud, le syndrome pulmonaire, le glaucome et dans les traitements post-opératoires, notamment après une chirurgie abdominale.

La présente invention a également pour objet des compositions pharmaceutiques contenant une dose efficace d'un composé selon l'invention ou d'un sel pharmaceutiquement acceptable et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique ou rectale, les principes actifs de formule (I) ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule (I) ci-dessus ou d'un des sels pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention.

Ainsi, selon la présente invention, on peut préparer des compositions pharmaceutiques contenant un composé selon l'invention associé à un composé agissant sur le système rénine-angiotensine tel qu' un inhibiteur de l'enzyme de conversion, un antagoniste de l'angiotensine II, un inhibiteur de la rénine. On peut également associer un composé selon l'invention, par exemple, avec un vasodilatateur périphérique, un inhibiteur calcique, un béta-bloquant, un alpha-1-bloquant ou un diurétique. De telles compositions seront utiles en particulier dans le traitement de l'hypertension ou de la défaillance cardiaque.

On peut également associer deux composés selon l'invention : un antagoniste spécifique du récepteur $V_1$ à un antagoniste spécifique du récepteur $V_2$ ou bien un antagoniste spécifique du récepteur $V_1$ à un antagoniste

spécifique de l'ocytocine.

Ces associations permettront de renforcer les activités thérapeutiques des composés selon l'invention.

Préparation des 1,3-dihydro-indol-2-one

Préparation 1 :

1,3-Dihydro-4,6-diméthyl-3-spirocyclohexane indol-2-one.

Ce composé est préparé selon Moore et Plant dans J. Chem. Soc., 1951, 3475.

On maintient à reflux sous atmosphère inerte un mélange contenant 15 ml de quinoléine et 10 g d'oxyde de calcium et l'on ajoute en 30 minutes 5 g de 3,5-diméthylphénylhydrazide de l'acide cyclohexane carboxylique (IV, $R'_1$, $R'_2$ = $CH_3$, $CR'_3R'_4$ = cyclohexane). Le milieu réactionnel est refroidi puis versé dans un mélange glace-acide chlorhydrique. On extrait par de l'acétate d'éthyle, lave par de l'acide chlorhydrique normal, par de l'eau jusqu'à neutralité, puis on sèche et concentre sous vide pour obtenir un solide brun. Par trituration dans l'éther iso, on obtient le composé attendu. F = 223°C

En opérant de la même façon et en faisant varier l'hydrazide de départ, on obtient les dérivés 1,3-dihydro-indol-2-one décrits dans le tableau 1 ci-dessous.

Ces composés sont purifiés par chromatographie sur colonne de silice, en éluant par du DCM ou par chromatographie sur colonne d'alumine en éluant par du DCM ou par de l'éther iso.

TABLEAU 1

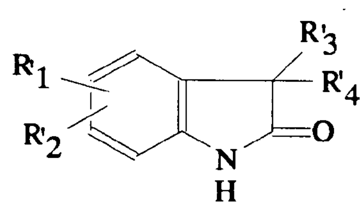

| $R'_1$ | $R'_2$ | $CR'_3R'_4$ | F °C |
|---|---|---|---|
| Cl-5 | H | cyclobutane | 191 |
| Cl-5 | H | cyclopentane | 189 |
| Cl-5 | H | cyclohexane | 186 |
| H | H | cyclohexane | 123-124 |
| $CH_3$-5 | H | cyclohexane | 164 |
| $CH_3O$-5 | H | cyclohexane | 226 |
| Cl-6 | H | cyclohexane | 168 |
| $CF_3O$-5 | H | cyclohexane | 164 |
| $C_6H_5O$-5 | H | cyclohexane | 160 |

Préparation 2 :

La 1,3-dihydro-3-spirocyclohexaneindol-2-one décrite dans le tableau 1 ci-dessus peut également être obtenue par alkylation de l'indol-2-one en utilisant le procédé selon A.S. Kende et J.C. Hodges ou une variante décrite ci-après.

On maintient à -40°C, sous atmosphère d'azote, une solution de 30 g de 1,3-dihydro-indol-2-one dans 900 ml de THF et l'on ajoute 101 g de *tert*-butylate de potassium. On laisse remonter la température à 0°C en 1 heure, puis on refroidit à -60°C et l'on ajoute goutte à goutte une solution de 52 g de 1,5-dibromopentane dans

50 ml de THF. Après 30 minutes à -60°C, on laisse remonter à TA, puis on ajoute 30 ml d'eau et évapore le solvant sous pression réduite. Le résidu est repris par 500 ml de DCM et 200 ml d'eau puis on filtre l'insoluble et sépare la phase organique qui est lavée avec 100 ml d'eau, séchée sur sulfate de magnésium et évaporée sous vide. Le résidu est chromatographié sur silice en éluant par un mélange cyclohexane-éther. On obtient le composé attendu qui est recristallisé dans l'heptane. m = 34 g, F = 123-124°C.

A partir d'autres 1,3-dihydro-indol-2-one et d'autres agents alkylants, on peut appliquer un mode opératoire semblable.

A titre d'exemple, parmi les composés de départ de formule (VII), la 5-chloro-1,3-dihydro-indol-2-one est décrite par Wright dans J. Am. Chem. Soc., 1956, 78, 221 et par Rajanbabu dans J. Org. Chem., 1986, 51, 1704. La 4-chloro-1,3-dihydro-indol-2-one peut être préparée à partir du 2-chloro-6-nitrotoluène, selon la méthode décrite dans J. Am. Chem. Soc., 1956, 78, 221.

La 1,3-dihydro-5-méthoxyindol-2-one est préparée à partir de la 4-méthoxyaniline selon la méthode décrite dans J. Am. Chem. Soc., 1974, 96, 5512. De la même manière à partir du dérivé aniline approprié, on prépare diverses 1,3-dihydro-indol-2-one :

Préparation 3

5-Ethoxy-1,3-dihydro-indol-2-one :

A - 3-Méthylthio-5-éthoxy-1,3-dihydro-indol-2-one.

A une solution refroidie vers -70°C de 12,5 g de chlore dans 400 ml de DCM on ajoute 23,6 g de méthylthio acétate d'éthyle dans 60 ml de DCM. Après 5 minutes d'agitation à cette même température on additionne une solution de 4-éthoxyaniline (48,3 g) dans 120 ml de DCM. On agite une heure vers -70°C, ajoute 39,3 ml de triéthylamine et laisse remonter à température ambiante. On ajoute 200 ml d'eau, décante la phase organique qui est séchée sur sulfate de magnésium et évaporée sous pression réduite. Le résidu est repris dans 500 ml d'isopropanol et 20 ml d'acide chlorhydrique concentré. On agite environ 16 heures à température ambiante, filtre et sépare le précipité. Le filtrat est concentré sous pression réduite pour donner le produit attendu.

B - 5-Ethoxy-1,3-dihydro-indol-2-one :

Le solide précédent dans 1500 ml d'isopropanol est déthiométhylé en présence de 100 g de nickel de Raney® (80 à 100 $m^2$ par g)à reflux durant 3 heures sous atmosphère d'azote. On filtre sur talc, rince avec 1000 ml d'isopropanol et concentre le filtrat sous pression réduite. On isole après recristallisation dans le toluène 16 g du produit attendu. F= 156°C

De la même manière, à partir des anilines correspondantes on isole:

5-benzyloxy-1,3-dihydro-indol-2-one F = 152°C
5-n-propyl-1,3-dihydro-indol-2-one F = 136°C
5-éthyl-1,3-dihydro-indol-2-one F = 152°C
5-(2,2,2-trifluoroéthoxy)-1,3-dihydro-indol-2-one F = 145°C
5-trifluorométhyl-1,3-dihydro-indol-2-one F = 193°C
5-fluoro-1,3-dihydro-indol-2-one F = 143°C.

En opérant selon la technique décrite dans la préparation 2 et en faisant varier le dérivé 1,3-dihydro-indol-2-one de départ et le réactif alkylant, on obtient les composés de formule (II) décrits ci-dessous.

TABLEAU 2

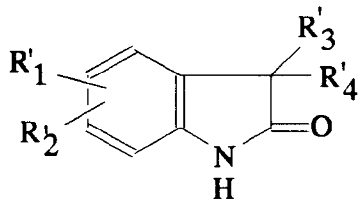

(II)

| $R'_1$ | $R'_2$ | $CR'_3R'_4$ | F °C | Réactif alkylant |
|---|---|---|---|---|
| Cl-5 | H | cyclohexane | 186-189 | $Br(CH_2)_5Br$ |
| Cl-5 | H | cycloheptane | 202 | $Br(CH_2)_6Br$ |
| Cl-5 | H | 4,4-diméthyl cyclohexane | 180 | $TsO(CH_2)_2C(CH_3)_2$- -$(CH_2)_2OTs$ |
| Cl-5 | H | hexahydroindane-2 | 223 | cis-diiodométhyl-1,2 cyclohexane |
| $CH_3O$-5 | H | 4,4-diméthyl cyclohexane | 202 | $TsO(CH_2)_2C(CH_3)_2$- -$(CH_2)_2$-OTs |
| Cl-5 | H | indane-2 | 228 | α,α'-dibromométhyl orthoxylène |
| Cl-5 | H | $C(CH_3)_2$ | 160 | $CH_3I$ |
| Cl-5 | H | $C(CH_2CH_3)_2$ | 156 | $CH_3CH_2I$ |
| Cl-5 | H | C(n Pr)$_2$ | 158 | nPrI |
| Cl-5 | H | C(iBu)$_2$ | 164 | iBuI |
| Cl-5 | H | N-méthyl pipéridine-4 | 260 | $Cl(CH_2)_2N(CH_3)$- -$(CH_2)_2Cl$ |
| Cl-5 | H | tétrahydropyranne-4 | 223 | $I(CH_2)_2O(CH_2)_2I$ |
| Cl-4 | H | cyclohexane | 215 | $Br(CH_2)_5Br$ |

TABLEAU 2 (suite)

| R'1 | R'2 | CR'3R'4 | F°C | Réactif alkylant |
|---|---|---|---|---|
| BzO-5 | H | cyclohexane | 162 | $Br(CH_2)_5Br$ |
| H | H | $C(CH_2C_6H_5)_2$ | 206 | $C_6H_5CH_2Br$ |
| Cl-5 | H | C(n-pentyl)$_2$ | 142 | $CH_3(CH_2)_4Br$ |
| Cl-5 | H | 2,3-dihydro phénalène-2 | - | 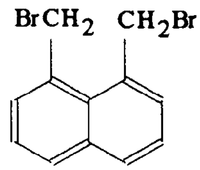 |
| BzO-5 | H | 4,4-diméthyl cyclohexane | 154 | $TsO(CH_2)_2C(CH_3)_2$- -$(CH_2)_2OTs$ |
| Cl-5 | H | 4-spirocyclopentane cyclohexane | 202 | 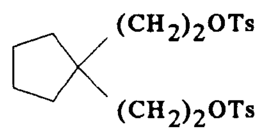 |
| nPr-5 | H | cyclohexane | 151 | $Br(CH_2)_5Br$ |
| EtO-5 | H | N-tBu pipéridine-4 | - | 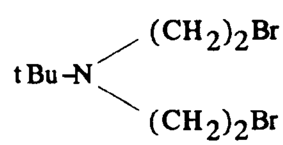 |
| Cl-5 | H | N-Bz pipéridine-4 | 165 | 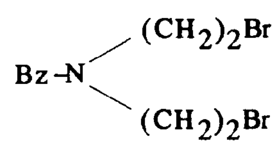 |
| Cl-5 | H | N-phényl pipéridine-4 | 188 | 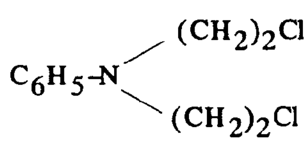 |

TABLEAU 2 (suite)

| R'1 | R'2 | CR'3R'4 | F°C | Réactif alkylant |
|---|---|---|---|---|
| Cl-5 | H | 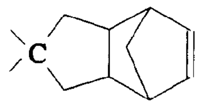 | 300 | 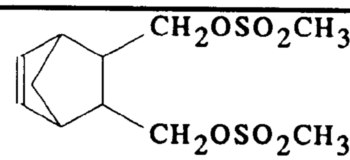 |
| EtO-5 | H | 4,4-diéthyl cyclohexane | 132 | $TsO(CH_2)_2C(C_2H_5)_2$ $-(CH_2)_2OTs$ |
| EtO-5 | H | cyclohexane | 163 | $Br(CH_2)_5Br$ |
| EtO-5 | H | 4,4-diméthyl cyclohexane | 178 | $TsO(CH_2)_2C(CH_3)_2-$ $-(CH_2)_2OTs$ |
| EtO-5 | H | cycloheptane | 139 | $Br(CH_2)_6Br$ |
| Et-5 | H | 4,4-diméthyl cyclohexane | 160 | $TsO(CH_2)_2C(CH_3)_2-$ $-(CH_2)_2OTs$ |
| $CF_3CH_2O$ -5 | H | 4,4-diméthyl cyclohexane | 164 | idem |
| H | H | 4,4-diméthyl cyclohexane | 169 | idem |
| $CF_3$-5 | H | 4,4-diméthyl cyclohexane | 211 | idem |
| F-5 | H | 4,4-diméthyl cyclohexane | 171 | idem |
| Cl-5 | H | 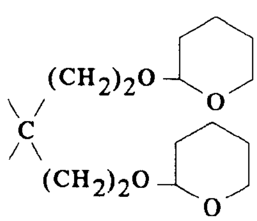 | 120 | 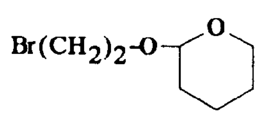 |

TABLEAU 2 (suite)

| R'$_1$ | R'$_2$ | CR'$_3$R'$_4$ | F°C | Réactif alkylant |
|---|---|---|---|---|
| EtO-5 | H | 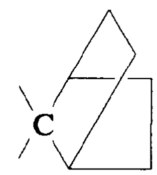 | 208 | 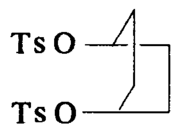 |
| EtO-5 | H | 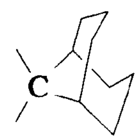 | 214 | 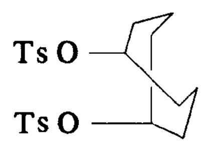 |
| EtO-5 | H | tétrahydro-pyrane-4 | 146 | $I(CH_2)_2O(CH_2)_2I$ |
| EtO-5 | H | 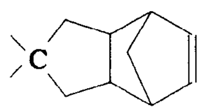 | 255 | 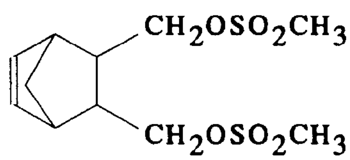 |

Préparation 4 :

1,3-Dihydro-3-spiroadamantaneindol-2-one.

Ce composé est préparé selon I. Fleming et al., Tetrahedron Letters, 1982, 2053-2056 à partir de 2-bromoaniline et d'adamantane-2-one.

Préparation 5 :

5-chloro-1,3-dihydro-3,3-diphénylindol-2-one.

Ce composé est préparé selon la méthode décrite dans Helv. Chim. Acta, 1946, 29, 415-431, par action du benzène sur la 5-chloroisatine en présence de chlorure d'aluminium. F = 281°C.

Préparation 6 :

1,3-Dihydro-5-nitro-3-spirocyclohexaneindol-2-one.

Ce composé est préparé selon la méthode décrite dans J. Am. Chem. Soc., 1945, 67, 499 par nitration de la 1,3-dihydro-3-spirocyclohexaneindol-2-one. F = 192°C.

De la même manière, à partir de 1,3-dihydro-3-spiroadamantaneindol-2-one, on prépare la 1,3-dihydro-5-nitro-3-spiroadamantaneindol-2-one. F > 260°C

On prépare également la 1,3-dihydro-5-nitro-3-spiro(4,4-diméthylcyclohexane)-indol-2-one. F = 195°C

Préparation 7 :

5-amino-1,3-dihydro-3-spirocyclohexaneindol-2-one.

Ce composé est préparé selon la méthode décrite dans J. Chem. Soc., 1951, 3475, par réduction de la 1,3-dihydro-5-nitro-3-spirocyclohexaneindol-2-one, préparée précédemment.F = 176°C.

De la même manière, on prépare la 5-amino-1,3-dihydro-3-spiroadamantaneindol-2-one. Fc = 245°C

Préparation 8 :

5-fluoro-1,3-dihydro-3-spirocyclohexaneindol-2-one.

A - tétrafluoroborate de 5-diazonium-1,3-dihydro-3-spirocyclohexaneindol-2-one.

On refroidit à 0°C, une solution contenant 4 g de 5-aminol,3-dihydro-3-spirocyclohexaneindol-2-one dans 9,2 ml d'acide chlorhydrique 6N et l'on ajoute 2,27 g de nitrite de sodium dans 2,6 ml d'eau puis 2,54 g de tétrafluoroborate de sodium dans 9 ml d'eau. Après 5 minutes sous agitation, on filtre le précipité, lave avec une solution de tétrafluoroborate à 5 %, avec 3 ml de méthanol refroidi vers 0°C, puis avec 5 ml d'éther. Le sel obtenu est séché sous vide à TA en présence d'anhydride phosphorique.

B - 5-fluoro-1,3-dihydro-3-spirocyclohexaneindol-2-one.

1 g du composé obtenu à l'étape A est placé dans 5 ml de xylène et chauffé vers 115°C pendant 2 heures. On refroidit à TA, filtre le précipité, rince au toluène et ajoute au filtrat 0,1 g de charbon actif. Après filtration, le solvant est évaporé sous pression réduite et l'on obtient 0,45 g du composé attendu que l'on recristallise dans du pentane.
F = 114°C.

Préparation 9 :

5-cyano-1,3-dihydro-3-spirocyclohexaneindol-2-one.
On dissout à TA 4,78 g de cyanure de potassium et 4,95 g de cyanure cuivreux dans 40 ml de DMSO. On refroidit vers 15°C et l'on ajoute 4,15 g du sel de diazonium obtenu dans la préparation précédente à l'étape A.
Après 30 minutes d'agitation à TA, on ajoute 100 ml d'eau et 100 ml d'éther puis on sépare la phase organique qui est séchée sur sulfate de magnésium et évaporée sous pression réduite. Le résidu est chromatographié sur silice en éluant avec un mélange cyclohexane-éther. On obtient le composé attendu qui est recristallisé dans l'heptane. m = 1,4 g, F = 216°C.

Préparation 10 :

5-chloro-1,3-dihydro-3-spiroadamantaneindol-2-one.
On dissout 1 g de p-chlorophénylhydrazide de l'acide adamantane-2-carboxylique dans du THF et on ajoute, à -40°C, 2,5 ml de n-butyllithium en solution (1,6 M dans l'hexane). Après 5 minutes d'agitation, on concentre sous vide en maintenant à une température inférieure à 30°C. On ajoute 30 ml de 1,2,3,4-tétraméthybenzène et l'on chauffe à reflux pendant 1 heure. On concentre sous pression réduite, reprend le résidu par de l'acide chlorhydrique normal, extrait à l'éther, lave, sèche et concentre sous vide. L'huile obtenue est chromatographiée sur colonne de silice en éluant par DCM; on obtient 0,3 g du produit attendu sous forme d'une cire que l'on cristallise dans l'éther iso. F = 249°C.

Préparation 11 :

5-chloro-3-cyclohexyl-1,3-dihydro-3-méthylindol-2-one.
On utilise la méthode décrite dans Synth. Commun. 1982, 12 (1), 1-10, pour préparer de façon intermédiaire le 5-chloro-3-cyclohexyl-1,3-dihydro-indol-2-one puis on obtient le composé attendu par action de l'iodure de méthyle.

Préparation 12 :

5-Acétyl-1,3-dihydro-3-spirocyclohexaneindol-2-one.
A une solution refroidie à 5°C de 4 g de 1,3-dihydro-3-spirocyclohexaneindol-2-one dans 35 ml de 1,2-dichloroéthane, on ajoute 2,56 g de chlorure d'acétyle puis 8,25 g de chlorure d'aluminium anhydre. On chauffe 2 heures à reflux, évapore le solvant sous pression réduite, hydrolyse le milieu avec 50 g de glace et extrait à l'acétate d'éthyle.

La phase organique est lavée à l'eau, séchée sur sulfate de magnésium puis évaporée sous pression réduite. On chromatographie le résidu sur une colonne de silice en éluant avec un mélange d'heptane et d'éther éthylique et obtient 3,6 g du produit attendu. F = 192°C.

Préparation 13 :

5-Chloro-1,3-dihydro-3-spiro(4-tétrahydrothiopyrane)indol-2-one.

A. 5-Chloro-1,3-dihydro-3,3-di(2-bromoéthyl)indol-2-one.

On ajoute lentement 7,66 g de brome dans un mélange refroidi vers 0°C de 12,4 g de triphénylphosphine dans 70 ml de DCM puis on ajoute 4,58 g de 5-chloro-1,3-dihydro-3,3-di[2-(tétrahydropyran-2-yloxy)éthyl]indol-2-one décrit dans le tableau 2. Après 16 heures à TA on ajoute 60 ml d'eau, sépare la phase organique qui est lavée avec 60 ml d'eau puis séchée sur sulfate de magnésium et évaporée sous vide. Le résidu est chromatographié sur une colonne de silice en éluant au DCM. On obtient 3,12 g du produit attendu. F = 215°C.

B. 5-Chloro-1,3-dihydro-3-spiro(4-tétrahydrothiopyrane)indol-2-one.

Sous atmosphère inerte, on ajoute 3 g du produit préparé à l'étape A dans 3,2 ml de DMF, 2 g de sulfure de sodium monohydrate et chauffe 2 heures à 50°C. On refroidit à TA, ajoute 6 ml d'eau et extrait au DCM. La phase organique est lavée à l'eau, séchée sur sulfate de magnésium et évaporée sous pression réduite. Le résidu huileux obtenu est purifié par chromatographie sur silice en éluant au DCM. On obtient 2,02 g du composé attendu.

Spectre RMN à 200 MHz dans $CDCl_3$.
8,12 ppm : s : 1 H
7,2 ppm : m : 2 H
6,8 ppm : d : 1 H
3,25 ppm : m : 2 H
2,65 ppm : m : 2 H
2 ppm : m : 4 H

Préparation 14

5-Ethoxy-1,3-dihydro-3-spiro[4-(méthoxyméthyloxy)cyclohexane]indol-2-one.

A- 3-(Méthoxyméthyloxy)pentane-1,5-diol.

On refroidit à 0°C 270 ml d'une solution d'hydrure d'aluminium et de lithium 1 M dans le THF diluée dans 540 ml de THF anhydre et ajoute une solution de 63 g de 3-(méthoxyméthyloxy)glutarate de diéthyle (préparé selon J.L. Gras dans Synthesis, 1985, 74) dans 400 ml de THF. On laisse 16 heures sous agitation à TA, puis refroidit à 0°C et ajoute successivement 9 ml d'eau, 30 ml d'une solution à 15 % de NaOH et 9 ml d'eau. On filtre les sels minéraux et évapore sous vide le filtrat. On obtient 24 g du produit attendu après distillation sous pression réduite, Eb = 125°C sous 1,2 Pa.

B- 3-(méthoxyméthyloxy)-1,5-ditosyloxypentane.

On refroidit à 0°C une solution de 46 g de chlorure de p-toluènesulfonyle, 38 ml de triéthylamine dans 80 ml de THF, ajoute une solution de 18 g du composé obtenu à l'étape précédente dans 100 ml de THF et laisse 16 heures sous agitation à TA. On ajoute 150 ml d'eau au mélange réactionnel, évapore le solvant sous vide, extrait à l'AcOEt et évapore ce dernier sous vide. On reprend l'huile obtenue dans 250 ml d'éther et 200 ml de NaOH 2N et laisse 16 heures sous agitation à TA. Après décantation, on sèchela phase organique sur sulfate de magnésium et évapore sous vide le solvant. On obtient 45 g du produit attendu après cristallisation dans le cyclohexane, F < 50°C.

C- 5-Ethoxy-1,3-dihydro-3-spiro[4-(méthoxyméthyloxy)cyclohexane]indol-2-one.

On prépare ce composé selon le mode opératoire décrit à la Préparation 2 à partir de 5-éthoxy-1,3-dihydroindol-2-one et du composé obtenu à l'étape précédente. On obtient le produit attendu sous forme d'un mélange

d'isomères, F = 98°C.

Préparation 15 :

5-Ethoxy-1,3-dihydro-3-spiro[4-tricyclo[5.2.1.$0^{2,6}$]décane]indol-2-one.

On hydrogène pendant 16 heures, à 40°C et sous une pression de 20 bars, un mélange de 3 g de 5-éthoxy-1,3-dihydro-3-spiro[4-tricyclo[5.2.1.$0^{2,6}$]déc-8-ène]indol-2-one décrit dans le tableau 2, 1,5 g de palladium sur charbon à 10 % dans 160 ml de MeOH. On filtre le catalyseur sur Célite®, lave au MeOH et évapore sous vide le filtrat. On obtient 2,95 g du produit attendu, F = 236°C.

Préparations 16 et 17 :

5-Ethoxy-1,3-dihydro-3-spiro(4-méthoxycyclohexane)indol-2-one, isomère le moins polaire et isomère le plus polaire.

A- 3-Méthoxypentane-1,5-diol

A une solution de 30 g de 3-hydroxyglutarate de diéthyle, 33 ml de 2,6-di-*tert*-butylpyridine dans 500 ml de DCM, on ajoute 25 ml de trifluorométhylsulfonate de méthyle et chauffe à reflux pendant 6 heures. Après refroidissement, on ajoute 500 ml d'une solution d'HCl 0,5 N, décante la phase organique, sèche sur sulfate de magnésium et évapore sous vide le solvant. On reprend le résidu obtenu dans 200 ml de THF anhydre, filtre puis refroidit le filtrat à -5°C. On ajoute alors lentement 160 ml d'une solution d'hydrure d'aluminium et de lithium 1M dans le THF et laisse 16 heures sous agitation en laissant remonter la température à TA. On refroidit le mélange réactionnel à 0°C et ajoute successivement 5,5 ml d'eau, 18 ml d'une solution à 15 % de NaOH et 5,5 ml d'eau. On filtre les sels minéraux et évapore sous vide le filtrat. On obtient le produit attendu après distillation sous pression réduite, Eb = 104°C sous 1,5 Pa.

B- 3-Méthoxy-1,5-ditosyloxypentane

On refroidit à 0°C une solution de 31 g de chlorure de p-toluènesulfonyle, 26 ml de triéthylamine dans 120 ml de THF, ajoute 10 g du composé obtenu à l'étape précédente et laisse 24 heures sous agitation à TA. On ajoute 120 ml d'eau au mélange réactionnel, évapore sous vide le solvant, extrait à l'AcOEt, sèche sur sulfate de magnésium et évapore sous vide le solvant. On reprend l'huile obtenue dans 200 ml d'éther et 200 ml de NaOH 2N et laisse 16 heures sous agitation à TA. Après décantation, on sèche la phase organique sur sulfate de magnésium et évapore sous vide le solvant. On obtient 26 g du produit attendu après cristallisation dans le cyclohexane, F = 58°C.

C- 5-Ethoxy-1,3-dihydro-3-spiro(4-méthoxycyclohexane)indol-2-one, isomère le moins polaire et isomère le plus polaire.

On prépare ces composés selon le mode opératoire décrit à la Préparation 2 à partir de 11,85 g de 5-éthoxy-1,3-dihydroindol-2-one, 34 g de *tert*-butylate de potassium et 26 g du composé obtenu à l'étape précédente. On chromatographie sur silice en éluant par le mélange cyclohexane/AcOEt (80/20 ; v/v). On sépare les deux isomères :

- le moins polaire, : composé de la Préparation 16, F = 173°C ;
- le plus polaire, : composé de la Préparation 17, F = 186°C.

Par ailleurs, en utilisant le mode opératoire décrit dans la partie générale, on a préparé les chlorures de benzènesulfonyle décrits dans le tableau ci-après :

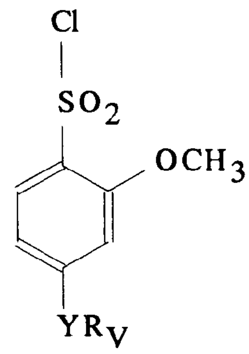

| Y | $R_V$ | F°C |
|---|---|---|
| S | $CH_3$ | 85 |
| O | $CH_2Bz$ | 95 |
| O | $CH_2CO_2Et$ | 89 |
| O | $(CH_2)_3Br$ | 106-108 |

A partir des différents 1,3-dihydro-indol-2-one décrits ci-dessus et de chlorures de benzènesulfonyle appropriés on a préparé les composés selon l'invention en utilisant les modes opératoires rapportés dans les exemples qui suivent.

EXEMPLES 1 et 2

5-Ethoxy-1,3-dihydro-1-[2-méthoxy-4-(pyrrol-1-yl)benzènesulfonyl]-3-spirocyclo-hexaneindol-2-one et 5-éthoxy-1,3-dihydro-1-[2-méthoxy-4-(Δ3-pyrrolin-1-yl)benzène-sulfonyl]-3-spirocyclohexane-indol-2-one.

A) 5-Ethoxy-1,3-dihydro-1-(2-méthoxy-4-nitrobenzènesulfonyl)-3-spirocyclohexaneindol-2-one.

On refroidit dans un bain de glace à une température comprise entre 10°C et 15°C une solution de 15 g de 5-éthoxy-1,3-dihydro-3-spirocyclohexaneindol-2-one dans 150 ml de THF. On ajoute en 2 heures, 2,4 g d'hydrure de sodium à 60 % dans l'huile puis on introduit, par portions, en 30 minutes, 18 g de chlorure de 2-méthoxy-4-nitrobenzènesulfonyle. Après 18 heures sous agitation à TA, on verse la suspension obtenue dans une solution glacée de chlorure de sodium puis on extrait par AcOEt. On sèche la phase organique sur sulfate de sodium, évapore à sec, puis le résidu est cristallisé à chaud dans 200 ml d'éther iso. On obtient 23 g du produit attendu, F = 160°C.

On obtient également ce composé en suivant le mode opératoire décrit ci-après.

A') 5-Ethoxy-1,3-dihydro-1-(2-méthoxy-4-nitrobenzènesulfonyl)-3-spirocyclohexane-indol-2-one.

On refroidit vers -50°C une solution de 15 g de 5-éthoxy-1,3-dihydro-3-spirocyclohexaneindol-2-one dans 300 ml de THF et ajoute 7,2 g de *tert*-butylate de potassium. On laisse sous agitation en laissant remonter la température vers 0°C, puis refroidit à -50°C et ajoute une solution de 16,2 g de chlorure de 2-méthoxy-4-nitrobenzènesulfonyle dans le THF. On laisse 1 heure sous agitation à TA, ajoute 100 ml d'eau et évapore sous vide le solvant. On extrait la phase aqueuse au DCM, sèche sur sulfate de magnésium et évapore sous vide le solvant. On obtient le produit attendu après cristallisation à chaud dans l'éther iso.

Selon ce dernier mode opératoire, en partant des 1,3-dihydro-indol-2-one convenables, on prépare les dérivés nitrés suivants :

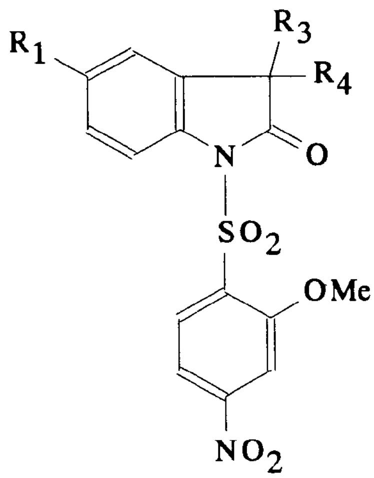

| $R_1$ | $CR_3R_4$ | F°C ou RMN |
|---|---|---|
| Cl- | 4,4-diméthylcylohexane | 169 |
| EtO- | 4,4-diméthylcyclohexane | 110 |
| F- | 4,4-diméthylcyclohexane | 137 |
| $CF_3$ | 4,4-diméthylcyclohexane | |
| Cl- | cycloheptane | 137 |
| EtO- | cycloheptane | 125 |
| Cl- | tétrahydropyrane-4 | 212 |

| $R_1$ | $CR_3R_4$ | F°C ou RMN |
|---|---|---|
| Cl- | tétrahydrothiopyrane-4 | 116 |
| EtO- | 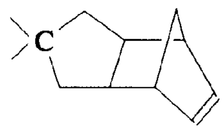 | 88 |
| EtO- | 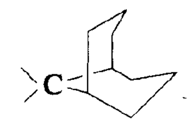 | 140 |
| EtO- | 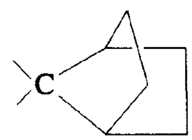 | RMN* |

* Spectre RMN à 200 MHz dans le DMSO :

| | | |
|---|---|---|
| 1,3 ppm | : t | : 3H |
| 1,3 – 1,5 ppm | : mt | : 6H |
| 2,2 ppm | : mt | : 4H |
| 3,7 ppm | : s | : 3H |
| 4,0 ppm | : q | : 2H |
| 7,0 ppm | : mt | : 2H |
| 7,6 ppm | : d | : 1H |
| 7,9 ppm | : mt | : 2H |
| 8,2 ppm | : d | : 1H |

B) 1-(4-Amino-2-méthoxybenzènesulfonyl)-5-éthoxy-1,3-dihydro-3-spirocyclohexane-indol-2-one.

On porte à reflux une suspension contenant 20 g du composé obtenu à l'étape précédente, 20 g de fer en poudre, 70 ml d'eau et 70 ml d'alcool 96 ; on ajoute en 30 minutes une solution de 7 ml d'acide chlorhydrique concentré dans 35 ml d'eau. Après 4 heures à reflux, on ajoute 15 ml de lessive de soude puis on extrait au DCM. La phase organique est filtrée sur Célite® puis séchée sur sulfate de sodium. Le résidu est cristallisé à chaud dans 100 ml d'un mélange éther iso/AcOEt (80/20, v/v). On obtient 15,8 g du produit attendu, F = 177°C.

On obtient également ce composé en suivant le mode opératoire décrit ci-après.

B') 1-(4-Amino-2-méthoxybenzènesulfonyl)-5-éthoxy-1,3-dihydro-3-spirocyclohexane-indol-2-one.

On refroidit vers 5°C une solution de 0,5 g du composé obtenu à l'étape A dans 10 ml d'EtOH et ajoute 0,8 ml d'HCl concentré et 0,4 g d'étain en poudre. On chauffe à 40°C pendant 45 minutes et évapore sous vide le solvant. On neutralise par ajout de NaOH, extrait à l'AcOEt, sèche sur sulfate de magnésium, filtre sur Célite® et évapore sous vide le filtrat. On chromatographie le résidu sur silice en éluant par le mélange DCM-/MeOH (99/1 ; v/v). On obtient le produit attendu.

On prépare également selon ce dernier mode opératoire les anilines suivantes :

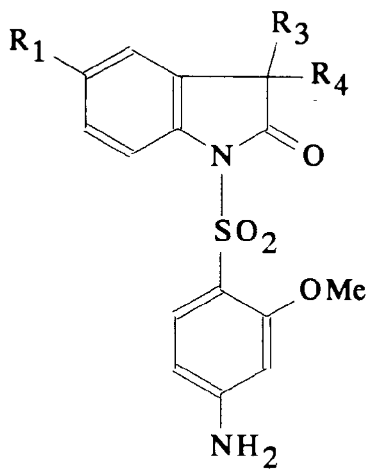

| $R_1$ | $CR_3R_4$ | F°C |
|---|---|---|
| Cl- | 4,4-diméthylcyclohexane | 222 |
| EtO- | 4,4-diméthylcyclohexane | 230 |
| F- | 4,4-diméthylcyclohexane | 130 |
| $CF_3$- | 4,4-diméthylcyclohexane | |
| Cl- | cycloheptane | 184 |
| EtO- | cycloheptane | 128 |
| Cl- | tétrahydropyrane-4 | 220 |
| Cl- | tétrahydrothiopyrane-4 | 229 |
| EtO- | 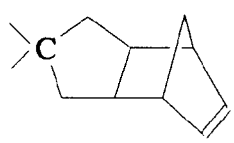 | 241 |

| $R_1$ | $CR_3R_4$ | F°C |
|---|---|---|
| EtO- | 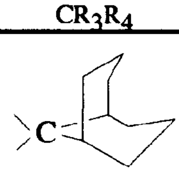 | 232 |
| EtO- | 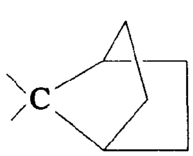 | 189 |

B") Le composé obtenu à l'étape B ci-dessus peut également être préparé à partir du dérivé nitré correspondant par hydrogénation pendant 2 heures, à 50°C et sous 1 bar de pression, en présence de palladium sur charbon à 10 %.

C) 5-Ethoxy-1,3-dihydro-1-[2-méthoxy-4-(pyrrol-1-yl)benzènesulfonyl]-3-spirocyclohexaneindol-2-one et 5-éthoxy-1,3-dihydro-1-[2-méthoxy-4-(Δ3-pyrrolin-1-yl)benzènesulfonyl]-3-spirocyclohexane-indol-2-one.

On chauffe à reflux pendant 4 heures un mélange contenant 500 mg du composé préparé à l'étape B, 10 ml de DMF, 0,3 g de carbonate de sodium et 300 mg de cis 1,4-dichlorobut-2-ène. Le mélange réactionnel est versé sur un mélange eau-glace puis on extrait par AcOEt et lave à l'eau ; on évapore le solvant, puis le résidu obtenu est chromatographié sur silice. On élue par un mélange DCM/heptane (95/5, v/v) puis par DCM pur. On recueille 70 mg du produit le moins polaire (composé de l'exemple 1), F = 174°C puis on recueille 60 mg du produit le plus polaire (composé de l'exemple 2), F = 170°C.

Les composés décrits dans les tableaux des étapes A et B ci-dessus peuvent être utilisés pour préparer des composés selon l'invention analogues à celui obtenu à l'étape C.

EXEMPLE 3

5-Ethoxy-1,3-dihydro-1-[2-méthoxy-4-(pyrrolidin-1-yl)benzènesulfonyl]-3-spirocyclohexaneindol-2-one.

450 mg du composé obtenu à l'exemple 2 sont placés dans 25 ml d'EtOH à 95 et 25 ml d'AcOEt, en présence de 150 mg de palladium sur charbon à 5 % et sont hydrogénés à 35°C sous pression de 40 bars pendant 4 heures. On filtre le catalyseur, et évapore à sec. Le produit attendu cristallise dans l'éther iso, m = 110 mg, F = 185°C.

EXEMPLE 4

5-Ethoxy-1,3-dihydro-1-[4-(isoindolin-2-yl)-2-méthoxybenzènesulfonyl]-3-spirocyclohexaneindol-2-one.

On porte à reflux pendant 2 heures un mélange contenant 500 mg du composé préparé à l'exemple 1, étape B, 10 ml de DMF, 0,5 ml de TEA et 310 mg de α,α'-dibromoorthoxylène. On verse le milieu réactionnel sur un mélange eau-glace, on extrait par AcOEt, sèche sur sulfate de sodium, et évapore à sec. Le résidu est chromatographié sur silice en éluant par DCM puis DCM/AcOEt (95/5, v/v). On obtient le produit attendu qui est cristallisé dans l'éther iso, m = 170 mg, F = 190°C.

EXEMPLE 5

5-Ethoxy-1,3-dihydro-1-[2-méthoxy-4-(((3-méthyl)thien-2-yl)carboxamido)benzènesulfonyl]-3-spirocyclo hexaneindol-2-one.

On laisse sous agitation pendant 3 heures à TA un mélange contenant 500 mg du composé préparé à l'exemple 1, étape B, 10 ml de DCM, 2 ml de pyridine et 250 mg de chlorure de l'acide 3-méthylthiophène-2-carboxylique. On lave le milieu à l'acide chlorhydrique 1N puis à l'eau. On sèche sur sulfate de sodium puis on évapore à sec. Le résidu est chromatographié sur silice en éluant par DCM. Le produit attendu recristallise dans l'éther iso, m = 0,21 g, F = 174°C.

EXEMPLE 6

1-[4-(N-Benzylcarbamoyl)-2-méthoxybenzènesulfonyl]-5-éthoxy-1,3-dihydro-3-spirocyclohexane-indol-2 -one.

A) Ester benzylique de l'acide 3-méthoxy-4-[(5-éthoxy-2,3-dihydro-2-oxo-3-spirocyclohexane-indol-1-yl)sulfonyl]benzoique.

On ajoute par petites fractions 1,4 g d'hydrure de sodium à une solution de 11,8 g de 5-éthoxy-1,3-dihydro-3-spirocyclohexaneindol-2-one dans 100 ml de THF. Après 30 minutes sous agitation à TA, on ajoute 17 g d'ester benzylique de l'acide 4-chlorosulfonyl-3-méthoxybenzoïque et on maintient l'agitation à TA pendant 1 heure. On verse sur 400 ml d'un mélange eau-alcool (50/50, v/v). Le précipité formé est filtré, séché puis recristallisé dans l'alcool, m = 22,65 g, F = 135°C.

B) Acide 3-méthoxy-4-[(5-éthoxy-2,3-dihydro-2-oxo-3-spirocyclohexaneindol-1-yl)sulfonyl]-benzoïque.

A une solution de 22,65 g d'ester benzylique préparé à l'étape précédente dans 600 ml d'AcOEt, on ajoute 2,5 g de palladium sur charbon à 10 % dans l'huile puis on hydrogène sous pression atmosphérique pendant 2 heures à 40°C. On filtre l'insoluble puis on concentre le milieu. Le produit attendu cristallise dans le pentane, m = 18,3 g, F = 181°C.

C) Chlorure de 3-méthoxy-4-[(5-éthoxy-2,3-dihydro-2-oxo-3-spirocyclohexaneindol-1-yl)sulfonyl]benzoyle.

On chauffe à reflux pendant 3 heures une solution de 2,3 g de l'acide précédent dans 20 ml de chlorure de thionyle. Le milieu réactionnel est concentré à sec, le produit est utilisé brut, en solution dans du DCM

à l'étape suivante.

D) 1-[4-(N-Benzylcarbamoyl-2-méthoxybenzènesulfonyl]-5-éthoxy-1,3-dihydro-3-spirocyclohexaneindol-2-one.

On laisse sous agitation à TA pendant 30 minutes, un mélange contenant 500 mg du composé préparé à l'étape précédente et 200 mg de benzylamine dans 20 ml de DCM et 0,5 ml de TEA. On lave par une solution de HCl 1N, sèche sur sulfate de sodium et concentre. Le produit attendu cristallise dans l'éther iso, m = 440 mg, F = 196°C.

Le composé de l'exemple 6 ne fait pas partie des composés de formule (I) selon l'invention, mais le composé intermédiaire obtenu à l'étape C est utilisé pour la préparation du composé de l'exemple 8.

Le mode opératoire décrit à l'exemple 6, étape D, est également utilisé pour la préparation des composés des exemples 44-54, en utilisant les amines appropriées.

EXEMPLE 7

1-[4-(2-Carbamoylpyrrolidin-1-ylcarbonyl)-2-méthoxybenzènesulfonyl]-5-chloro-1,3-dihydro-3-spirocyclohexaneindol-2-one.

A) Ester phénylique de l'acide 4-[(5-chloro-2,3-dihydro-2-oxo-3-spirocyclohexane-indol-1-yl)sulfonyl]-3-méthoxybenzoique.

On agite pendant 30 minutes, à TA, sous azote, un mélange contenant 1 g de 5-chloro-1,3-dihydro-3-spirocyclohexaneindol-2-one, 50 ml de THF et 115 mg d'hydrure de sodium. On introduit 1,5 g de chlorure de 2-méthoxy-4-phénoxycarbonylbenzènesulfonyle et on maintient l'agitation pendant 20 heures à TA. On concentre sous vide le milieu réactionnel, reprend le résidu par 30 ml d'eau et extrait par AcOEt. On lave la phase organique, sèche et concentre sous vide. Le produit obtenu est purifié par chromatographie sur silice en éluant par un mélange éther iso/hexane (40/60, v/v). On obtient 1,4 g du composé attendu, F = 165°C.

B) 1-[4-(2-Carbamoylpyrrolidin-1-ylcarbonyl)-2-méthoxybenzènesulfonyl]-5-chloro-1,3-dihydro-3-spirocyclohexaneindol-2-one.

On porte à reflux pendant 1 heure un mélange contenant 300 mg du composé préparé à l'étape précédente, 500 mg de chlorhydrate de (L) prolinamide, 1 ml de TEA et 20 ml de prehnitène. Après refroidissement, le milieu réactionnel est repris par AcOEt, lavé par une solution de HCl 1N, par de l'eau, puis séché sur sulfate de sodium et concentré. Le résidu est chromatographié sur silice en éluant par un mélange DCM/MeOH (98/2, v/v). On obtient 0,09 g du produit attendu, F = 142°C.

EXEMPLE 8

5-Ethoxy-1,3-dihydro-1-[2-méthoxy-4-(2-méthoxycarbonylpyrrolidin-1-ylcarbonyl)-benzènesulfonyl]-3-spirocyclohexaneindol-2-one.

A 500 mg du chlorure de 3-méthoxy-4-[(5-éthoxy-2,3-dihydro-2-oxo-3-spirocyclohexaneindol-1-yl)sulfonyl]benzoyle préparé à l'exemple 6, étape C, on ajoute 500 mg de chlorhydrate de l'ester méthylique de la (L) proline, 0,5 ml de TEA et 20 ml de DCM. Après une heure sous agitation à TA, on lave par HCl 1N, sèche et concentre. Le résidu est chromatographié sur silice en éluant par un mélange DCM/MeOH (98/2, v/v). On obtient 300 mg du produit attendu caractérisé par son spectre de RMN.

RMN à 200 MHz dans le DMSO (2,5 ppm).

1,25 ppm : t : 3 H ;
1,3-2,35 ppm : m : 14 H ;
3,1-3,7 ppm : m : 8 H ;
3,8 ppm : q : 2 H ;
4,35 ppm : mt : 1 H ;
6,7-8 ppm : m : 6 H .

EXEMPLE 9

1-[4-(N'-cyclopentylureido)-2-méthoxybenzènesulfonyl]-5-éthoxy-1,3-dihydro-3-spirocyclohexaneindol-2-one.

A) 5-Ethoxy-1,3-dihydro-1-(2-méthoxy-4-phénoxycarboxamido-benzènesulfonyl)-3-spirocyclohexaneindol-2-one.

On refroidit à une température inférieure à 5°C un mélange contenant 2 g de 1-(4-amino-2-méthoxybenzènesulfonyl)-5-éthoxy-1,3-dihydro-3-spirocyclohexaneindol-2-one (préparé à l'exemple 1, étape B),

3,6 ml de chloroformiate de phényle et 120 ml d'éther. On ajoute 960 mg de soude dans 16 ml d'eau et laisse remonter la température pendant 24 heures sous agitation. On filtre le précipité, lave à l'eau puis à l'éther. Le résidu est chromatographié sur silice en éluant par DCM. On obtient le produit attendu, F = 181°C.

B) 1-[4-(N'-cyclopentylureido)-2-méthoxybenzènesulfonyl]-5-éthoxy-1,3-dihydro-3-spirocyclo-hexaneindol-2-one.

On dissout 2 g du composé préparé à l'étape précédente dans 40 ml d'EtOH 100 et 30 ml de DCM. On ajoute 2 ml de cyclopentylamine et on laisse 18 heures sous agitation à TA. L'alcool est évaporé sous vide puis le résidu est chromatographié sur silice en éluant par un mélange DCM/AcOEt (95/5, v/v). On obtient 1,8 g du produit attendu qui cristallise dans l'éther iso, F = 195°C.

EXEMPLE 10

5-Chioro-1,3-dihydro-1-[4-(N',N'-diéthyluréido)-2-méthoxybenzènesulfonyl]-3-spiro(4-tétrahydrothiopyrane-1-oxyde)indol-2-one.

On en prépare de la même manière qu'à l'exemple 9 (étape A et B).

A) 5-Chloro-1,3-dihydro-1-[4-(N',N'-diéthyluréido)-2-méthoxybenzènesulfonyl]-3-spiro(tétrahydrothiopyrane-4)indol-2-one, F = 214° C à partir du 5-chloro-1,3-dihydro-1-[4-amino-2-méthoxybenzènesulfonyl]-3-spiro(tétrahydrothiopyrane-4)indol-2-one, F = 229° C.

B) 5-Chloro-1,3-dihydro-1-[4-(N',N'-diéthylureido)-2-méthoxybenzènesulfonyl]-3-spiro(4-tétrahydrothiopyrane-1-oxyde)indol-2-one.

On agite durant 24 heures à TA un mélange de 0,45 g du composé préparé à l'étape A, 0,2 g de périodate de sodium dans 3 ml de méthanol et 2 ml d'eau. On filtre le précipité et évapore le méthanol du filtrat sous pression réduite. On extrait au DCM, sèche sur sulfate de magnésium et évapore le solvant. Le résidu est chromatographié sur une colonne de silice en éluant avec un mélange DCM/méthanol 98/2 (v/v) et isole 0,4 g du produit attendu, F = 217° C.

EXEMPLE 11

5-Chloro-1,3-dihydro-1-[4-(N',N'-diéthylureido)-2-méthoxybenzènesulfonyl]-3-spiro(4-tétrahydrothiopyrane-1,1-dioxyde)indole-2-one.

On agite à TA durant 4 heures un mélange de 0,45 g du composé de l'exemple 10 et de 0,5 g d'acide métachloroperbenzoïque dans 10 ml de DCM. On ajoute alors 12 ml d'une solution aqueuse saturée de bicarbonate de sodium, décante et lave à l'eau la phase organique qui est séchée et évaporée sous pression réduite. Le résidu est recristallisé dans un mélange de cyclohexane et d'acétate d'éthyle 7/3 (v/v) pour donner 0,35 g du produit attendu, F = 211° C.

EXEMPLE 12

1-[4-(N'-cyclopentylthioureido)-2-méthoxybenzènesulfonyl]-5-éthoxy-1,3-dihydro-3-spirocyclohexaneindol-2-one.

Le composé obtenu à l'exemple 9 est placé dans 50 ml de toluène en présence de 1,5 g de réactif de Lawesson. On chauffe à reflux pendant 24 heures. On chasse le toluène puis le résidu est chromatographié sur silice en éluant par DCM pur puis par un mélange DCM/AcOEt allant jusqu'à (90/10, v/v). On obtient le produit attendu qui cristallise dans l'éther iso, F = 197°C.

EXEMPLE 13

1-[4-(3-(N-Boc amino)azétidin-1-ylcarboxamido)-5-méthoxybenzènesulfonyl]-2-éthoxy-1,3-dihydro-3-spirocyclohexaneindol-2-one.

A) 1-Benzhydryl-3-(N-Boc amino)azétidine.

On agite à TA pendant 2 heures, un mélange contenant 5 g de 3-amino-1-benzhydrylazétidine et 5 g de $(Boc)_2O$ dans 130 ml de dioxane et 4 ml de TEA. On évapore le dioxane, reprend par AcOEt et lave à l'eau. On sèche sur sulfate de sodium puis on évapore à sec. Le produit attendu cristallise dans l'éther iso, m = 6 g.

B) Chlorhydrate de 3-(N-Boc amino)azétidine.

On prépare un mélange contenant 6 g du composé préparé à l'étape précédente, 300 ml d'EtOH 95, 700 mg d'hydroxyde de palladium et 3 ml d'HCl concentré. On hydrogène pendant 2 heures à 35-40°C

sous 2 bars de pression. On filtre le catalyseur et évapore à sec. Le produit attendu cristallise dans l'éther iso, m = 3,4 g.

C) 1-[4-(3-(N-Boc amino)azétidin-1-ylcarboxamido)-5-méthoxybenzènesulfonyl]-2-éthoxy-1,3-dihydro-3-spirocyclohexaneindol-2-one.

On laisse sous agitation pendant 24 heures à TA un mélange contenant : 500 mg du composé préparé à l'exemple 9, étape A, 20 ml d'alcool 100, 15 ml de DCM, 250 mg du composé préparé à l'étape B et 0,5 ml de TEA. On évapore les solvants puis on effectue une chromatographie sur colonne de silice en éluant par le mélange DCM/AcOEt (95/5, v/v). Le produit attendu cristallise dans l'éther iso, m = 200 mg, F = 156°C.

EXEMPLE 14

1-[4-(N'-Carboxyméthyl-N'-méthylureido)-2-méthoxybenzènesulfonyl]-5-éthoxy-1,3-dihydro-3-spirocyclohexaneindol-2-one.

On prépare un mélange contenant 500 mg du composé préparé à l'exemple 9, étape A, 20 ml d'EtOH 100, 15 ml de DCM, 1,5 g de sarcosine et 2 ml de triéthylamine. Après 24 heures sous agitation à TA, on évapore les solvants puis on reprend par AcOEt à chaud et on filtre l'insoluble. Le filtrat est évaporé à sec puis le résidu est chromatographié sur silice en éluant par DCM pur puis par le mélange DCM/MeOH (85/15, v/v). On obtient le produit attendu qui est caractérisé par son spectre de RMN à 200 MHz dans le DMSO (2,5 ppm).

1,3 ppm : t : 3 H ;
1,4-2,1ppm : m : 10 H ;
2,95 ppm : s : 3 H ;
3,5 ppm : s : 3 H ;
3,9 ppm : s : 2 H ;
4 ppm : q : 2 H ;
6,7-7,9 ppm : m : 6 H ;
9,45 ppm : s.e. : 1 H ;

EXEMPLE 15

5-Ethoxy-1,3-dihydro-1-[2-méthoxy-4-(1-méthyl-2,4-dioxoimidazolin-3-yl)benzène sulfonyl]-3-spirocyclohexaneindol-2-one.

Par chauffage à 100°C pendant 24 heures, sous vide, le produit obtenu à l'exemple précédent, est cyclisé. On obtient 230 mg du produit attendu, F = 200°C.

EXEMPLE 16

1-[4-(N',N'-Diéthylthioureido)-2-méthoxybenzènesulfonyl]-5-éthoxy-1,3-dihydro-3-spirocyclohexaneindol-2-one.

A) 5-Ethoxy-1,3-dihydro-1-(2-méthoxy-4-phénoxythiocarboxamidobenzènesulfonyl)-3-spirocyclohexaneindol-2-one.

On prépare un mélange contenant 500 mg du composé obtenu à l'exemple 9, étape A, 900 mg de chlorure de phénoxythiocarbonyle, 30 ml d'éther, 8 ml d'eau, 120 mg de soude et 20 ml de DCM. Après 24 heures sous agitation à TA, on évapore les solvants puis le résidu est chromatographié sur silice en éluant par DCM. Le produit attendu cristallise dans l'éther iso, m = 140 mg, F = 157°C.

B) 1-[4-(N',N'-Diéthylthioureido)-2-méthoxybenzènesulfonyl]-5-éthoxy-1,3-dihydro-3-spirocyclohexaneindol-2-one.

On laisse sous agitation à TA pendant 18 heures un mélange contenant : 140 mg du composé préparé à l'étape A, 20 ml d'EtOH 100, 5 ml de DCM et 1 ml de diéthylamine. On évapore les solvants puis on chromatographie le résidu sur colonne de silice en éluant par DCM pur puis un mélange DCM/AcOEt jusqu'à (90/10, v/v). Le produit attendu cristallise dans l'éther iso, m = 105 mg, F = 167°C.

EXEMPLE 17

5-Ethoxy-1,3-dihydro-1-(2-méthoxy-4-guanidinobenzènesulfonyl)-3-spirocyclo-hexaneindol-2-one.

On porte à reflux pendant 1 heure un mélange contenant 500 mg du composé préparé à l'exemple 1, étape B, 125 mg de cyanamide, 7 ml d'AcOEt, 1 ml d'EtOH et 0,2 ml d'une solution à 20 % acide chlorhydrique dans l'EtOH. On évapore les solvants puis on chromatographie le résidu sur colonne de silice en éluant par DCM

puis MeOH. Le produit isolé concrétise dans l'éther. On ajoute une solution 2N de $NaHCO_3$ puis on extrait la base formée par AcOEt ; on évapore à sec et le produit attendu concrétise dans l'éther iso, m = 0,055 g, F = 235°C.

EXEMPLE 18

5-Ethoxy-1,3-dihydro-1-[2-méthoxy-4-(N-méthylcarbamoylméthoxy)-benzènesulfonyl]-3-spirocyclohexaneindol-2-one.

A) 2-Méthoxy-4-(éthoxycarbonylméthoxy)phénylsulfonate de potassium.

On mélange à 0°C 10 g d'ester éthylique de l'acide (3-méthoxyphénoxy)acétique et 30 ml de DCM et l'on ajoute en 20 minutes 7,5 ml de chlorure de triméthylsilylsulfonyle dans 30 ml de DCM. On laisse remonter à TA sous agitation et après 2 heures, on ajoute 30 g de glace et l'on agite à nouveau. Après décantation on lave la phase aqueuse par de l'éther et on ajoute une quantité suffisante de carbonate de potassium pour atteindre pH 7. Le précipité blanc qui se forme est filtré puis on le lave par de l'acétone et de l'éther. On obtient ainsi 3,1 g du produit attendu qui est caractérisé par son spectre de RMN.

B) Chlorure de 2-méthoxy-4-(éthoxycarbonylméthoxy)phénylsulfonyle.

On porte à reflux pendant 3 heures 3,1 g du composé obtenu à l'étape précédente dans 30 ml d'oxychlorure de phosphore. Le milieu est concentré sous vide puis repris par de la glace. On extrait à l'acétate d'éthyle, lave à l'eau, par la soude N puis à nouveau par de l'eau, sèche sur sulfate de sodium et concentre sous vide. Le produit attendu cristallise dans l'éther iso, m = 2,5 g, F = 89°C.

C) Ester éthylique de l'acide 3-méthoxy-4-[(5-éthoxy-2,3-dihydro-2-oxo-3-spirocyclohexaneindol-1-yl)sulfonyl]phénoxyacétique.

On prépare un mélange contenant 0,5 g de 5-éthoxy-1,3-dihydro-3-spirocyclohexaneindol-2-one, 5 ml de THF et 60 mg d'hydrure de sodium. Après 15 minutes sous agitation à 15°C, on ajoute 0,66 g du composé préparé à l'étape précédente. On maintient l'agitation pendant 15 minutes puis on concentre le milieu sous vide. On extrait à l'éther, lave à l'eau puis on cristallise le produit attendu dans l'éther iso, m = 0,85 g, F = 160°C.

D) 5-Ethoxy-1,3-dihydro-1-[2-méthoxy-4-(N-méthylcarbamoylméthoxy)-benzène-sulfonyl]-3-spirocyclohexaneindol-2-one.

On laisse pendant 4 jours à TA sous agitation un mélange contenant 0,5 g du composé préparé à l'étape précédente, 15 ml d'une solution à 33 % de méthylamine dans le MeOH et 15 ml d'EtOH. On concentre sous vide puis on chromatographie le résidu sur silice. On lave la colonne par DCM, puis on élue par AcOEt pour obtenir le produit attendu, m = 0,1 g, F = 192-195°C.

EXEMPLE 19

5-Ethoxy-1,3-dihydro-1-[2-méthoxy-4-(N-méthyl-N-(2-méthylallyl)-amino)benzène-sulfonyl]-3-spirocyclo hexaneindol-2-one.

A) 5-Ethoxy-1,3-dihydro-1-[2-méthoxy-4-(N-méthylaminobenzènesulfonyl]-3-spirocyclohexaneindol-2-one.

2 g du composé préparé à l'exemple 1, étape B, refroidis dans un bain de glace à 10°C sont dissous dans 6 ml de formaldehyde à 37 % dans l'eau et 40 ml d'acétonitrile. On ajoute 1,7 g de cyanoborohydrure de sodium à 85 % de pureté puis 0,5 ml d'acide acétique et on laisse sous agitation à TA pendant 24 heures. On évapore l'acétonitrile, reprend par de l'eau et AcOEt. On décante, sèche la phase organique puis on effectue 2 chromatographies successives sur silice en éluant par le mélange DCM/heptane (85/15, v/v) puis par DCM pur et enfin par le mélange DCM/AcOEt (98/2, v/v). On obtient le composé attendu, m = 0,36 g, F = 157°C.

B) 5-Ethoxy-1,3-dihydro-1-[2-méthoxy-4-(N-méthyl-N-(2-méthylallyl)amino)benzènesulfonyl]-3-spirocyclohexaneindol-2-one.

On porte à reflux pendant 10 heures un mélange contenant 500 mg du composé obtenu à l'étape précédente, 10 ml de DMF, 0,5 ml de diéthylamine et 0,5 ml de 3-chloro-2-méthylpropène. On verse le milieu réactionnel sur un mélange eau-glace puis on extrait par AcOEt, lave à l'eau et chromatographie sur silice en éluant par DCM. Le composé attendu cristallise dans le pentane, m = 190 mg, F = 118°C.

En procédant selon les modes opératoires décrits dans les exemples précédents, on a préparé les composés (I) selon l'invention décrits dans le tableau 3 ci-dessous.

TABLEAU 3

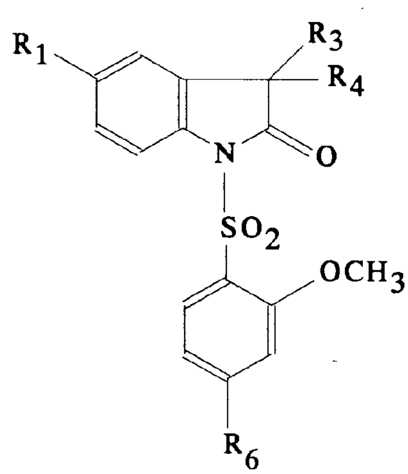

| Exemple | $R_1$ | $CR_3R_4$ | $R_6$ | F°C |
|---|---|---|---|---|
| 20 (a) | Cl- | cyclohexane | 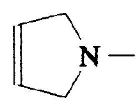 | 179 |
| 21 (a) | EtO- | 4,4-diméthyl cyclohexane | 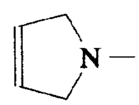 | 167 |
| 22 (b) | Cl- | cyclohexane | $EtOCOCH_2NHCONH-$ | 167 |

TABLEAU 3 (suite)

| Exemple | $R_1$ | $CR_3R_4$ | $R_6$ | F°C |
|---|---|---|---|---|
| 23<br>(b) | Cl– | cyclohexane | 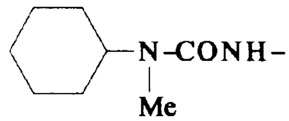 | 139 |
| 24<br>(c) | EtO– | cyclohexane | 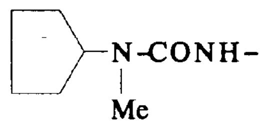 | 150 |
| 25<br>(c) | EtO– | cyclohexane | 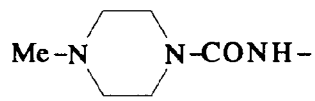 | 197 |
| 26<br>(c) | EtO– | cyclohexane | MeONCONH–<br>Me | 177 |
| 27<br>(c) | EtO– | cyclohexane | $MeNH(CH_2)_2NCONH-$<br>Me | 170<br>chlorhydrate<br>monohydrate |
| 28<br>(c) | EtO– | cyclohexane | HONCONH–<br>Me | 185 |
| 29<br>(c) | EtO– | cyclohexane | $HOCH_2CH_2NCONH-$<br>Me | 128 |
| 30<br>(c) | EtO– | cyclohexane | 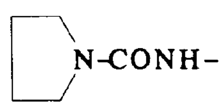 | 139 |
| 31<br>(c) | EtO– | cyclohexane | 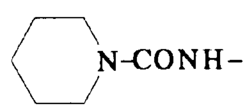 | 210 |

TABLEAU 3 (suite)

| Exemple | $R_1$ | $CR_3R_4$ | $R_6$ | F°C |
|---|---|---|---|---|
| 32 (c) | EtO– | cyclohexane | 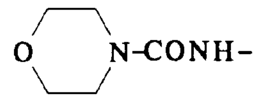 | 190 |
| 33 (c) | EtO– | cyclohexane | 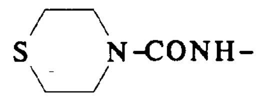 | 212 |
| 34 (c) | EtO– | cyclohexane | 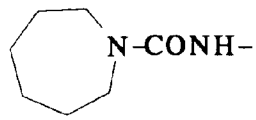 | 179 |
| 35 (c) | EtO– | cyclohexane | 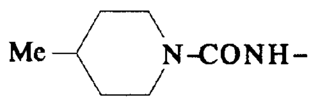 | 146 |
| 36 (c) | EtO– | cyclohexane | 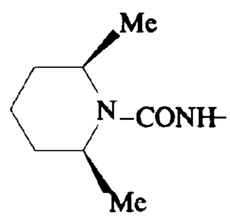 | 198 |
| 37 (c) | EtO– | cyclohexane | 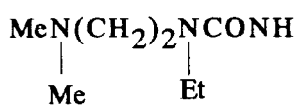 | 208 chlorhydrate monohydrate |
| 38 (c) | EtO– | cyclohexane | 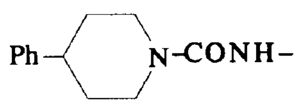 | 195 |
| 39 (c) | EtO– | cyclohexane | 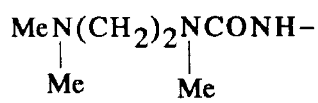 | 200 fumarate |

TABLEAU 3 (suite)

| Exemple | $R_1$ | $CR_3R_4$ | $R_6$ | F°C |
|---|---|---|---|---|
| 40 (c) | EtO– | cyclohexane | 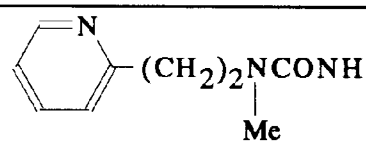 | 159 |
| 41 (d) | Cl– | 4,4–diméthyl–cyclohexane | 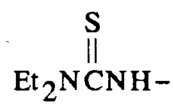 | 179 |
| 42 (d) | EtO– | 4,4–diméthyl–cyclohexane | 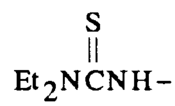 | 146 |
| 44 (e) | EtO– | cyclohexane | 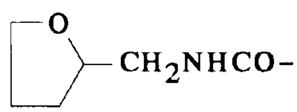 | 189 |
| 45 (e) | EtO– | cyclohexane | $CF_3CH_2NHCO$– | 210 |
| 46 (e) | EtO– | cyclohexane | 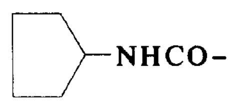 | 196 |
| 47 (e) | EtO– | cyclohexane | 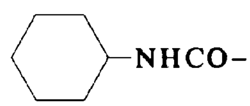 | 155 |
| 48 (e) | EtO– | cyclohexane | 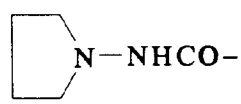 | 204 |

TABLEAU 3 (suite)

| Exemple | $R_1$ | $CR_3R_4$ | $R_6$ | F°C |
|---|---|---|---|---|
| 49 (e) | EtO– | cyclohexane | 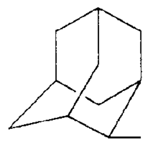 $CH_2NHCO-$ | 143 |
| 50 (e) | EtO– | cyclohexane | $EtO(CH_2)_2NHCO-$ | 166 |
| 51 (e) | EtO– | cyclohexane | $H_2NCOCH_2NHCO-$ | 245 |
| 52 (e) | EtO– | cyclohexane | $Et_2N-COCH_2NHCO-$ | 161 |
| 53 (e) | EtO– | cyclohexane | $(Et_2N-COCH_2)_2NCO-$ | 141 |
| 54 (e) | EtO– | cyclohexane | $NC-C(Me)_2NHCO-$ | 198 |
| 55 (f) | EtO– | cyclohexane | $Et_2NC(NH)NH-$ | 137 chlorhydrate hémihydrate |
| 56 (g) | Cl– | 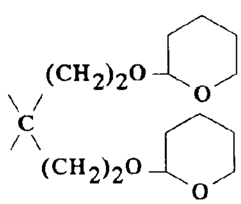 | $CH_3O-$ | 136 |

TABLEAU 3 (suite)

| Exemple | $R_1$ | $CR_3R_4$ | $R_6$ | F°C |
|---|---|---|---|---|
| 57 (1) (b) | EtO- | 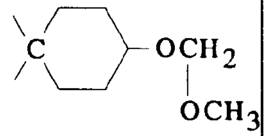 | $Et_2NCONH-$ | 88 |
| 58 (1) (h) | EtO- | 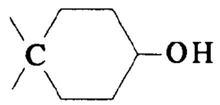 | $Et_2NCONH-$ | 138 |

(a) on prépare ce composé selon le mode opératoire décrit à l'EXEMPLE 2 étape C, en effectuant la réaction sous atmosphère inerte et en utilisant la triéthylamine comme base.
(b) on prépare ce composé selon les modes opératoires décrits à l'EXEMPLE 9 étape A puis étape B en utilisant les dérivés aminés ou les hétérocycles azotés appropriés.
(c) on prépare ce composé selon le mode opératoire décrit à l'EXEMPLE 9 étape B en utilisant les dérivés aminés ou les hétérocycles azotés appropriés.
(d) on prépare ce composé selon les modes opératoires décrits à l'EXEMPLE 16 étape A puis étape B.
(e) on prépare ce composé selon le mode opératoire décrit à l'EXEMPLE 6 étape D en utilisant les amines appropriés.
(f) on prépare ce composé selon le mode opératoire décrit à l'EXEMPLE 17 en utilisant le N,N-diéthylcyanamide.
(g) on prépare ce composé selon le mode opératoire décrit à l'EXEMPLE 1 étape A en utilisant le chlorure de 2,4-diméthoxybenzènesulfonyle et l'indol-2-one approprié.
(h) on prépare ce composé selon le mode opératoire décrit à l'EXEMPLE 68 à partir du composé obtenu à l'EXEMPLE 57 (mélange d'isomères).
I) mélange d'isomères.

EXEMPLE 59

5-Ethoxy-1-[4-(N',N'-diéthyluréido)-2-méthoxybenzènesulfonyl]-1,3-dihydro-3-spiro(4-oxocyclohexane) indol-2-one, 0,25 hydrate.

On laisse 16 heures sous agitation à TA, un mélange de 0,3 g du composé obtenu à l'EXEMPLE 68, 0,3 g de chlorochromate de pyridinium et 1,5 ml de DCM. On ajoute 10 ml d'eau au mélange réactionnel, évapore sous vide le DCM, extrait à l'AcOEt, sèche sur sulfate de magnésium et concentre sous vide le solvant. On chromatographie le résidu sur silice en éluant par le mélange DCM/MeOH (98/2; v/v). On obtient 0,26 g du produit attendu, F = 100°C.

EXEMPLE 60

5-Fluoro-1,3-dihydro-1-[2-méthoxy-4-(Δ3-pyrrolin-1-yl)benzènesulfonyl]-3-spiro(4,4-diméthylcyclohexane) indol-2-one.

On prépare ce composé selon le mode opératoire décrit à l'EXEMPLE 2 étape C, en effectuant la réaction sous atmosphère inerte et en utilisant la triéthylamine comme base.

EXEMPLE 61

5-Chloro-3,3-dihydroxyéthyl-1,3-dihydro-1-(2,4-diméthoxybenzène-sulfonyl)indol-2-one.

Ce composé est préparé selon la méthode décrite dans J. Org. Chem., 1977, 42, 3772. A 0,92 g du composé de l'exemple 56 décrit dans le tableau 3 ci-dessus, on ajoute 0,037 g de pyridiniumtoluènesulfate dans 12

ml d'éthanol et chauffe vers 55°C durant 3 heures. On évapore le solvant sous pression réduite et chromatographie le résidu sur une colonne de silice en éluant au DCM. On isole le produit attendu qui est cristallisé à chaud dans un mélange cyclohexane/acétate d'éthyle 50/50 (v/v). F = 166°C.

EXEMPLES 62 et 63

5-Ethoxy-1,3-dihydro-1-(2-méthoxy-4-nitrobenzènesulfonyl)-3-spiro[4-(méthoxyméthyloxy)cyclohexane] indol-2-one, isomère le moins polaire et isomère le plus polaire.

On prépare ces composés selon le mode opératoire décrit à l'EXEMPLE 1 étape A' à partir du 5-éthoxy-1,3-dihydro-3-spiro[4-(méthoxyméthyloxy)cyclohexane]indol-2-one. On chromatographie sur silice en éluant par le mélange cyclohexane/AcOEt (95/5 ; v/v). On sépare les deux isomères :

- le moins polaire : composé de l'EXEMPLE 62, F = 127°C ;
- le plus polaire : composé de l'EXEMPLE 63, F = 118°C.

EXEMPLES 64 et 65

1-(4-Amino-2-méthoxybenzènesulfonyl)-5-éthoxy-1,3-dihydro-3-spiro[4-(méthoxy-méthyloxy)cyclohexane] indol-2-one, isomère le moins polaire et isomère le plus polaire.

On prépare ces composés selon le mode opératoire décrit à l'EXEMPLE 1 étape B" à partir du mélange des composés obtenus aux EXEMPLES 62 et 63 avant chromatographie. On chromatographie sur silice en éluant par le mélange cyclohexane/AcOEt (95/5 ; v/v). On sépare les deux isomères :

- le moins polaire : composé de l'EXEMPLE 64, F = 103°C ;
- le plus polaire : composé de l'EXEMPLE 65, F = 111°C.

EXEMPLE 66

5-Ethoxy-1-[4-(N',N'-diéthyluréido)-2-méthoxybenzènesulfonyl]-1,3-dihydro-3-spiro[4-(méthoxyméthyloxy) cyclohexane]indol-2-one, isomère le moins polaire.

On prépare ce composé selon le mode opératoire décrit à l'EXEMPLE 9 étape A puis étape B, à partir du composé obtenu à l'EXEMPLE 64, et en utilisant lors de la 2ème étape la diéthylamine. On obtient le produit attendu après recristallisation dans le mélange cyclohexane/AcOEt, F = 160°C.

EXEMPLE 67

5-Ethoxy-1-[4-(N',N'-diéthyluréido)-2-méthoxybenzènesulfonyl]-1,3-dihydro-3-spiro[4-(méthoxyméthyloxy) cyclohexane]indol-2-one, isomère le plus polaire.

On prépare ce composé selon le mode opératoire décrit à l'EXEMPLE 9 étape A puis étape B, à partir du composé obtenu à l'EXEMPLE 65, et en utilisant lors de la 2ème étape, la diéthylamine. On obtient le produit attendu après recristallisation dans le mélange cyclohexane/AcOEt, F = 137°C.

EXEMPLE 68

5-Ethoxy-1-[4-(N',N'-diéthyluréido)-2-méthoxybenzènesulfonyl]-1,3-dihydro-3-spiro(4-hydroxycyclohexane) indol-2-one, monohydrate, isomère le moins polaire.

On chauffe à 50°C pendant 30 minutes une solution de 2,2 g du composé obtenu à l'EXEMPLE 66 dans 6 ml de MeOH et 1,2 ml d'HCl concentré. On ajoute 10 ml d'eau au mélange réactionnel, extrait à l'AcOEt, sèche sur sulfate de magnésium et évapore sous vide le solvant. On chromatographie le résidu sur silice en éluant par le mélange DCM/MeOH (90/10 ; v/v). On obtient 1,9 g du produit attendu après recristallisation dans un mélange cyclohexane/AcOEt, F = 138°C.

EXEMPLE 69

5-Ethoxy-1-[4-(N',N'-diéthyluréido)-2-méthoxybenzènesulfonyl]-1,3-dihydro-3-spiro(4-hydroxycyclohexane) indol-2-one, monohydrate, isomère le plus polaire.

On prépare ce composé selon le mode opératoire décrit à l'EXEMPLE 68 à partir du composé obtenu à l'EXEMPLE 67. On obtient le produit attendu après recristallisation dans un mélange cyclohexane/AcOEt, F = 144°C.

EXEMPLE 70

5-Ethoxy-1,3-dihydro-1-(2-méthoxy-4-nitrobenzènesulfonyl)-3-spiro(4-méthoxy-cyclohexane)indol-2-one, isomère le plus polaire.

On prépare ce composé selon le mode opératoire décrit à l'EXEMPLE 1 étape A' à partir du 5-éthoxy-1,3-dihydro-3-spiro(4-méthoxycyclohexane)indol-2-one, isomère le plus polaire (composé de la Préparation 17). On obtient le produit attendu, F = 141°C.

EXEMPLE 71

1-(4-Amino-2-méthoxybenzènesulfonyl)-5-éthoxy-1,3-dihydro-3-spiro(4-méthoxy-cyclohexane)indol-2-one, isomère le plus polaire.

On prépare ce composé selon le mode opératoire décrit à l'EXEMPLE 1 étape B" à partir du composé obtenu à l'EXEMPLE 70, F = 199°C.

EXEMPLE 72

5-Ethoxy-1-[4-(N',N'-diéthyluréido)-2-méthoxybenzènesulfonyl]-1,3-dihydro-3-spiro(4-méthoxycyclohexane)indol-2-one, isomère le moins polaire.

A) 5-Ethoxy-1,3-dihydro-1-(2-méthoxy-4-nitrobenzènesulfonyl)-3-spiro(4-méthoxy-cyclohexane)indol-2-one, isomère le moins polaire.

On prépare ce composé selon le mode opératoire décrit à l'EXEMPLE 1 étape A' à partir du composé obtenu à la Préparation 16 (isomère le moins polaire).

B) 1-(4-Amino-2-méthoxybenzènesulfonyl)-5-éthoxy-1,3-dihydro-3-spiro(4-méthoxy-cyclohexane)indol-2-one, isomère le moins polaire.

On prépare ce composé selon le mode opératoire décrit à l'EXEMPLE 1 étape B" à partir du composé obtenu à l'étape précédente.

C) 5-Ethoxy-1-[4-(N',N'-diéthyluréido)-2-méthoxybenzènesulfonyl]-1,3-dihydro-3-spiro(4-méthoxycyclohexane)indol-2-one, isomère le moins polaire.

On prépare ce composé selon le mode opératoire décrit à l'EXEMPLE 9 étape A puis étape B à partir du composé obtenu à l'étape précédente et en utilisant la diéthylamine, F = 118°C.

EXEMPLE 73

5-Ethoxy-1-[4-(N',N'-diéthyluréido)-2-méthoxybenzènesulfonyl]-1,3-dihydro-3-spiro(4-méthoxycyclohexane)indol-2-one, isomère le plus polaire.

On prépare ce composé selon le mode opératoire décrit à l'EXEMPLE 9 étape A puis étape B, à partir du composé obtenu à l'EXEMPLE 71, et en utilisant lors de la 2ème étape la diéthylamine. On obtient le produit attendu après recristallisation dans le mélange cyclohexane/AcOEt, F = 164°C.

EXEMPLE 74

5-Ethoxy-1,3-dihydro-1-(2-méthoxy-4-nitrobenzènesulfonyl)-3-spiro(4-hydroxy-cyclohexane)indol-2-one, isomère le plus polaire.

On chauffe à 50°C pendant 1 heure un mélange de 2,2 g du composé obtenu à l'EXEMPLE 63, 1,2 ml d'HCl concentré dans 6 ml de MeOH. On ajoute 10 ml d'eau au mélange réactionnel, essore le précipité formé, le lave à l'eau et sèche sous vide à 50°C. On obtient 1,3 g du produit attendu, F = 135°C.

EXEMPLE 75

5-Ethoxy-1-[4-(N',N'-diéthyluréido)-2-méthoxybenzènesulfonyl]-1,3-dihydro-3-spiro(4-éthoxycyclohexane)indol-2-one.

A) 5-Ethoxy-1,3-dihydro-1-(2-méthoxy-4-nitrobenzènesulfonyl)-3-spiro(4-éthoxy-cyclohexane)indol-2-one.

On laisse sous agitation pendant 6 heures à 40°C une solution de 0,25 g de 5-éthoxy-1,3-dihydro-1-(2-méthoxy-4-nitrobenzènesulfonyl)-3-spiro(4-hydroxycyclohexane)-indol-2-one (préparé selon le mode opératoire décrit à l'EXEMPLE 74 à partir du mélange d'isomères obtenus aux EXEMPLES 62 et 63 avant chromatographie), 0,6 ml de 2,6-*di-tert*-butylpyridine, 0,37 ml de trifluorométhanesulfonate d'éthyle dans du DCM. On ajoute 5 ml d'HCl 5N au mélange réactionnel, extrait à l'AcOEt, lave trois fois la phase organique par de l'HCl 0,5N, par une solution saturée de NaCl, sèche sur sulfate de magnésium et évapore sous vide le solvant. On obtient 0,5 g du produit attendu sous forme d'huile que l'on utilise tel quel à l'étape suivante.

B) 1-(4-Amino-2-méthoxybenzènesulfonyl)-5-éthoxy-1,3-dihydro-3-spiro(4-éthoxy-cyclohexane)indol-2-one.

On prépare ce composé selon le mode opératoire décrit à l'EXEMPLE 1 étape B' par réduction chimique du composé obtenu à l'étape précédente. On chromatographie sur silice en éluant par le mélange DCM/MeOH (99/1 ; v/v). On obtient le produit attendu, F = 110°C.

C) 5-Ethoxy-1,3-dihydro-1-(2-méthoxy-4-phénoxycarboxamidobenzènesulfonyl)-3-spiro(4-éthoxycyclohexane)indol-2-one.

On prépare ce composé selon le mode opératoire décrit à l'EXEMPLE 9 étape A à partir du composé obtenu à l'étape précédente. On obtient le produit attendu que l'on utilise tel quel à l'étape suivante.

D) 5-Ethoxy-1-[4-(N',N'-diéthyluréido)-2-méthoxybenzènesulfonyl]-1,3-dihydro-3-spiro(4-éthoxycyclohexane)indol-2-one.

On prépare ce composé selon le mode opératoire décrit à l'EXEMPLE 9 étape B à partir du composé obtenu à l'étape précédente et de diéthylamine. On obtient le produit attendu, F = 121°C.

EXEMPLE 76

5-Ethoxy-1-[4-(N',N'-diéthyluréido)-2-méthoxybenzènesulfonyl]-1,3-dihydro-3-spiro[4-(2-méthoxyéthyloxy)cyclohexane]indol-2-one.

A) 5-Ethoxy-1,3-dihydro-1-(2-méthoxy-4-nitrobenzènesulfonyl)-3-spiro[4-(2-méthoxy-éthyloxy)cyclohexane]indol-2-one.

On laisse 24 heures sous agitation à TA un mélange de 0,2 g de 5-éthoxy-1,3-dihydro-1-(2-méthoxy-4-nitrobenzènesulfonyl)-3-spiro(4-hydroxycyclohexane)indol-2-one, 2 g de 1-iodo-2-méthoxyéthane, 0,9 ml de 2,6-*di-tert*-butylpyridine, 2,15 g de trifluorométhanesulfonate d'argent dans 17 ml de $CCl_4$ et 8 ml de DCM. On ajoute 100 ml d'HCl 0,1N au mélange réactionnel, évapore sous vide les solvants, extrait à l'AcOEt, sèche sur sulfate de magnésium et évapore sous vide le solvant. On chromatographie le résidu sur silice en éluant au cyclohexane. On obtient 0,36 g du produit attendu que l'on utilise tel quel à l'étape suivante.

B) 1-(4-Amino-2-méthoxybenzènesulfonyl)-5-éthoxy-1,3-dihydro-3-spiro[4-(2-méthoxyéthyloxy)cyclohexane]indol-2-one.

On prépare ce composé selon le mode opératoire décrit à l'EXEMPLE 1 étape B' par réduction du composé obtenu à l'étape précédente. On obtient le produit attendu, F = 118°C.

C) 5-Ethoxy-1,3-dihydro-1-(2-méthoxy-4-phénoxycarboxamidobenzènesulfonyl)-3-spiro[4-(2-méthoxyéthyloxy)cyclohexane]indol-2-one.

On prépare ce composé selon le mode opératoire décrit à l'EXEMPLE 9 étape A à partir du composé obtenu à l'étape précédente. On obtient le produit attendu que l'on utilise tel quel à l'étape suivante.

D) 5-Ethoxy-1-[4-(N',N'-diéthyluréido)-2-méthoxybenzènesulfonyl]-1,3-dihydro-3-spiro[4-(2-méthoxyéthyloxy)cyclohexane]indol-2-one.

On prépare ce composé selon le mode opératoire décrit à l'EXEMPLE 9 étape B à partir du composé obtenu à l'étape précédente et de diéthylamine. On obtient le produit attendu, F = 98°C.

EXEMPLE 77

5-Ethoxy-1-[4-(N',N'-diéthyluréido)-2-méthoxybenzènesulfonyl]-1,3-dihydro-3-spiro(4-n-propyloxycyclohexane)indol-2-one.

On prépare ce composé selon les modes opératoires décrits à l'EXEMPLE 76 étape A, à partir de 5-éthoxy-1,3-dihydro-1-(2-méthoxy-4-nitrobenzènesulfonyl)-3-spiro(4-hydroxycyclohexane)indol-2-one et de 1-iodopropane dans le benzène, puis aux étapes B, C et D. On obtient le produit attendu, F = 115°C.

EXEMPLE 78

5-Ethoxy-1-[4-(N',N'-diéthyluréido)-2-méthoxybenzènesulfonyl]-1,3-dihydro-3-spiro[4-isopropyloxycyclohexane]indol-2-one, hémihydrate.

On prépare ce composé selon les modes opératoires décrits à l'EXEMPLE 76 étape A, à partir de 5-éthoxy-1,3-dihydro-1-(2-méthoxy-4-nitrobenzènesulfonyl)-3-spiro(4-hydroxycyclohexane)indol-2-one et de 2-iodopropane, puis aux étapes B, C et D. On obtient le produit attendu, F = 130°C.

EXEMPLE 79

5-Ethoxy-1-[4-(N',N'-diéthyluréido)-2-méthoxybenzènesulfonyl]-1,3-dihydro-3-spiro[4-(2-*tert*-butyloxyéthyloxy)cyclohexane]indol-2-one.

On prépare ce composé selon les modes opératoires décrits à l'EXEMPLE 76 étape A, à partir de 5-éthoxy-1,3-dihydro-1-(2-méthoxy-4-nitrobenzènesulfonyl)-3-spiro(4-hydroxycyclohexane)indol-2-one et de 1-iodo-2-*tert*-butyloxyéthane, puis aux étapes B, C et D. On obtient le produit attendu, F = 103°C.

EXEMPLE 80

5-Ethoxy-1-[4-(N',N'-diéthyluréido)-2-méthoxybenzènesulfonyl]-1,3-dihydro-3-spiro[4-(2-hydroxyéthyloxy)cyclohexane]indol-2-one.

On laisse 2 heures sous agitation à TA un mélange de 0,35 g du composé obtenu à l'EXEMPLE 79, 4 ml d'acide trifluoroacétique dans 15 ml de DCM. On ajoute 40 ml d'une solution saturée de $NaHCO_3$, après décantation, lave la phase organique à l'eau, sèche sur sulfate de magnésium et évapore sous vide le solvant. On chromatographie le résidu sur silice en éluant par le mélange DCM/MeOH (90/10 ; v/v). On obtient le produit attendu, F = 109°C.

EXEMPLE 81

5-Ethoxy-1-[4-(N',N'-diéthyluréido)-2-méthoxybenzènesulfonyl]-1,3-dihydro-3-spiro(4-formyloxycyclohexane)indol-2-one, isomère le plus polaire.

On chauffe à 40°C pendant 12 heures un mélange de 0,25 g du composé obtenu à l'EXEMPLE 69, 0,18 g de carbonate de césium, 0,45 ml de diméthylsulfate et 12 ml de DMF. On ajoute 10 ml d'eau, extrait le mélange réactionnel à l'AcOEt, lave la phase organique à l'eau, sèche sur sulfate de magnésium et évapore sous vide le solvant. On chromatographie le résidu sur silice en éluant au DCM. On obtient 0,2 g du produit attendu après recristallisation dans le mélange cyclohexane/AcOEt, F = 155°C.

EXEMPLE 82

5-Ethoxy-1-[4-(N',N'-diéthyluréido)-2-méthoxybenzènesulfonyl]-1,3-dihydro-3-spiro[4-acétoxycyclohexane] indol-2-one, isomère le plus polaire.

On chauffe à 40°C pendant 5 heures un mélange de 3 g du composé obtenu à l'EXEMPLE 69, 0,75 g de 4-diméthylaminopyridine, 3 ml d'anhydride acétique et 5 ml de DCM. On ajoute de l'eau au mélange réactionnel, extrait au DCM, lave à l'eau, sèche sur sulfate de magnésium et évapore sous vide le solvant. On chromatographie le résidu sur silice en éluant par le mélange DCM/cyclohexane. On obtient le produit attendu après recristallisation dans l'éther iso, F = 140°C.

EXEMPLE 83

5-Ethoxy-1,3-dihydro-1-(2,4-diméthoxybenzènesulfonyl)-3-spiro(8,9-dihydroxytricyclo[5.2.1.$0^{2,6}$]décane-4) indol-2-one.

A) 5-Ethoxy-1,3-dihydro-1-(2,4-diméthoxybenzènesulfonyl)-3-spiro(8,9-époxy-tricyclo[5.2.1.$0^{2,6}$]décane-4)indol-2-one.

On laisse 3 heures sous agitation à TA, un mélange de 0,3 g de 5-éthoxy-1,3-dihydro-1-(2,4-diméthoxybenzènesulfonyl)-3-spiro(tricyclo[5.2.1.$0^{2,6}$]déc-8-éne-4)indol-2-one, 0,2 g d'acide métachloroperbenzoïque dans 20 ml de DCM. On ajoute 15 ml d'une solution saturée de $NaHCO_3$, après décantation, extrait au DCM, sèche sur sulfate de magnésium et évapore sous vide le solvant. On chromatographie le résidu sur silice en éluant au DCM. On obtient 0,25 g du produit attendu après recristallisation dans un mélange acétone/DCM, F = 263°C.

B) 5-Ethoxy-1,3-dihydro-1-(2,4-diméthoxybenzènesulfonyl)-3-spiro(8,9-dihydroxytricyclo[5.2.1.$0^{2,6}$]décane-4)indol-2-one.

On chauffe à reflux pendant 8 heures un mélange de 0,2 g du composé obtenu à l'étape précédente, 20 ml d'eau, 2 ml d'acide sulfurique concentré et 20 ml de THF. On neutralise le mélange réactionnel par ajout d'une solution saturée de $NaHCO_3$, évapore le solvant sous vide, extrait au DCM, sèche sur sulfate de magnésium et évapore sous vide le solvant. On chromatographie le résidu sur silice en éluant par le mélange DCM/MeOH (99/1 ; v/v). On obtient 0,17 g du produit attendu, F = 150°C.

**Revendications**

1. Un composé de formule :

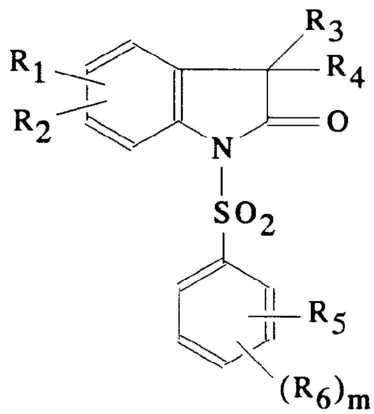

(I)

dans laquelle

- $R_1$ et $R_2$ représentent chacun indépendamment un hydrogène ; un hydroxyle ; un ω -halogénoalcoxy en $C_1$-$C_7$ ; un halogène, ;un alkyle en $C_1$-$C_7$ ; un trifluorométhyle ; un alcoxy en $C_1$-$C_7$ ; un polyhalogénoalcoxy en $C_1$-$C_7$ ; un ω -hydroxyalcoxy en $C_2$-$C_7$ ; un ω -méthoxyalcoxy dans lequel l'alkyle est en $C_2$-$C_7$ ; un ω -aminoalcoxy en $C_2$-$C_7$ libre ou substitué par un ou deux alkyles en $C_1$-$C_7$ ; un cycloalkyloxy en $C_3$-$C_7$ ; un cycloalkylméthoxy dans lequel le cycloalkyle est en $C_3$-$C_7$ ; un phénoxy; un benzyloxy ; un alkylthio en C1-C7; un phénylthio ; un nitro ; un amino libre ou substitué par un ou deux alkyles en $C_1$-$C_7$ ; un cyano ; un ($C_1$-$C_6$)alkylcarbonyle ; un formyle ; un ($C_1$-$C_6$) alkylcarbonyloxy ; un formyloxy ; un alkylsulfonamido en $C_1$-$C_7$ ; un phénylsulfonamido ; un benzylsulfonamido ; un alkylamido en $C_1$-$C_7$ ; un alcoxycarbonylamino en $C_1$-$C_7$ ; un uréido non substitué ou substitué par un phényle, par un benzyle ou par un ou deux alkyles en $C_1$-$C_7$ ; un thiouréido non substitué ou substitué par un phényle, par un benzyle ou par un ou deux alkyles en $C_1$-$C_7$ ;
- $R_3$ et $R_4$ représentent chacun indépendamment un alkyle en $C_1$-$C_7$ ; un cycloalkyle en $C_3$-$C_7$ ; un phényle ; un benzyle ; un cycloalkylméthyle dans lequel le cycloalkyle est en $C_3$-$C_7$ ; un ω -hydroxyalkyle en $C_2$-$C_7$ dans lequel l'hydroxyle est libre ou substitué par un groupe choisi parmi les groupes alkyle en $C_1$-$C_4$, ($C_1$-$C_5$) alcoxyalkyle dans lequel l'alkyle est en $C_1$-$C_4$, phénylalcoxyalkyle dans lequel l'alcoxy est en $C_1$-$C_2$ et l'alkyle en $C_1$-$C_4$, tétrahydrofuranyle et tétrahydropyranyle ;

ou

- $R_3$ et $R_4$ ensemble constituent un groupe $(CH_2)_pX(CH_2)_q$- ;

ou

- $R_3$ et $R_4$ ensemble avec le carbone auquel ils sont liés constituent un cycle hydrocarboné en $C_3$-$C_{12}$, saturé ou insaturé, éventuellement condensé, non substitué ou substitué par un ou plusieurs groupes alkyles en $C_1$-$C_7$ ; par un groupe oxo ; par un spirocycloalkyle en $C_3$-$C_5$ ; par un ou deux hydroxy libres ou substitués par un groupe choisi parmi les groupes alkyle en $C_1$-$C_4$, ($C_1$-$C_5$) alcoxyalkyle dans lequel l'alkyle est en $C_1$-$C_4$, ω -hydroxyalkyle dans lequel l'alkyle est en $C_1$-$C_4$, triphénylméthoxyalkyle dans lequel l'alkyle est en $C_1$-$C_4$, phénylalcoxyalkyle dans lequel l'alcoxy est en $C_1$-$C_2$ et l'alkyle en $C_1$-$C_4$, tétrahydrofuranyle, tétrahydropyranyle, formyle et ($C_1$-$C_7$)alkylcarbonyle ;
- $R_5$ et $R_6$ représentent chacun indépendamment un hydrogène ; un halogène ; un alkyle en $C_1$-$C_7$ ; un trifluorométhyle ; un cyano ; un nitro ; un amino libre ou substitué par un ou deux alkyles en $C_1$-$C_7$ ; un hydroxylamino ; un hydroxy ; un carboxy ; un guanidino non substitué ou substitué une ou deux fois par un alkyle en $C_1$-$C_7$, un phényle ou un benzyle; un groupe $OR_7$ ; un groupe $SR_7$ ; un ($C_1$-$C_6$)alkylcarbonyle ; un formyle ; un alcoxycarbonyle en $C_1$-$C_7$ ; un phénoxycarbonyle ; un benzyloxycarbonyle ; un carbamoyle substitué par des groupes $R'_6$ et $R''_6$ ; un thiocarbamoyle libre ou substitué par un ou deux alkyles en $C_1$-$C_7$ ; un sulfamoyle ; un alkylsulfamoyle ou dialkylsulfamoyle dans lequel l'alkyle est en $C_1$-$C_7$ ; un groupe $SO_2R'_7$ ; un alkylsulfonamido dans lequel l'alkyle est en $C_1$-$C_7$ ; un phénylsulfonamido ; un benzylsulfonamido ; un groupe $COR'_7$ ; un groupe $NR_8R_9$ ; un groupe CO-NH-$CR_{10}R'_{10}$-$COR_{12}$ ; le cas échéant, le groupe phényle constitutif du substituant $R_5$ et/ou $R_6$, peut être non substitué ou substitué une ou plusieurs fois par un alkyle en $C_1$-$C_7$, un trifluorométhyle, un alcoxy en $C_1$-$C_7$, un halogène, un sulfamoyle, un alkylsulfamoyle dans lequel l'alkyle est en $C_1$-$C_7$,

un carboxy, un alcoxycarbonyle dans lequel l'alkyle est en $C_1$-$C_7$, un ($C_1$-$C_6$)alkylcarbonyloxy, un imidazolyle ;

- $R'_6$ et $R''_6$ représentent chacun indépendamment l'hydrogène ; un alkyle en $C_1$-$C_7$ non substitué ou substitué par un ou plusieurs halogènes ou par $R'''_6$ ; un cycloalkyle en $C_3$-$C_7$ non substitué ou substitué par un ($C_1$-$C_4$)alkyle ; un phényle ; un pyridyle; un méthylpyridyle ; un pipéridin-4-yle ; un méthylpipéridin-4-yle ; un pyrrolidin-1-yle ; ou $R'_6$ et $R''_6$ constituent avec l'atome d'azote auquel ils sont liés un groupe pyrrolidino substitué par un hydroxyméthyle, un carbamoyle libre ou substitué par un ou deux alkyles en $C_1$-$C_7$ ;
- $R'''_6$ représente un hydroxyle ; un alcoxy en $C_1$-$C_7$ ; un amino libre ou substitué par un ou deux alkyles en $C_1$-$C_7$ ; un carbamoyle libre ou substitué par un ou deux alkyles en $C_1$-$C_7$ ou dans lequel les deux substituants ensemble avec l'atome d'azote auquel ils sont liés constituent un pyrrolidino, un pipéridino ou un perhydroazépino ; un cyano ; un carboxy libre ou estérifié par un alkyle en $C_1$-$C_7$ ou par un benzyle ; un phényle ; un cycloalkyle en $C_3$-$C_7$ ; un adamantyle ; un radical hétérocyclique choisi parmi les groupes pyridyle, méthylpyridyle, furanyle, tétrahydrofuranyle, thiényle, méthylthiényle, pyrrolidino, pipéridino, perhydroazépino ;
- $R_7$ représente un alkyle en $C_1$-$C_7$ ; un phényle ; un benzyle ; un cycloalkyle en $C_3$-$C_7$ ; un alcényle en $C_2$-$C_7$ ; un ω -halogénoalkyle en $C_2$-$C_7$ ; un polyhalogénoalkyle en $C_1$-$C_7$ ; un ω -hydroxyalkyle en ($C_2$-$C_7$) ; un ($C_1$-$C_6$)alkylcarbonyle ; un formyle ; un ω -carboxyalkyle en $C_1$-$C_7$ libre ou estérifié par un alkyle en $C_1$-$C_7$ ou par un benzyle ; un ω -aminoalkyle en $C_2$-$C_7$ dans lequel le groupe amino est libre, substitué par un ou deux alkyles en $C_1$-$C_7$ ou sous forme d'ion ammonium ; un ω -carbamoylalkyle en $C_1$-$C_7$ libre ou substitué par un ou deux alkyles en $C_1$-$C_7$ ;
- $R'_7$ représente un groupe pipérazin-1-yl non substitué ou substitué en 4 par un groupe $R''_7$ ; un groupe pipéridino non substitué ou substitué en 4 par un groupe $R'''_7$ un groupe azétidin-1-yl non substitué ou substitué en 3 par un groupe $R'''_7$ ; un groupe pyrrolidino non substitué ou substitué par un groupe $R''''_7$;
- $R''_7$ représente un alkyle en $C_1$-$C_7$, un phényle, un benzyle, un ($C_1$-$C_6$)alkylcarbonyle ; un formyle ;
- $R'''_7$ représente $R''_7$ ou un amino libre ou portant un groupe protecteur ;
- $R''''_7$ représente $R'''_7$ ou un groupe carboxy libre ou estérifié par un alkyle en $C_1$-$C_7$;
- $R_8$ et $R_9$ représentent chacun indépendamment un hydrogène ; un alkyle en $C_1$-$C_7$ ; un benzyle ; un phényle ; $R_9$ peut de plus représenter un alcène en $C_3$-$C_8$; un ($C_1$-$C_6$)alkylcarbonyle ; un formyle ; un ($C_1$-$C_6$)alkylthiocarbonyle ; un cycloalkylcarbonyle dans lequel le cycloalkyle est en $C_3$-$C_7$ ; un cycloalkylthiocarbonyle dans lequel le cycloalkyle est en $C_3$-$C_7$ ; un ω-amino($C_2$-$C_6$)alkylcarbonyle ; un ω-hydroxy($C_1$-$C_6$)alkylcarbonyle ; un ω-benzyloxy($C_1$-$C_6$)alkylcarbonyle ; un phénoxycarbonyle ; un phénoxythiocarbonyle ; un thiénocarbonyle ; un pyridylcarbonyle ; un méthylpyridylcarbonyle ; un alcoxycarbonyle en $C_1$-$C_7$ ; un benzoyle ; un phénacétyle ; un groupe $CO-CR_{10}R'_{10}-NR_{11}R'_{11}$ ; un groupe $CR_{10}R'_{10}COR_{12}$ ; un groupe $(CH_2)_t\,COR_{12}$; un groupe $CO(CH_2)_{t'}COR_{12}$ ; un carbamoyle non substitué ou substitué par $R_{14}$ et $R'_{14}$ ; un thiocarbamoyle non substitué ou substitué par $R_{14}$ et $R'_{14}$ ; un radical hétérocyclique choisi parmi les groupes pyrazolyle, imidazolyle, triazolyle, tétrazolyle, pyridinyle, pyridazinyle, pyrimidinyle, pyridyle, thiazolyle ;
- ou $R_8$ et $R_9$ ensemble avec l'atome d'azote auquel ils sont liés constituent l'hydantoïne, la N-méthyl hydantoïne ou un hétérocycle choisi parmi le pyrrol-1-yle, le Δ3-pyrrolin-1-yle, le pyrrolidin-1-yle ou l'isoindolin-2-yle dans lequel le noyau benzénique peut être non substitué ou substitué par un halogène, un alkyle en $C_1$-$C_7$, un trifluorométhyle ou un méthoxy ;
- $R_{10}$ et $R'_{10}$ représentent chacun indépendamment l'hydrogène, un alkyle en $C_1$-$C_7$ ou un benzyle ou $R_{10}$ et $R'_{10}$ ensemble avec l'atome de carbone auquel ils sont liés constituent un cycloalkyle en $C_3$-$C_7$ ;
- $R_{11}$ et $R'_{11}$ représentent chacun indépendamment l'hydrogène ou un alkyle en $C_1$-$C_7$ ;
- $R_{12}$ représente un hydroxy, un alcoxy en $C_1$-$C_7$, un amino non substitué ou substitué par un ou deux alkyles en $C_1$-$C_7$ ;
- $R_{13}$ représente l'hydrogène, un alkyle en $C_1$-$C_7$, un phényle, un benzyle, un ($C_1$-$C_6$)alkylcarbonyle, un formyle ; un alcoxycarbonyle en $C_1$-$C_7$, un carbamoyle non substitué ou substitué par un ou 2 alkyles en $C_1$-$C_7$ ;
- $R_{14}$ et $R'_{14}$ représentent chacun indépendamment un alkyle en $C_1$-$C_7$ non substitué ou substitué par $R_{15}$, un phényle non substitué ou substitué par $R'_{15}$, un cycloalkyle en $C_3$-$C_7$, un adamantyle ;
- ou $R_{14}$ et $R'_{14}$ ensemble avec l'atome d'azote auquel ils sont liés constituent un hétérocycle choisi parmi : la morpholine, la thiomorpholine, la pipérazine, l'azétidine, la pyrrolidine, la pipéridine ou la perhydroazépine, ledit hétérocycle étant non substitué ou substitué par un ou plusieurs groupes méthyles, par un phényle, par un groupe amino libre ou portant un groupe protecteur ;

- $R_{15}$ représente un phényle, un pyridyle, un hydroxy, un alcoxy en $C_1$-$C_7$, un amino libre ou substitué par un ou deux alkyles en $C_1$-$C_7$, un carboxy libre ou estérifié par un alkyle en $C_1$-$C_7$ ;
- $R'_{15}$ représente un hydroxy, un amino libre ou substitué par un ou deux alkyles en $C_1$-$C_7$ ;
- m est 1 ou, lorsque $R_6$ est un halogène, un alkyle en $C_1$-$C_7$ ou un alcoxy en $C_1$-$C_7$, m peut également être 2, 3 ou 4 ou bien $(R_6)_m$ peut représenter m substituants ayant différentes valeurs choisies parmi halogène, alkyle en $C_1$-$C_7$ ou alcoxy en $C_1$-$C_7$ ;
- p et q représentent chacun un nombre entier, leur somme peut varier de 3 à 6 ;
- t représente un nombre entier qui peut varier de 2 à 5 ;
- t' est un nombre entier qui peut varier de 0 à 3 ;
- X représente l'oxygène, un groupe $S(O)_n$, un groupe $NR_{13}$, un groupe $N(O)R_{13}$ ;
- n représente 0, 1 ou 2

avec la limitation que lorsque :

- $R_1$ et $R_2$ sont tels que définis ci-dessus à l'exception du groupe uréido substitué par un groupe benzyle ou du groupe thiouréido non substitué ou substitué par un phényle, un benzyle ou par un ou deux alkyles en $C_1$-$C_4$ ;
- $R_3$ et $R_4$ représentent chacun indépendamment un alkyle en $C_1$-$C_6$, un cycloalkyle en $C_3$-$C_7$, un phényle, un benzyle, un cycloalkylméthyle dans lequel le cycloalkyle est en $C_3$-$C_7$ ;

ou

- $R_3$ et $R_4$ ensemble constituent un groupe $-(CH_2)_pX(CH_2)_q-$ ; dans lequel X représente l'oxygène, le soufre ou un groupe $NR_{13}$,

ou

- $R_3$ et $R_4$ ensemble avec le carbone auquel ils sont liés constituent un cycle hydrocarboné en $C_3$-$C_{10}$, saturé ou insaturé, éventuellement condensé, non substitué ou substitué par un ou plusieurs groupes alkyles en $C_1$-$C_7$ ou par un spirocycloalkyle en $C_3$-$C_5$ ;
- $R_5$ et $R_6$ soient autres qu'un hydrogène ; un halogène ; un alkyle en $C_1$-$C_7$ ; un trifluorométhyle ; un cyano ; un nitro ; un amino libre ou substitué par un ou deux alkyles en $C_1$-$C_7$ ; un hydroxylamino ; un hydroxy ; un carboxy ; un groupe $OR_7$ ; un groupe $SR_7$ ; un $(C_1$-$C_6)$alkylcarbonyle ; un formyle ; un alcoxycarbonyle en $C_1$-$C_7$ ; un phénoxycarbonyle ; un benzyloxycarbonyle ; un carbamoyle substitué par des groupes $R'_6$ et $R''_6$ ; un thiocarbamoyle libre ou substitué par un ou deux alkyles en $C_1$-$C_7$ ; un sulfamoyle ; un alkylsulfamoyle ou dialkylsulfamoyle dans lequel l'alkyle est en $C_1$-$C_7$ ; un groupe $SO_2R'_7$ ; un alkylsulfonamido dans lequel l'alkyle est en $C_1$-$C_7$ ; un groupe $COR'_7$ ; un groupe $NR_6R_9$ ; un groupe $CO-NH-CH(R_{10})-COR_{12}$ ; le cas échéant, le groupe phényle constitutif du substituant $R_6$ et/ou $R_6$, pouvant être non substitué ou substitué une ou plusieurs fois par un alkyle en $C_1$-$C_7$, un trifluorométhyle, un méthoxy, un halogène, un sulfamoyle, un alkylsulfamoyle dans lequel l'alkyle est en $C_1$-$C_7$, un carboxy, un alcoxycarbonyle dans lequel l'alkyle est en $C_1$-$C_7$, un $(C_1$-$C_6)$alkylcarbonyloxy, un formyloxy, un imidazolyle ;

dans lesquels groupes $R_6$ et/ou $R_6$ :

- $R'_6$ et $R''_6$ représentent chacun indépendamment l'hydrogène ; un alkyle en $C_1$-$C_7$ non substitué ou substitué par $R'''_6$ ; un phényle ; un pyridyle ; un méthylpyridyle; un pipéridin-4-yle ; un méthylpipéridin-4-yle ;
- $R'''_6$ représente un hydroxy ; un cyano ; un carboxy libre ou estérifié par un alkyle en $C_1$-$C_7$ ou par un benzyle ; un phényle ; un pyridyle ; un méthylpyridyle; un amino libre ou substitué par un ou deux alkyles en $C_1$-$C_7$;
- $R_7$ représente un alkyle en $C_1$-$C_7$ ; un phényle ; un benzyle ; un cycloalkyle en $C_3$-$C_7$ ; un alcényle en $C_2$-$C_4$ ; un $\omega$ -halogénoalkyle en $C_2$-$C_7$ ; un polyhalogénoalkyle en $C_1$-$C_7$ ; un $(C_1$-$C_6)$alkylcarbonyle ; un formyle ; un $\omega$ -carboxyalkyle en $C_1$-$C_7$ libre ou estérifié par un alkyle en $C_1$-$C_4$ ou par un benzyle ; un $\omega$ -aminoalkyle en $C_2$-$C_7$ dans lequel le groupe amino est libre, substitué par un ou deux alkyles en $C_1$-$C_4$ ou sous forme d'ion ammonium ;
- $R'_7$ représente un groupe pipérazin-1-yl non substitué ou substitué en 4 par un groupe $R''_7$, un groupe pipéridino non substitué ou substitué en 4 par un groupe $R'''_7$, un groupe azétidin-1-yl non substitué ou substitué en 3 par un groupe $R'''_7$,
- $R''_7$ représente un alkyle en $C_1$-$C_4$, un phényle, un benzyle, un $(C_1$-$C_3)$alkylcarbonyle ;
- $R'''_7$ représente $R''_7$ ou un amino libre ou portant un groupe protecteur ;
- $R_8$ et $R_9$ représentent chacun indépendamment un hydrogène ; un alkyle en $C_1$-$C_7$ ; un phényle ; un benzyle ; $R_9$ peut de plus représenter un $(C_1$-$C_6)$alkylcarbonyle ; un formyle ; un cycloalkylcarbonyle dans lequel le cycloalkyle est en $C_3$-$C_7$ ; un cycloalkylthiocarbonyle dans lequel le cycloalkyle est en $C_2$-$C_7$ ; un $\omega$-amino$(C_1$-$C_3)$alkylcarbonyle ; un $\omega$-hydroxy$(C_1$-$C_3)$alkylcarbonyle ; un $\omega$-benzyloxy$(C_1$-$C_3)$alkylcarbonyle ; un phénoxycarbonyle ; un thiénocarbonyle ; un pyridylcarbonyle ; un méthylpy-

ridylcarbonyle ; un alcoxycarbonyle en $C_1$-$C_4$; un benzoyle ; un groupe CO-CH($R_{10}$)-$NR_{11}R'_{11}$ ; un groupe CH($R_{10}$)$CO_2R_{11}$ ; un groupe $(CH_2)_{t''}$ $COR_{12}$ ; un groupe $CO(CH_2)_{t''}COR_{12}$ ; un carbamoyle non substitué ou substitué par un phényle ou par un ou deux alkyles en $C_1$-$C_4$ ;

- t" représente un nombre entier qui peut varier de 1 à 3 ;
- $R_{10}$ représente l'hydrogène, un alkyle en $C_1$-$C_4$ ou un benzyle ;
- $R_{11}$ et $R'_{11}$ représentent chacun indépendamment l'hydrogène ou un alkyle en $C_1$-$C_4$ ;
- $R_{12}$ représente un hydroxy, un alcoxy en $C_1$-$C_4$, un amino non substitué ou substitué par un ou deux alkyles en $C_1$-$C_4$ ;
- $R_{13}$ représente l'hydrogène ; un alkyle en $C_1$-$C_4$ ; un phényle ; un benzyle ; un ($C_1$-$C_3$)alkylcarbonyle ; un formyle ; un alcoxycarbonyle en $C_1$-$C_4$ ; un carbamoyle non substitué ou substitué par un ou 2 alkyles en $C_1$-$C_4$ ;

ainsi que ses sels éventuels.

2. Un composé de formule (I) selon la revendication 1 dans lequel $R_1$ est un atome de chlore, de fluor ou un groupe éthoxy en position 5 du 1,3-dihydro-indol-2-one et $R_2$ est l'hydrogène.

3. Un composé de formule (I) selon l'une quelconque des revendications 1 ou 2 dans lequel $R_3$ et $R_4$ ensemble avec le carbone auquel ils sont liés constituent un cycle hydrocarboné en $C_3$-$C_{12}$.

4. Un composé de formule (I) selon la revendication 3 dans lequel $R_3$ et $R_4$ ensemble avec le carbone auquel ils sont liés constituent un cycloheptane, un adamantane, un tricyclo [5.2.1.$0^{2,6}$]dec-8-ène, un tricyclo [5.2.1.$0^{2,6}$]décane, un bicyclo [2.2.1]heptane, un bicyclo [3.3.1]nonane, ou un cyclohexane non substitué ou substitué par un spirocycloalkyle en $C_3$-$C_5$ ou par un ou deux groupes alkyles en $C_1$-$C_7$.

5. Un composé de formule (I) selon l'une quelconque des revendications 1 ou 2 dans lequel $R_3$ et $R_4$ ensemble avec le carbone auquel ils sont liés constituent un cyclohexane substitué par un groupe choisi parmi : méthoxy, éthoxy, 2-méthoxyéthoxy.

6. Un composé de formule (I) selon l'une quelconque des revendications 1 à 5 dans lequel $R_5$ représente un groupe orthométhoxy et $R_5$ en position para représente un groupe choisi parmi :
   - (pipéridin-1-yl)carboxamido,
   - (2-cyanoprop-2-yl)carbonyle,
   - pyrrolidin-1-yl,
   - 3,3-diéthylguanidino
   - N',N'-diéthylthioureido.

7. Un procédé de préparation d'un composé de formule (I) selon la revendication 1 et de ses sels caractérisé en ce que :

   1/ on fait agir sur un 1,3-dihydro-indol-2-one disubstitué en position 3, de formule :

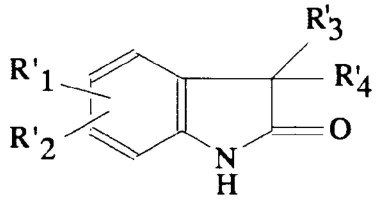

(II)

dans laquelle $R'_1$, $R'_2$, $R'_3$ et $R'_4$ représentent, respectivement, soit $R_1$, $R_2$, $R_3$ et $R_4$ tels que définis pour (I) dans la revendication 1, soit des groupes précurseurs de $R_1$, $R_2$, $R_3$ et $R_4$, un halogénure de benzènesulfonyle de formule :

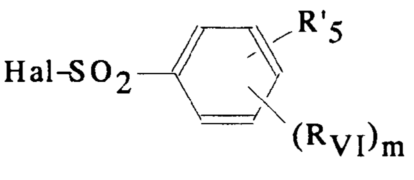

(III)

dans laquelle Hal représente un atome d'halogène, de préférence le chlore et $R'_5$ et $R_{VI}$ représentent, respectivement, soit $R_5$ et $R_6$ tels que définis pour (I) dans la revendication 1, soit des groupes précurseurs de $R_5$ et $R_6$ ; et,

2/ soit, lorsque $R'_1 = R_1$, $R'_2 = R_2$, $R'_3 = R_3$, $R'_4 = R_4$, $R'_5 = R_6$ et $R_{VI} = R_6$, on isole le composé de formule (I) ainsi obtenu ;

3/ soit, lorsque l'un quelconque des groupes $R'_1$, $R'_2$, $R'_3$, $R'_4$, $R'_5$ et/ou $R_{VI}$ représente respectivement un groupe précurseur de $R_1$, $R_2$, $R_3$, $R_4$, $R_6$ et/ou $R_6$, on soumet le composé obtenu, à l'étape 1, à un traitement ultérieur pour préparer le composé de formule (I) par transformation de l'un quelconque des groupes $R'_1$, $R'_2$, $R'_3$, $R'_4$, $R'_5$ et/ou $R_{VI}$ en, respectivement, $R_1$, $R_2$, $R_3$, $R_4$, $R_5$ et/ou $R_6$ ; et,

4/ le cas échéant, on transforme le composé de formule (I) obtenu en un de ses sels.

**8.** Composition pharmaceutique contenant à titre de principe actif, un composé selon l'une quelconque des revendications 1 à 6.

**9.** Composition pharmaceutique contenant un composé selon l'une quelconque des revendications 1 à 6 en association avec un autre principe actif.

**10.** Composition pharmaceutique contenant deux composés selon l'une quelconque des revendications 1 à 6, l'un étant un antagoniste spécifique du récepteur $V_1$ et l'autre étant un antagoniste spécifique du récepteur $V_2$.

**11.** Composition pharmaceutique contenant deux composés selon l'une quelconque des revendications 1 à 6, l'un étant un antagoniste spécifique du récepteur $V_1$ et l'autre étant un antagoniste spécifique de l'ocytocine.

Office européen des brevets

# RAPPORT DE RECHERCHE EUROPEENNE

Numero de la demande
EP 94 40 1737

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X,P, D | WO-A-93 15051 (ELF SANOFI) 5 Août 1993<br>* le document en entier * | 1-11 | C07D209/34<br>C07D209/96<br>A61K31/44<br>A61K31/40<br>C07D491/10<br>C07D409/12<br>C07D403/12<br>C07D401/12<br>C07D495/10<br>C07D405/12<br>C07D405/14 |
| A,D | EP-A-0 450 761 (MERCK & CO., INC.)<br>* page 2; revendications 1,4; exemples * | 1-11 | |
| A,D | US-A-4 803 217 (MERCK & CO., INC.)<br>* le document en entier * | 1-11 | |
| X | CHEMICAL ABSTRACTS, vol. 77, no. 17, 23 Octobre 1972, Columbus, Ohio, US;<br>abstract no. 114172c,<br>M. NATSUME ET AL. 'Formation of 3-cyano-1,4-dihydroquinolines and their conversion to indole derivatives.'<br>page 429 ;<br>*CAS RN 36797-34-9 et 36797-35-0*<br>* abrégé *<br>& CHEMICAL AND PHARMACEUTICAL BULLETIN.<br>vol. 20, no. 7 , 1972 , TOKYO JP<br>pages 1595 - 1598 | 1 | DOMAINES TECHNIQUES RECHERCHES (Int.Cl.6) |
| X | CHEMICAL ABSTRACTS, vol. 72, no. 11, 16 Mars 1970, Columbus, Ohio, US;<br>abstract no. 55170x,<br>T. OISHI ET AL. 'Reactions of activated amides. I. Reactions of imino ethers derived from the secondary amides with the nucleophiles.'<br>page 397 ;<br>*CAS RN 25560-29-6*<br>* abrégé *<br>& CHEMICAL AND PHARMACEUTICAL BULLETIN.<br>vol. 17, no. 11 , 1969 , TOKYO JP<br>pages 2306 - 2313 | 1 | C07D<br>A61K |

Le présent rapport a été établi pour toutes les revendications

| Lieu de la recherche | Date d'achèvement de la recherche | Examinateur |
|---|---|---|
| LA HAYE | 6 Septembre 1994 | Bosma, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)